(19) Europäisches Patentamt
European Patent Office
Office européen des brevets

(11) EP 4 335 852 B1

(12) EUROPEAN PATENT SPECIFICATION

(45) Date of publication and mention of the grant of the patent:
**05.08.2026 Bulletin 2026/32**

(21) Application number: **22798604.9**

(22) Date of filing: **28.04.2022**

(51) International Patent Classification (IPC):
***C07D 487/06*** (2006.01) ***A61K 31/519*** (2006.01)
***A61P 35/00*** (2006.01)

(52) Cooperative Patent Classification (CPC):
**A61P 35/00; C07D 487/06**

(86) International application number:
**PCT/CN2022/089956**

(87) International publication number:
**WO 2022/233263 (10.11.2022 Gazette 2022/45)**

# (54) TRICYCLIC UBIQUITIN SPECIFIC PROTEASE 1 INHIBITOR AND USE THEREOF

TRICYCLISCHER UBIQUITINSPEZIFISCHER PROTEASE-1-INHIBITOR UND DESSEN VERWENDUNG

INHIBITEUR DE PROTÉASE 1 SPÉCIFIQUE DE L'UBIQUITINE TRICYCLIQUE ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2021 CN 202110486972**
**16.07.2021 CN 202110806606**
**13.12.2021 CN 202111514025**
**19.01.2022 CN 202210062340**

(43) Date of publication of application:
**13.03.2024 Bulletin 2024/11**

(73) Proprietor: **Xuanzhu Biopharmaceutical Co., Ltd.**
**Shijiazhuang, Hebei 050035 (CN)**

(72) Inventors:
- **LIU, Bin**
  **Jinan, Shandong 250101 (CN)**
- **CHEN, Bo**
  **Jinan, Shandong 250101 (CN)**

(74) Representative: **Murgitroyd & Company**
**165-169 Scotland Street**
**Glasgow G5 8PL (GB)**

(56) References cited:
**WO-A1-2011/036280** **WO-A1-2020/132269**
**JP-A- 2017 043 603** **US-A1- 2011 098 483**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The invention relates to the technical field of medicine, and particularly relates to a tricyclic ubiquitin specific protease 1 inhibitor compound, a pharmaceutically acceptable salt thereof, an ester thereof, a deuterated compound or a stereoisomer thereof; a pharmaceutical composition and a formulation containing the compound, the pharmaceutically acceptable salt thereof, the ester thereof, the deuterated compound or the stereoisomer thereof, a method for preparing the compound, the pharmaceutically acceptable salt thereof, the ester thereof, the deuterated compound or the stereoisomer thereof, and a use of the compound, the pharmaceutically acceptable salt thereof, the ester thereof, the deuterated compound or the stereoisomer thereof in the manufacture of a medicament for treating and/or preventing a disease mediated by USP1 and a disease related thereto.

Esters are not included within the scope of the invention.

### BACKGROUND

There are many relevant targets in the occurrence and development of tumors. Deubiquitinating enzyme DUB encodes more than 100 human genes and is divided into 6 families, where ubiquitin-specific protease (USP) contains 50 members and is the largest family of DUB. Ubiquitination is a reversible process. DUB acts on ubiquitin-protease systems, lysing an isopeptide bond between lysine and a C-terminus of UBQ (Ubiquitin), affecting cell proliferation, cycle, apoptosis, DNA damage response, tumor suppression, occurrence and metastasis.

USP1 (Ubiquitin specific protease) is a member of the USP family, and is a cysteine isopeptidase with a triple structure containing Cys90, His593 and Asp751. Human USP1 gene was cloned in 1998, encoding one protein with 785 amino acids. In a normal state, USP1 is relatively inactive. After combining with UAF1 (USP1-associated factor 1, one cofactor containing WD40(Conserved sequence of 40 amino acids ending with tryptophan and aspartic acid) repeats and regulating USP1 activity) to form a heterodimeric complex, USP1 is activated to play a role of deubiquitinating enzyme, stabilize a replication fork and locate in a nucleus.

USP1 is highly expressed in cancers such as breast cancer and ovarian cancer, and the expression of USP1 is also increased in other cancers. The overexpression of USP1 is related to breast/ovarian cancer BRCA1 deficiency. USP1 deubiquitously participates in various processes related to cancer, and acts on pathways such as Fanconi anemia (FA), Transdamage DNA Synthesis (TLS), cell differentiation, etc. In FA, USP1 deubiquitinates Fanconi anabema group D2 Protein (FANCD2). In TLS, USP1 deubiquitinates Proliferating cell nuclear antigen (PCNA). In cell differentiation, USP1 affects ubiquitination of DNA binding protein inhibitor family (ID), and regulates cell proliferation and differentiation.

These DNA damage response (DDR) pathways are critical to repair of DNA damage induced by DNA cross-linking agents (such as cisplatin and ultraviolet radiation, etc.). In the TLS pathway, PCNA affected by USP1 together with USP1/UAF1 and BRCA1/2 participates in DNA break repair. After stagnation of the replication fork, RAD18-mediated PCNA mono-ubiquitination promotes conversion of PCNA from replication-type polymerase (pol $\delta/\varepsilon$) to TLS polymerase (such as POLLK), after bypassing the lesion by the TLS polymerase, USP1 deubiquitinates the PCNA again and promotes PCNA binding to convert back to replication polymerase. Inhibiting USP1 leads to instability of the replication fork and has synthetic lethality with BRCA mutations.

A USP1 inhibitor inhibits DNA break repair jointly participated by PCNA with USP1/UAF1 and BRCA1/2, making the replication fork unstable. Therefore, using a small molecule inhibitor to inhibit USP1 has the potential for the treatment of cancers and other diseases, and has not been commercialized or clinically developed.

PCT Patent Application number WO 2011/036280 A1 discloses fused tricyclic derivatives having a PI3K inhibition activity for useagainst cancer.

PCT Patent Application number WO 2020/132269 A1 discloses pyrazolo-pyrimidines derivatives which show some USP-1inhibition activity.

US Patent Application number US 2011/098483 A1 discloses a nitrogen heterocyclic compound for treating cancer, which is structurally remote from the compound for treating cancer in the present application.

Japanese Patent Application JP 2017 043603 A discloses a 3,4-disubstituted cyclic tricyclic indole derivative or benzofuran compound for treating cancer, which is structurally remote from the compound for treating cancer in the present application.

### SUMMARY

The invention is as defined in claims 1 to 14.

Therefore, the present invention aims at providing a tricyclic ubiquitin specific protease 1 inhibitor and use thereof. The specific technical solutions are as follows.

Solution 1: the present invention first provides a compound of general formula (I), or a pharmaceutically acceptable salt, ester, deuterated compound or stereoisomer thereof;

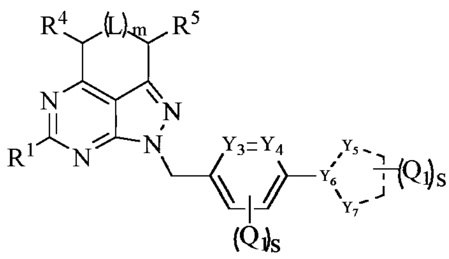

formula (II-2)

wherein:

$R^1$ is selected from phenyl or 5-6 membered heteroaryl optionally substituted by 1-4 $Q_1$;
$R^4$ and $R^5$ are each independently selected from deuterium, hydrogen, carboxyl, cyano, nitro, amino, halogen, $C_{2-6}$ alkenyl, $C_{2-6}$ alkynyl, or from $C_{1-6}$ alkyl, $C_{1-6}$ alkoxy, $C_{1-6}$ alkylamino, di($C_{1-6}$ alkyl)amino, halogenated $C_{1-6}$ alkyl, hydroxy $C_{1-6}$ alkyl, amino $C_{1-6}$ alkyl, carboxyl $C_{1-6}$ alkyl or halogenated $C_{1-6}$ alkoxy, each of which is optionally deuterated;
each $Q_1$ is independently selected from deuterium, halogen, cyano, carboxyl, hydroxyl, amino, nitro, sulfonamido, or from $C_{1-6}$ alkylamino, di($C_{1-6}$ alkyl)amino, halogenated $C_{1-6}$ alkyl, halogenated $C_{1-6}$ alkoxy, hydroxy $C_{1-6}$ alkyl, amino $C_{1-6}$ alkyl, carboxyl $C_{1-6}$ alkyl, $C_{1-6}$ alkylcarbonyl, $C_{1-6}$ alkoxycarbonyl, $C_{1-6}$ alkylaminoacyl, $C_{1-6}$ alkylamido, $C_{1-6}$ alkylsulfonyl,$C_{1-6}$ alkylsulfonamido, $C_{1-6}$ alkylaminosulfonyl, $-(L)_m-C_{1-6}$ alkyl, $-(L)_m-C_{2-6}$ alkenyl, $-(L)_m-C_{2-6}$ alkynyl, $-(L)_m-C_{1-6}$ alkoxy, $-(L)_m$-6-10 membered aryl, $-(L)_m$-5-12 membered heteroaryl, $-(L)_m$-3-8 membered cycloalkyl or $-(L)_m$-3-8 membered heterocyclyl, each of which is optionally substituted by 1-4 substituents $Q_2$; and each $Q_2$ is independently selected from deuterium, halogen, carboxyl, hydroxyl, cyano, nitro, amino, $C_{1-6}$ alkyl, hydroxy $C_{1-6}$ alkyl, carboxyl $C_{1-6}$ alkyl, $C_{1-6}$ alkylamino, di($C_{1-6}$ alkyl)amino, -CO-$C_{1-6}$ alkylene-$NH_2$, -CO-$C_{1-6}$ alkyl, $C_{1-6}$ alkoxy, halogenated $C_{1-6}$ alkyl and halogenated $C_{1-6}$ alkoxy;
each L is independently selected from $-CR^aR^b-$;
each $R^a$ and each $R^b$ are each independently selected from deuterium, hydrogen, or from $C_{1-6}$ alkyl, $C_{1-6}$ alkoxy, each of which is optionally deuterated;
$Y_3$, $Y_4$, and $Y_6$ are each independently selected from N, C or $CR^a$;
$Y_5$ and $Y_7$ are each independently selected from N, $NR^c$, C, $CR^aR^b$ or $CR^a$;
each $R^c$ is independently selected from deuterium, hydrogen, or from $C_{1-6}$ alkyl, halogenated $C_{1-6}$ alkyl, halogenated $C_{1-6}$ alkoxy, hydroxy $C_{1-6}$ alkyl, amino $C_{1-6}$ alkyl and carboxyl $C_{1-6}$ alkyl, each of which is optionally deuterated;
each m is independently an integer of 0-3;
.-- and - is each independently selected from a single bond or a double bond, and two adjacent bonds are incapable of being double bonds at the same time; and
each s is independently selected from an integer of 0-2.

Solution 2: the compound or the pharmaceutically acceptable salt, ester, deuterated compound or stereoisomer thereof according to the solution 1, wherein:

$R^1$ is selected from phenyl or 5-6 membered heteroaryl, each of which is optionally substituted by 1-3 $Q_1$;
$R^4$ and $R^5$ are each independently selected from deuterium, hydrogen, cyano, halogen, or from $C_{1-6}$ alkyl, $C_{1-6}$ alkoxy, $C_{1-6}$ alkylamino, di($C_{1-6}$ alkyl)amino, halogenated $C_{1-6}$ alkyl, hydroxy $C_{1-6}$ alkyl, amino $C_{1-6}$ alkyl, carboxyl $C_{1-6}$ alkyl or halogenated $C_{1-6}$ alkoxy, each of which is optionally deuterated;
each $Q_1$ is independently selected from deuterium, hydrogen, or from $C_{1-6}$ alkyl, $C_{1-6}$ alkoxy, halogenated $C_{1-6}$ alkyl, halogenated $C_{1-6}$ alkoxy, and 3-6 membered cycloalkyl, each of which is optionally substituted by 1-3 substituents $Q_2$; and each $Q_2$ is independently selected from deuterium, $C_{1-6}$ alkyl, $C_{1-6}$ alkoxy, halogenated $C_{1-6}$ alkyl and halogenated $C_{1-6}$ alkoxy;
each L is independently selected from $-CR^aR^b-$;
each $R^a$ and each $R^b$ are each independently selected from deuterium, hydrogen, or from methyl, ethyl, propyl, isopropyl, butyl, isobutyl, sec-butyl, tert-butyl, methoxy, ethoxy, propoxy or isopropoxy,, each of which is optionally deuterated;
$Y_3$ and $Y_4$ are each independently selected from N, C or $CR^a$;

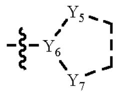

is selected from the following structures:

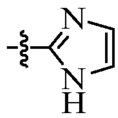, 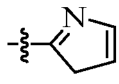, 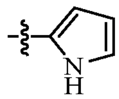, 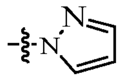,

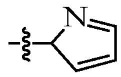, and 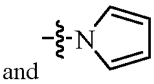;

each $R^c$ is independently selected from deuterium, hydrogen, or from $C_{1-6}$ alkyl, halogenated $C_{1-6}$ alkyl and halogenated $C_{1-6}$ alkoxy, each of which is optionally deuterated;
each m is independently an integer of 0-2;
-- and - is each independently selected from a single bond or a double bond, and two adjacent bonds are incapable of being double bonds at the same time; and
each s is independently selected from an integer of 0-2.

Solution 3: the compound or the pharmaceutically acceptable salt, ester, deuterated compound or stereoisomer thereof according to the solution 1 or 2, wherein:

$R^1$ is selected from phenyl or 5-6 membered heteroaryl optionally substituted by 1-4 $Q_1$;
$R^4$ and $R^5$ are each independently selected from deuterium, hydrogen, or from $C_{1-4}$ alkyl, $C_{1-4}$ alkoxy, $C_{1-4}$ alkylamino, di($C_{1-4}$ alkyl)amino, halogenated $C_{1-4}$ alkyl, hydroxy $C_{1-4}$ alkyl, amino $C_{14}$ alkyl, carboxyl $C_{1-4}$ alkyl or halogenated $C_{1-4}$ alkoxy, each of which is optionally deuterated;
each $Q_1$ is independently selected from deuterium, halogen, or from $C_{1-4}$ alkoxy, $C_{1-4}$ alkylamino, di($C_{1-4}$ alkyl)amino, halogenated $C_{1-4}$ alkyl, halogenated $C_{1-4}$ alkoxy, hydroxy $C_{1-4}$ alkyl, amino $C_{1-4}$ alkyl, carboxyl $C_{1-4}$ alkyl, $-(L)_m-C_{1-4}$ alkyl, or $-(L)_m$-3-6 membered cycloalkyl, each of which is optionally substituted by 1-3 substituents $Q_2$; and each $Q_2$ is independently selected from deuterium, halogen, carboxyl, hydroxyl, cyano, nitro, amino, $C_{1-4}$ alkyl, hydroxy $C_{1-4}$ alkyl, carboxyl $C_{1-4}$ alkyl, $C_{1-4}$ alkylamino, di($C_{1-4}$ alkyl)amino, $C_{1-4}$ alkoxy, halogenated $C_{1-4}$ alkyl and halogenated $C_{1-4}$ alkoxy;
each L is independently selected from $-CR^aR^b$;
each $R^a$ and each $R^b$ are each independently selected from deuterium, hydrogen, or from $C_{1-4}$ alkyl, $C_{1-4}$ alkoxy, each of which is optionally deuterated;
each $R^c$ is independently selected from deuterium, hydrogen, or from $C_{1-4}$ alkyl, halogenated $C_{1-4}$ alkyl and halogenated $C_{1-4}$ alkoxy, each of which is optionally deuterated; and
each m is independently an integer of 0-2.

Solution 4: the compound or the pharmaceutically acceptable salt, ester, deuterated compound or stereoisomer thereof according to any of the preceding solutions, wherein:

$R^1$ is selected from phenyl, furyl, thienyl, pyrrolyl, thiazolyl, isothiazolyl, thiadiazolyl, oxazolyl, isoxazolyl, oxadiazole, imidazolyl, pyrazolyl, 1,2,3-triazolyl, 1,2,4-triazolyl, pyridyl, 2-pyridone, 4-pyridone, pyrimidinyl, pyridazinyl, pyrazinyl, 1,2,3-triazolyl, 1,3,5-triazinyl and 1,2,4,5-tetrazinyl, each of which is optionally substituted by 1-3 $Q_1$;
$R^4$ and $R^5$ are each independently selected from deuterium, hydrogen, or from methyl, ethyl, propyl, isopropyl, butyl, isobutyl, sec-butyl, tert-butyl, methoxy, ethoxy, propoxy, isopropoxy, methylamino, dimethylamino, monofluoromethyl, difluoromethyl, monofluoromethoxy, difluoromethoxy, trifluoromethyl, hydroxymethyl, hydroxyethyl, hydroxypropyl, hydroxybutyl, aminomethyl, carboxymethyl, carboxyethyl and trifluoromethoxy, each of which is optionally deuterated;
each $Q_1$ is independently selected from deuterium, fluorine, chlorine, bromine, iodine, or from methoxy, ethoxy, propoxy, isopropoxy, methylamino, dimethylamino, monofluoromethyl, difluoromethyl, trifluoromethyl, monofluoromethoxy, difluoromethoxy, trifluoromethoxy, hydroxymethyl, hydroxyethyl, hydroxypropyl, hydroxybutyl, aminomethyl, carboxymethyl, carboxyethyl, $-(L)_m-C_{1-4}$ alkyl, or $-(L)_m$-3-6 membered cycloalkyl, each of which is optionally substituted by 1-3 substituents $Q_2$, and each $Q_2$ is independently selected from deuterium, halogen, carboxyl,

hydroxyl, cyano, nitro, amino, methyl, ethyl, propyl, isopropyl, butyl, isobutyl, sec-butyl, tert-butyl, methoxy, ethoxy, propoxy, isopropoxy, hydroxymethyl, hydroxyethyl, hydroxypropyl, hydroxybutyl, carboxymethyl, carboxyethyl, methylamino, dimethylamino, monofluoromethyl, difluoromethyl, trifluoromethyl, monofluoromethoxy, difluoromethoxy and trifluoromethoxy;
each L is independently selected from $-CR^aR^b-$;
each $R^a$ and each $R^b$ are each independently selected from deuterium, hydrogen;
$Y_3$, $Y_4$, and $Y_6$ are each independently selected from N, C or $CR^a$;
$Y_5$ and $Y_7$ are each independently selected from N, $NR^c$, C, $CR^aR^b$ or $CR^a$;
each $R^c$ is independently selected from deuterium, hydrogen, or from $C_{1-4}$ alkyl, halogenated $C_{1-4}$ alkyl and halogenated $C_{1-4}$ alkoxy, each of which is optionally deuterated;
each m is independently selected from 0, 1 or 2;
-- and - is each independently selected from a single bond or a double bond, and two adjacent bonds are incapable of being double bonds at the same time; and
each s is independently 0, 1 or 2.

Solution 5: the compound, the pharmaceutically acceptable salt thereof, the ester thereof, the deuterated compound or the stereoisomer thereof according to any of the preceding solutions, wherein:
$R^1$ is phenyl or 5-6 membered nitrogen-containing heteroaryl, each of which is optionally substituted by 1-4 $Q_1$.

Solution 6: the compound or the pharmaceutically acceptable salt, ester, deuterated compound or stereoisomer thereof according to any of the preceding solutions, wherein:
$R^1$ is selected from phenyl, furyl, thienyl, pyrrolyl, thiazolyl, isothiazolyl, oxazolyl, isoxazolyl, imidazolyl, pyrazolyl, pyridyl, pyrimidinyl, pyridazinyl and pyrazinyl, each of which is optionally substituted by 1-3 $Q_1$.

Solution 7: the compound or the pharmaceutically acceptable salt, ester, deuterated compound or stereoisomer thereof according to any of the preceding solutions, wherein:
$R^1$ is selected from phenyl, pyrrolyl, thiazolyl, isothiazolyl, oxazolyl, isoxazolyl, imidazolyl, pyrazolyl, pyridyl, pyrimidinyl, pyridazinyl and pyrazinyl, each of which is optionally substituted by 1-3 $Q_1$.

Solution 7-1: the compound or the pharmaceutically acceptable salt, ester, deuterated compound or stereoisomer thereof according to any of the preceding solutions, wherein:
$R^1$ is selected from phenyl, pyrimidinyl, pyridyl, pyrazolyl, imidazolyl, pyrrolyl, pyridazinyl and pyrazinyl, each of which is optionally substituted by 1-3 $Q_1$.

Solution 7-2: the compound or the pharmaceutically acceptable salt, ester, deuterated compound or stereoisomer thereof according to any of the preceding solutions, wherein:

$R^1$ is selected from

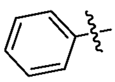, 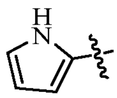, 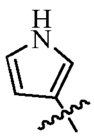, 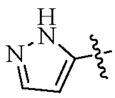, 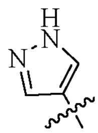, 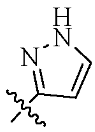, 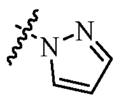,

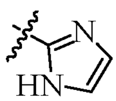, 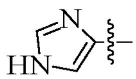, 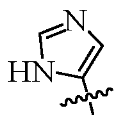, 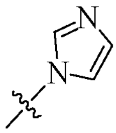, 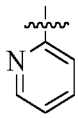, 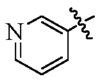, 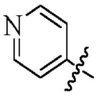, 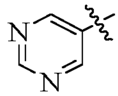, 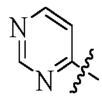,

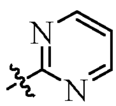, 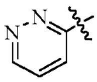 or 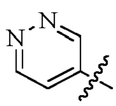,

each of which is optionally substituted by 1-3 $Q_1$.

Solution 7-3: the compound or the pharmaceutically acceptable salt, ester, deuterated compound or stereoisomer thereof according to any of the preceding solutions, wherein:
$R^1$ is selected from

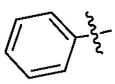, 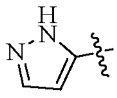, 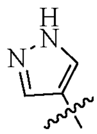, 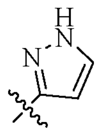, 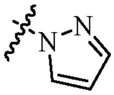, 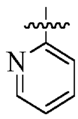, 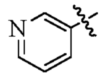,

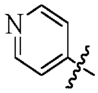, 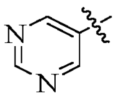, 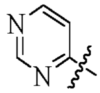 or 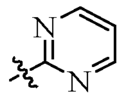,

each of which is optionally substituted by 1-3 $Q_1$; preferably, $R^1$ is selected from

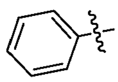, 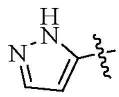, 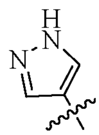, 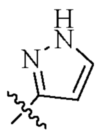, 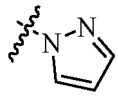, 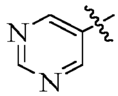, 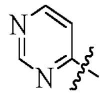 or 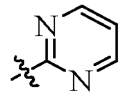,

each of which is optionally substituted by 1-3 $Q_1$.

Solution 9: the compound or the pharmaceutically acceptable salt, ester, deuterated compound or stereoisomer thereof according to any of the preceding solutions, has a structure of general formula (III):

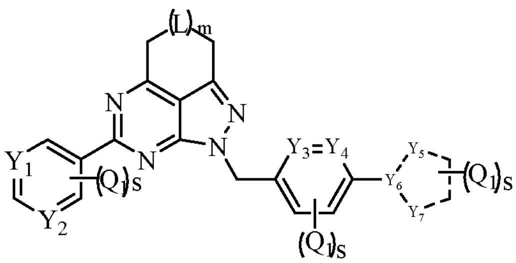

formula (III)

wherein:

- $Y_1$, $Y_2$, $Y_3$, $Y_4$ and $Y_6$ are each independently selected from N, C or $CR^a$;
- $Y_5$ and $Y_7$ are each independently selected from N, $NR^c$, C, $CR^aR^b$ or $CR^a$;
- -- and - is each independently selected from a single bond or a double bond, and two adjacent bonds are incapable of being double bonds at the same time;
- each s is independently selected from an integer of 0-2; and
- each $Q_1$, m, each L, each $R^a$, each $R^b$ and each $R^c$ are as defined in any of the preceding solutions.

Solution 9-1: the compound or the pharmaceutically acceptable salt, ester, deuterated compound or stereoisomer thereof according to any of the preceding solutions, has a structure of general formula (III-1):

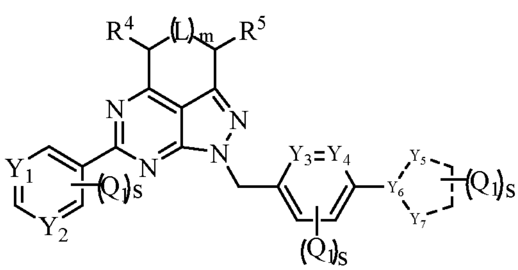

formula (III-1)

wherein:

- $Y_1$, $Y_2$, $Y_3$, $Y_4$ and $Y_6$ are each independently selected from N, C or $CR^a$,
- $Y_5$ and $Y_7$ are each independently selected from N, $NR^c$, C, $CR^aR^b$ or $CR^a$;

-- and - is each independently selected from a single bond or a double bond, and two adjacent bonds are incapable of being double bonds at the same time;

each s is independently selected from an integer of 0-3; and

each $Q_1$, m, each L, each $R^a$, each $R^b$, each $R^c$, $R^4$ and $R^5$ are as defined in any of the preceding solutions.

Solution 9-2: the compound or the pharmaceutically acceptable salt, ester, deuterated compound or stereoisomer thereof according to any of the preceding solutions, has a structure of general formula (III-1'):

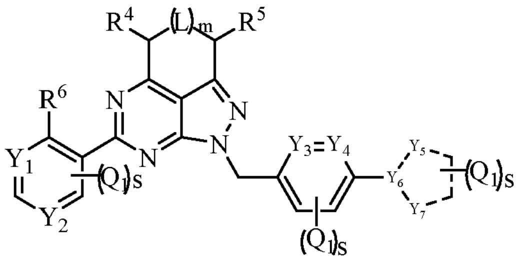

formula (III-1')

wherein, $R^6$ is selected from hydrogen, or $C_{1-6}$ alkyl, $C_{1-6}$ alkoxy, halogenated $C_{1-6}$ alkyl and halogenated $C_{1-6}$ alkoxy, each of which is optionally deuterated;

$Y_1$, $Y_2$, $Y_3$, $Y_4$ and $Y_6$ are each independently selected from N, C or $CR^a$;

$Y_5$ and $Y_7$ are each independently selected from N, $NR^c$, C, $CR^aR^b$ or $CR^a$;

-- and - is each independently selected from a single bond or a double bond, and two adjacent bonds are incapable of being double bonds at the same time;

each s is independently selected from an integer of 0-3; and

each $Q_1$, m, each L, each $R^a$, each $R^b$, each $R^c$, $R^4$ and $R^5$ are as defined in any of the preceding solutions.

Solution 9-3: the compound or the pharmaceutically acceptable salt, ester, deuterated compound or stereoisomer thereof according to any of the preceding solutions, wherein $R^6$ is selected from hydrogen, deuterated $C_{1-6}$ alkyl or deuterated $C_{1-6}$ alkoxy.

Solution 9-4: the compound or the pharmaceutically acceptable salt, ester, deuterated compound or stereoisomer thereof according to any of the preceding solutions, wherein:

$Y_1$, $Y_2$, $Y_3$, $Y_4$ and $Y_6$ are each independently selected from N, C or CH; and

$Y_5$ and $Y_7$ are each independently selected from N, NH, C, $CH_2$ or CH.

Solution 9-5: the compound or the pharmaceutically acceptable salt, ester, deuterated compound or stereoisomer thereof according to any of the preceding solutions, wherein:

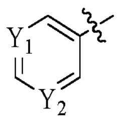

is selected from the following structures:

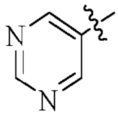

,

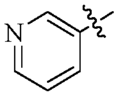

or

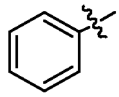

.

Solution 9-6: the compound or the pharmaceutically acceptable salt, ester, deuterated compound or stereoisomer thereof according to any of the preceding solutions, wherein:

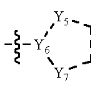

is selected from the following structures:

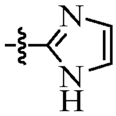 , 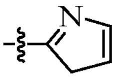 , 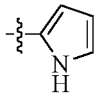 , 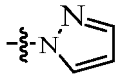 ,

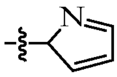 or 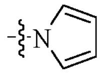 .

Solution 10: the compound or the pharmaceutically acceptable salt, ester, deuterated compound or stereoisomer thereof according to any of the preceding solutions, has a structure of general formula (IV):

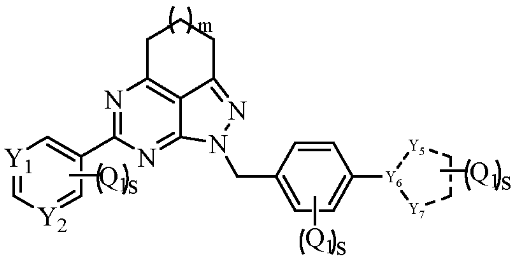

formula (IV)

wherein:

each $Q_1$ and m are as defined in any of the preceding solutions,
-- and - is each independently selected from a single bond or a double bond, and two adjacent bonds are incapable of being double bonds at the same time; and
$Y_1$, $Y_2$, $Y_5$, $Y_6$, $Y_7$ and each s are as defined in any of the preceding solutions.

Solution 10-1: the compound or the pharmaceutically acceptable salt, ester, deuterated compound or stereoisomer thereof according to any of the preceding solutions, has a structure of general formula (IV-1):

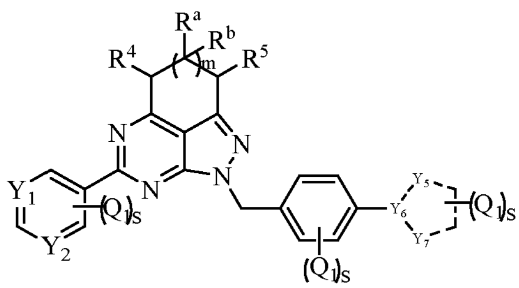

formula (IV-1)

wherein:

-- and - is each independently selected from a single bond or a double bond, and two adjacent bonds are incapable of being double bonds at the same time; and
each $Q_1$, m, $R^4$, $R^5$, each $R^a$, each $R^b$, $Y_1$, $Y_2$, $Y_5$, $Y_6$, $Y_7$ and each s are as defined in any of the preceding solutions.

Solution 10-2: the compound or the pharmaceutically acceptable salt, ester, deuterated compound or stereoisomer thereof according to any of the preceding solutions, has a structure of general formula (IV-1'):

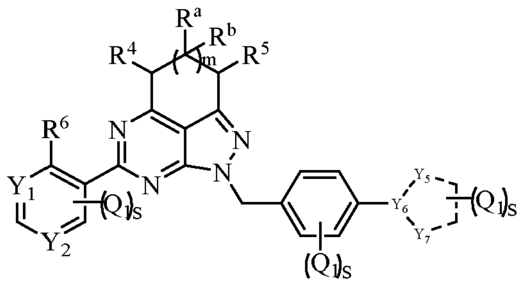

formula (IV-1')

wherein, $R^6$ is selected from hydrogen, or optionally deuterated $C_{1-6}$ alkyl, $C_{1-6}$ alkoxy, halogenated $C_{1-6}$ alkyl and halogenated $C_{1-6}$ alkoxy;

Yi and $Y_2$ are each independently selected from N, C or $CR^a$;

$Y_6$ is selected from N, C or $CR^a$;

$Y_5$ and $Y_7$ are each independently selected from N, $NR^c$, $CR^aR^b$ or $CR^a$;

-- and - is each independently selected from a single bond or a double bond, and two adjacent bonds are incapable of being double bonds at the same time; and

each s is independently selected from an integer of 0-2.

Solution 10-3: the compound or the pharmaceutically acceptable salt, ester, deuterated compound or stereoisomer thereof according to any of the preceding solutions, wherein $R^6$ is selected from hydrogen, deuterated $C_{1-6}$ alkyl or deuterated $C_{1-6}$ alkoxy.

Solution 10-4: the compound or the pharmaceutically acceptable salt, ester, deuterated compound or stereoisomer thereof according to any of the preceding solutions, wherein $R^a$ and $R^b$ are each independently selected from hydrogen.

Solution 11: the compound or the pharmaceutically acceptable salt, ester, deuterated compound or stereoisomer thereof according to any of the preceding solutions, has a structure of general formula (V):

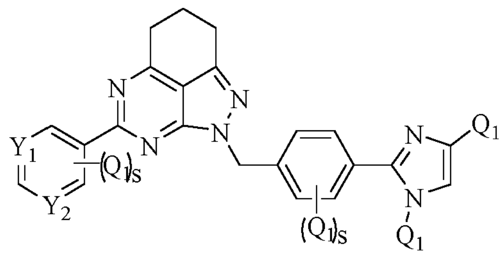

formula (V)

wherein:

each $Q_1$, $Y_1$, $Y_2$ and each s are as defined in any of the preceding solutions.

Solution 11-1: the compound or the pharmaceutically acceptable salt, ester, deuterated compound or stereoisomer thereof according to any of the preceding solutions, has a structure of general formula (V-1):

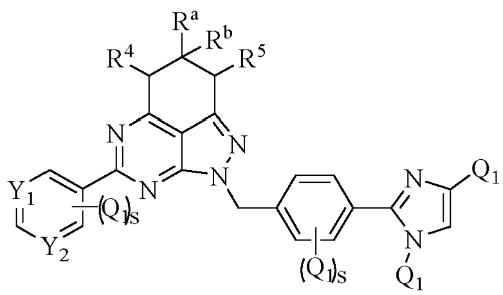

formula (V-1)

wherein:

each $Q_1$, $R^4$, $R^5$, $R^a$, $R^b$, $Y_1$, $Y_2$ and each s are as defined in any of the preceding solutions.

Solution 11-2: the compound or the pharmaceutically acceptable salt, ester, deuterated compound or stereoisomer thereof according to any of the preceding solutions, has a structure of general formula (V-1'):

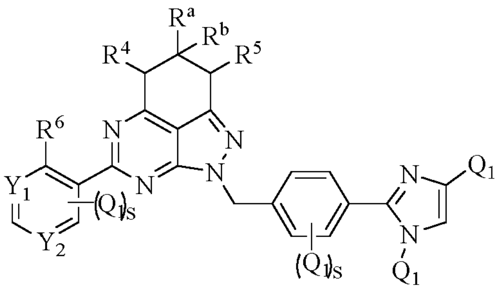

formula (V-1')

wherein, $R^6$ is selected from hydrogen, or from $C_{1-6}$ alkyl, $C_{1-6}$ alkoxy, halogenated $C_{1-6}$ alkyl and halogenated $C_{1-6}$ alkoxy, each of which is optionally deuterated; and

each $Q_1$, $R^4$, $R^5$, $R^a$, $R^b$, $Y_1$, $Y_2$ and each s are as defined in any of the preceding solutions.

Solution 11-3: the compound or the pharmaceutically acceptable salt, ester, deuterated compound or stereoisomer thereof according to any of the preceding solutions, wherein $R^6$ is selected from hydrogen, deuterated $C_{1-6}$ alkyl or deuterated $C_{1-6}$ alkoxy.

Solution 12: the compound or the pharmaceutically acceptable salt, ester, deuterated compound or stereoisomer thereof according to any of the preceding solutions, wherein:

each $Q_1$ is independently selected from deuterium, halogen, or from $C_{1-6}$ alkyl, $C_{1-6}$ alkoxy, $C_{1-6}$ alkylamino, di($C_{1-6}$ alkyl) amino, halogenated $C_{1-6}$ alkyl, halogenated $C_{1-6}$ alkoxy, hydroxy $C_{1-6}$ alkyl, amino $C_{1-6}$ alkyl, carboxyl $C_{1-6}$ alkyl, $-(L)_m$-3-6 membered cycloalkyl, or $-(L)_m$-3-6 membered heterocyclyl, each of which is optionally substituted by 1-4 substituents $Q_2$; and each $Q_2$ is independently selected from deuterium, halogen, carboxyl, hydroxyl, cyano, nitro, amino, $C_{1-6}$ alkyl, $C_{1-6}$ alkoxy, halogenated $C_{1-6}$ alkyl and halogenated $C_{1-6}$ alkoxy.

Solution 12-1: the compound or the pharmaceutically acceptable salt, ester, deuterated compound or stereoisomer thereof according to any of the preceding solutions, wherein:

each $Q_1$ is independently selected from deuterium, halogen, or from $C_{1-6}$ alkyl, $C_{1-6}$ alkoxy, halogenated $C_{1-6}$ alkyl, halogenated $C_{1-6}$ alkoxy and 3-6 membered cycloalkyl, each of which is optionally substituted by 1-3 substituents $Q_2$; and each $Q_2$ is independently selected from deuterium, halogen, $C_{1-6}$ alkyl, $C_{1-6}$ alkoxy, halogenated $C_{1-6}$ alkyl and halogenated $C_{1-6}$ alkoxy.

Solution 13: the compound or the pharmaceutically acceptable salt, ester, deuterated compound or stereoisomer thereof according to any of the preceding solutions, wherein:

each $Q_1$ is independently selected from deuterium, halogen, or from $C_{1-4}$ alkyl, $C_{1-4}$ alkoxy, $C_{1-4}$ alkylamino, di($C_{1-4}$ alkyl) amino, halogenated $C_{1-4}$ alkyl, halogenated $C_{1-4}$ alkoxy, hydroxy $C_{1-4}$ alkyl, amino $C_{1-4}$ alkyl, carboxyl $C_{1-4}$ alkyl and 3-6 membered cycloalkyl, each of which is optionally substituted by 1-3 substituents $Q_2$; and each $Q_2$ is independently selected from deuterium, halogen, carboxyl, hydroxyl, cyano, nitro, amino, $C_{1-4}$ alkyl, $C_{1-4}$ alkoxy, halogenated $C_{1-4}$ alkyl and halogenated $C_{1-4}$ alkoxy.

Solution 13-1: the compound or the pharmaceutically acceptable salt, ester, deuterated compound or stereoisomer thereof according to any of the preceding solutions, wherein:

each $Q_1$ is independently selected from deuterium, halogen, or from $C_{1-4}$ alkyl, $C_{1-4}$ alkoxy, halogenated $C_{1-4}$ alkyl, halogenated $C_{1-4}$ alkoxy and 3-6 membered cycloalkyl, each of which is optionally substituted by 1-3 substituents $Q_2$; and each $Q_2$ is independently selected from deuterium, $C_{1-4}$ alkyl, $C_{1-4}$ alkoxy, halogenated $C_{1-4}$ alkyl and halogenated $C_{1-4}$ alkoxy.

Solution 14: the compound or the pharmaceutically acceptable salt, ester, deuterated compound or stereoisomer thereof according to any of the preceding solutions, wherein:

each $Q_1$ is independently selected from deuterium, fluorine, chlorine, bromine, iodine, or from methyl, ethyl, propyl, isopropyl, butyl, isobutyl, sec-butyl, tert-butyl, methoxy, ethoxy, propoxy, isopropoxy, methylamino, dimethylamino, monofluoromethyl, difluoromethyl, trifluoromethyl, monofluoromethoxy, difluoromethoxy, trifluoromethoxy, hydroxymethyl, hydroxyethyl, hydroxypropyl, hydroxybutyl, aminomethyl, carboxymethyl, carboxyethyl, cyclopropyl, cyclobutyl or cyclopentyl, each of which is optionally substituted by 1-3 substituents $Q_2$; and each $Q_2$ is independently selected from deuterium, halogen, carboxyl, hydroxyl, cyano, nitro, amino, methyl, ethyl, propyl, isopropyl, butyl, isobutyl, sec-butyl, tert-butyl, methoxy, ethoxy, propoxy, isopropoxy, monofluoromethyl, difluoromethyl, trifluoromethyl, monofluoromethoxy, difluoromethoxy or trifluoromethoxy.

Solution 14-1: the compound or the pharmaceutically acceptable salt, ester, deuterated compound or stereoisomer thereof according to any of the preceding solutions, wherein:

each $Q_1$ is independently selected from deuterium, fluorine, chlorine, bromine, iodine, or from methyl, ethyl, propyl, isopropyl, butyl, isobutyl, sec-butyl, tert-butyl, methoxy, ethoxy, propoxy, isopropoxy, monofluoromethyl, difluoromethyl,

trifluoromethyl, monofluoromethoxy, difluoromethoxy, trifluoromethoxy, cyclopropyl, cyclobutyl or cyclopentyl, each of which is optionally substituted by 1-3 substituents $Q_2$; and each $Q_2$ is independently selected from deuterium, fluorine, chlorine, bromine, iodine, methyl, ethyl, propyl, isopropyl, butyl, isobutyl, sec-butyl, tert-butyl, methoxy, ethoxy, propoxy, isopropoxy, monofluoromethyl, difluoromethyl, trifluoromethyl, monofluoromethoxy, difluoromethoxy and trifluoromethoxy.

Solution 15: the compound or the pharmaceutically acceptable salt, ester, deuterated compound or stereoisomer thereof according to any of the preceding solutions, wherein:

each L is independently selected from $-CR^aR^b-$; and
each $R^a$ and each $R^b$ are each independently selected from deuterium, hydrogen, or from $C_{1-4}$ alkyl, $C_{1-4}$ alkoxy, halogenated $C_{1-4}$ alkyl and halogenated $C_{1-4}$ alkoxy, each of which is optionally deuterated.

Solution 15-1: the compound or the pharmaceutically acceptable salt, ester, deuterated compound or stereoisomer thereof according to any of the preceding solutions, wherein:

each L is independently selected from $-CR^aR^b-$; and
each $R^a$ and each $R^b$ are each independently selected from deuterium, hydrogen, or from $C_{1-6}$ alkyl, $C_{1-6}$ alkoxy, halogenated $C_{1-6}$ alkyl and halogenated $C_{1-6}$ alkoxy, each of which is optionally deuterated.

Solution 16: the compound or the pharmaceutically acceptable salt, ester, deuterated compound or stereoisomer thereof according to any of the preceding solutions, wherein:
each L is independently selected from $-CH_2-$.

Solution 17: the compound or the pharmaceutically acceptable salt, ester, deuterated compound or stereoisomer thereof according to any of the preceding solutions, has a structure of general formula (VI):

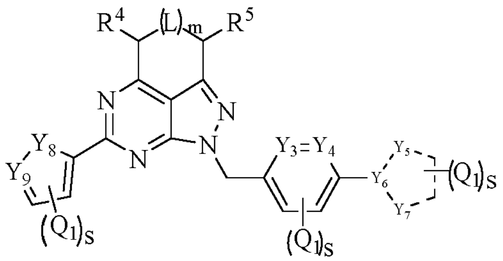

formula (VI)

$Y_8$ is selected from N, $NR^c$, C, $CR^aR^b$ or $CR^a$;
$Y_9$ is selected from N, C or $CR^a$;
-- and - is each independently selected from a single bond or a double bond, and two adjacent bonds are incapable of being double bonds at the same time; and
each $Q_1$, each L, m, $R^4$, $R^5$, $Y_3$, $Y_4$, $Y_5$, $Y_6$, $Y_7$ and each s are as defined in any of the preceding solutions.

Solution 17-1: the compound or the pharmaceutically acceptable salt, ester, deuterated compound or stereoisomer thereof according to the preceding solution 17, wherein:

$Y_8$ is selected from N, NH, C, $CH_2$ or CH;
$Y_9$ is selected from N, C or CH;
-- and - is each independently selected from a single bond or a double bond, and two adjacent bonds are incapable of being double bonds at the same time; and
each $Q_1$, each L, m, $R^4$, $R^5$, $Y_3$, $Y_4$, $Y_5$, $Y_6$, $Y_7$ and each s are as defined in any of the preceding solutions.

Solution 18: the compound or the pharmaceutically acceptable salt, the ester, deuterated compound or stereoisomer thereof according to any of the preceding solutions, has a structure of general formula (VII):

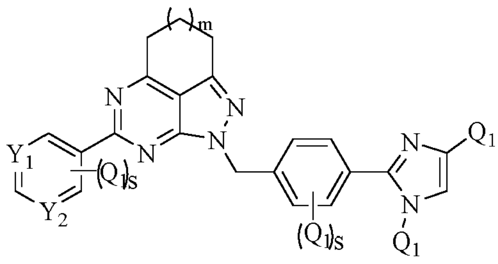

formula (VII)

wherein, each $Q_1$, m, $Y_1$, $Y_2$ and each s are as defined in any of the preceding solutions.

Solution 18-1: the compound or the pharmaceutically acceptable salt, ester, deuterated compound or stereoisomer thereof according to any of the preceding solutions, has a structure of general formula (VII-1):

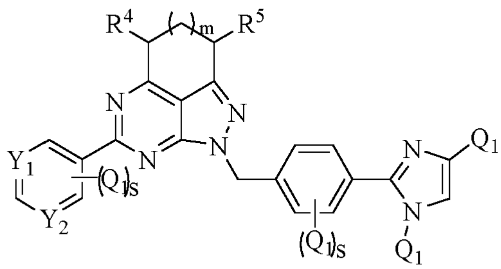

formula (VII-1)

wherein, each $Q_1$, m, $Y_1$, $Y_2$, each s, $R^4$ and $R^5$ are as defined in any of the preceding solutions.

Solution 18-2: the compound or the pharmaceutically acceptable salt, ester, deuterated compound or stereoisomer thereof according to any of the preceding solutions, has a structure of general formula (VII-1'):

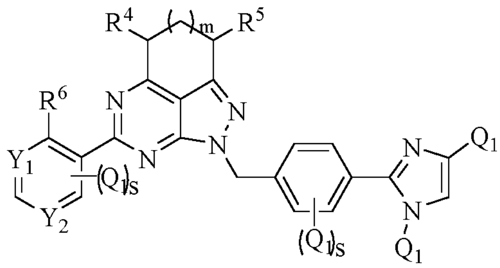

formula (VII-1')

wherein, $R^6$ is selected from hydrogen, or from $C_{1-6}$ alkyl, $C_{1-6}$ alkoxy, halogenated $C_{1-6}$ alkyl and halogenated $C_{1-6}$ alkoxy, each of which is optionally deuterated; and
each Qi, m, $Y_1$, $Y_2$, each s, $R^4$ and $R^5$ are as defined in any of the preceding solutions.

Solution 18-3: the compound or the pharmaceutically acceptable salt, ester, deuterated compound or stereoisomer thereof according to any of the preceding solutions, wherein $R^6$ is selected from hydrogen, deuterated $C_{1-6}$ alkyl or deuterated $C_{1-6}$ alkoxy.

Solution 19: the compound or the pharmaceutically acceptable salt, ester, deuterated compound or stereoisomer thereof according to any of the preceding solutions, has a structure of general formula (VIII):

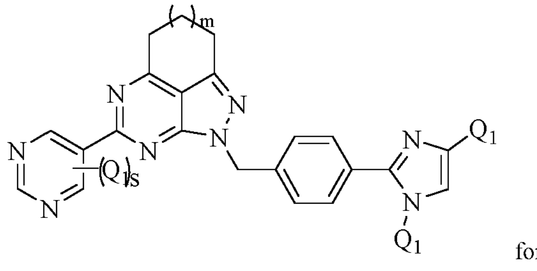

formula (VIII)

wherein, each $Q_1$, m and each s are as defined in any of the preceding solutions.

Solution 19-1: the compound or the pharmaceutically acceptable salt, ester, deuterated compound or stereoisomer thereof according to any of the preceding solutions, has a structure of general formula (VIII-1):

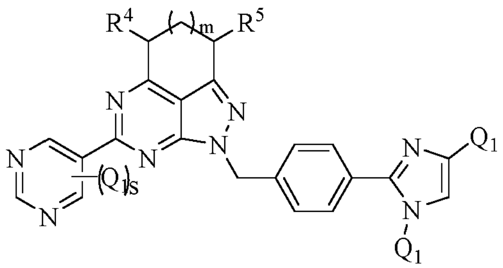

formula (VIII-1)

wherein, each $Q_1$, m, $R^4$, $R^5$ and each s are as defined in any of the preceding solutions.

Solution 19-2: the compound or the pharmaceutically acceptable salt, ester, deuterated compound or stereoisomer thereof according to any of the preceding solutions, has a structure of general formula (VIII-1'):

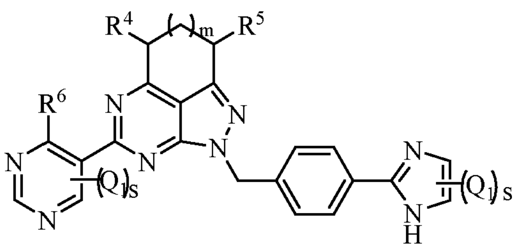

formula (VIII-1')

wherein, $R^6$ is selected from hydrogen, or from $C_{1-6}$ alkyl, $C_{1-6}$ alkoxy, halogenated $C_{1-6}$ alkyl and halogenated $C_{1-6}$ alkoxy, each of which is optionally deuterated; and
each Qi, m, $R^4$, $R^5$ and each s are as defined in any of the preceding solutions.

Solution 19-3: the compound or the pharmaceutically acceptable salt, ester, deuterated compound or stereoisomer thereof according to any of the preceding solutions, wherein $R^6$ is selected from hydrogen, deuterated $C_{1-6}$ alkyl or deuterated $C_{1-6}$ alkoxy.

Solution 20: the compound or the pharmaceutically acceptable salt, ester, deuterated compound or stereoisomer thereof according to any of the preceding solutions, has a structure of general formula (IX):

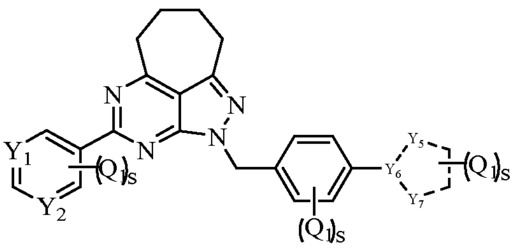

formula (IX)

-- and - is each independently selected from a single bond or a double bond, and two adjacent bonds are incapable of being double bonds at the same time; and
each $Q_1$, $Y_1$, $Y_2$, $Y_5$, $Y_6$, $Y_7$ and each s are as defined in any of the preceding solutions.

Solution 20-1: the compound or the pharmaceutically acceptable salt, ester, deuterated compound or stereoisomer thereof according to any of the preceding solutions, has a structure of general formula (IX-1):

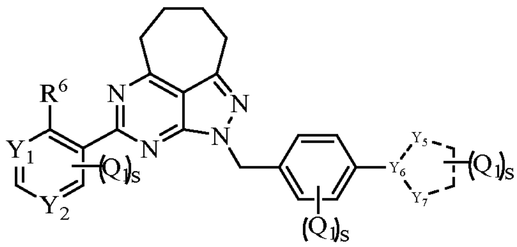

formula (IX-1)

wherein, $R^6$ is selected from hydrogen, or optionally deuterated $C_{1-6}$ alkyl, $C_{1-6}$ alkoxy, halogenated $C_{1-6}$ alkyl and halogenated $C_{1-6}$ alkoxy,

-- and - is each independently selected from a single bond or a double bond, and two adjacent bonds are incapable of being double bonds at the same time; and

each $Q_1$, $Y_1$, $Y_2$, $Y_5$, $Y_6$, $Y_7$ and each s are as defined in any of the preceding solutions.

Solution 20-2: the compound or the pharmaceutically acceptable salt, ester, deuterated compound or stereoisomer thereof according to any of the preceding solutions, where $R^6$ is selected from hydrogen, or optionally deuterated $C_{1-6}$ alkyl or deuterated $C_{1-6}$ alkoxy.

Solution 21: the compound or the pharmaceutically acceptable salt, ester, deuterated compound or stereoisomer thereof according to any of the preceding solutions, where:

$R^4$, $R^5$, each $R^a$, each $R^b$ and each $R^c$ are each independently selected from hydrogen.

Solution 22: the compound or the pharmaceutically acceptable salt, ester, deuterated compound or stereoisomer thereof according to any of the preceding solutions, has a structure of general formula (II-2):

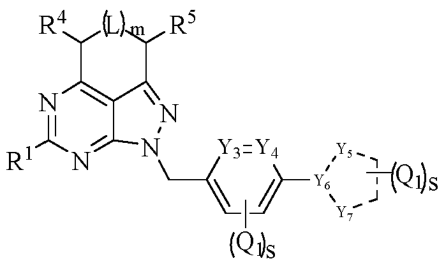

formula (II-2)

wherein, $Y_3$, $Y_4$ and $Y_6$ are each independently selected from N, C or $CR^a$;

$Y_5$ and $Y_7$ are each independently selected from N, $NR^c$, C, $CR^aR^b$ or $CR^a$;

-- and - is each independently selected from a single bond or a double bond, and two adjacent bonds are incapable of being double bonds at the same time;

each s is independently selected from an integer of 0-2; and

each $Q_1$, each $Q_2$, each L, m, $R^1$, $R^4$, $R^5$, each $R^a$, each $R^b$ and each $R^c$ are as defined in any of the preceding solutions.

Solution 23: the compound or the pharmaceutically acceptable salt, ester, deuterated compound or stereoisomer thereof according to any of the preceding solutions, has a structure of general formula (II-3):

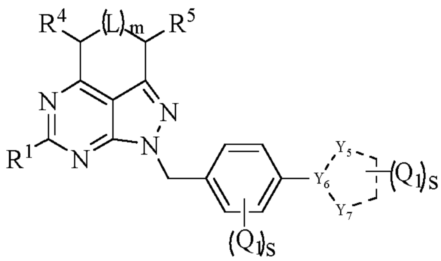

formula (II-3)

each $Q_1$, each $Q_2$, each s, each L, m, $R^1$, $R^4$, $R^5$, *Ys*, $Y_6$, $Y_7$, each $R^a$, each $R^b$ and each $R^c$ are as defined in any of the preceding solutions.

Solution 24: the compound or the pharmaceutically acceptable salt, ester, deuterated compound or stereoisomer thereof according to any of the preceding solutions, has a structure of general formula (II-3):

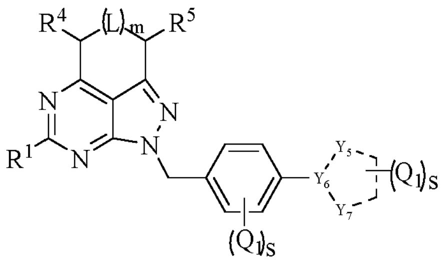

formula (II-3)

$R^1$ is selected from phenyl or 5-6 membered nitrogen-containing heteroaryl, each of which is optionally substituted by 1-3 $Q_1$; preferably, $R^1$ is selected from

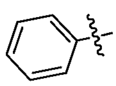 , 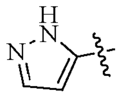 , 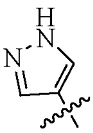 , 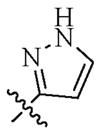 , 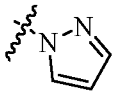 , 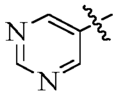 ,

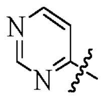 or 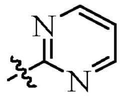

optionally substituted by 1-3 $Q_1$;

$R^4$ and $R^5$ are each independently selected from deuterium, hydrogen, or from $C_{1-6}$ alkyl, $C_{1-6}$ alkoxy, halogenated $C_{1-6}$ alkyl or halogenated $C_{1-6}$ alkoxy, each of which is optionally deuterated;

each L is independently selected from $-CR^aR^b-$;

each $R^a$ and each $R^b$ are each independently selected from deuterium, hydrogen, or from $C_{1-6}$ alkyl, $C_{1-6}$ alkoxy, halogenated $C_{1-6}$ alkyl or halogenated $C_{1-6}$ alkoxy, each of which is optionally deuterated;

each $Q_1$ is independently selected from deuterium, halogen, or from $C_{1-6}$ alkyl, $C_{1-6}$ alkoxy, halogenated $C_{1-6}$ alkyl, halogenated $C_{1-6}$ alkoxy or 3-6 membered cycloalkyl, each of which is optionally substituted by 1-3 substituents $Q_2$; and each $Q_2$ is independently selected from deuterium, halogen, $C_{1-6}$ alkyl, $C_{1-6}$ alkoxy, halogenated $C_{1-6}$ alkyl and halogenated $C_{1-6}$ alkoxy;

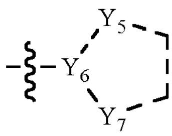

is selected from the following structure:

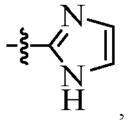 , 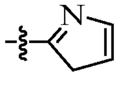 , 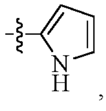 , 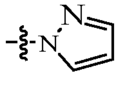 ,

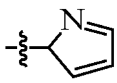 or 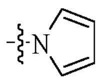 ;

m is an integer of 1 or 2; and

each s is independently an integer of 0, 1 and 2.

Solution 25: the compound or the pharmaceutically acceptable salt, ester, deuterated compound or stereoisomer

thereof according to any of the preceding solutions, has a structure of general formula (II-4):

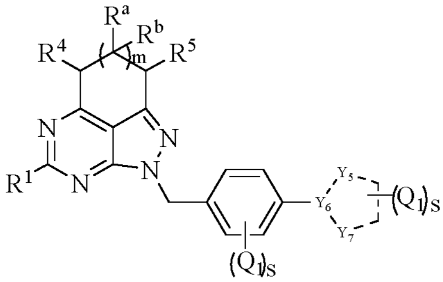

formula (II-4)

each $Q_1$, each $Q_2$, each s, m, $R^1$, $R^4$, $R^5$, Ys, $Y_6$, $Y_7$, each $R^a$, each $R^b$ and each $R^c$ are as defined in any of the preceding solutions.

Solution 26: the compound or the pharmaceutically acceptable salt, ester, deuterated compound or stereoisomer thereof according to any of the preceding solutions, has a structure of general formula (II-4):

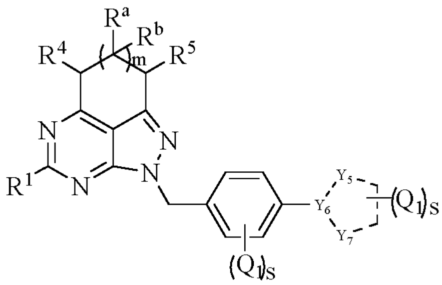

formula (II-4)

wherein, $R^1$ is selected from phenyl or 5-6 membered nitrogen-containing heteroaryl, each of which is optionally substituted by 1-3 $Q_1$; preferably, $R^1$ is selected from

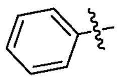 , 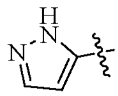 , 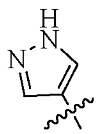 , 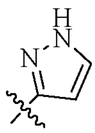 , 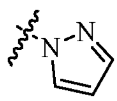 ,

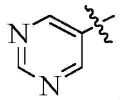 , 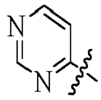 or 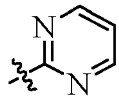 ,

each of which is optionally substituted by 1-3 $Q_1$;

$R_4$ and $R_5$ are each independently selected from deuterium, hydrogen, or optionally deuterated $C_{1-6}$ alkyl, $C_{1-6}$ alkoxy, halogenated $C_{1-6}$ alkyl and halogenated $C_{1-6}$ alkoxy;

each $Q_1$ is independently selected from deuterium, halogen, or from $C_{1-6}$ alkyl, $C_{1-6}$ alkoxy, halogenated $C_{1-6}$ alkyl, halogenated $C_{1-6}$ alkoxy and 3-6 membered cycloalkyl, each of which is optionally substituted by 1-3 substituents $Q_2$; and each $Q_2$ is independently selected from deuterium, halogen, $C_{1-6}$ alkyl, $C_{1-6}$ alkoxy, halogenated $C_{1-6}$ alkyl and halogenated $C_{1-6}$ alkoxy,

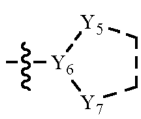

is selected from the following structure:

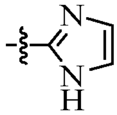, 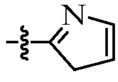, 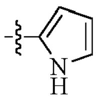, 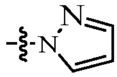,

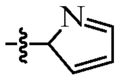 or 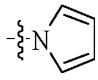;

m is an integer of 1 or 2; and
each s is independently an integer of 0, 1 and 2.

Solution 27: the compound or the pharmaceutically acceptable salt, ester, deuterated compound or stereoisomer thereof according to any of the preceding solutions, has a structure of general formula (II-5):

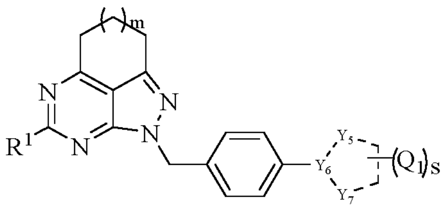
formula (II-5)

each $Q_1$, each $Q_2$, s, m, $R^1$, $Y_5$, $Y_6$, $Y_7$, each $R^a$, each $R^b$ and each $R^c$ are as defined in any of the preceding solutions.

Solution 28: the compound or the pharmaceutically acceptable salt, ester, deuterated compound or stereoisomer thereof according to any of the preceding solutions, has a structure of general formula (II-5):

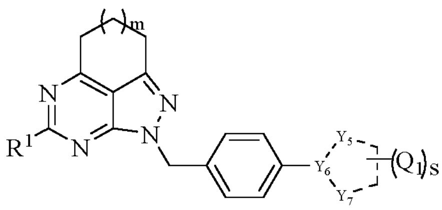
formula (II-5)

$R^1$ is selected from phenyl or 5-6 membered nitrogen-containing heteroaryl optionally substituted by 1-3 $Q_1$; preferably, $R^1$ is selected from

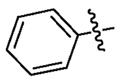, 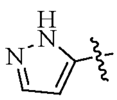, 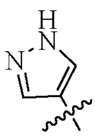, 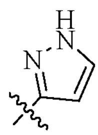, 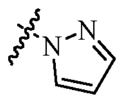, 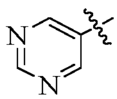, 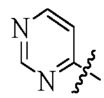,

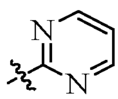, 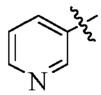, 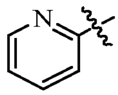 or 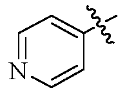,

each of which is optionally substituted by 1-3 $Q_1$;
each $Q_1$ is independently selected from deuterium, halogen, or from $C_{1-6}$ alkyl, $C_{1-6}$ alkoxy, halogenated $C_{1-6}$ alkyl, halogenated $C_{1-6}$ alkoxy or 3-6 membered cycloalkyl, each of which is optionally substituted by 1-3 substituents $Q_2$;
and each $Q_2$ is independently selected from deuterium, $C_{1-6}$ alkyl, $C_{1-6}$ alkoxy, halogenated $C_{1-6}$ alkyl and halogenated $C_{1-6}$ alkoxy,

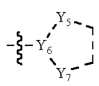

is selected from the following structure:

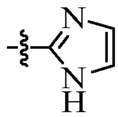, 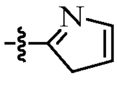, 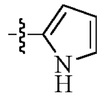, 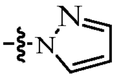,

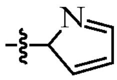 or 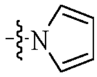;

m is an integer of 1 or 2; and
each s is independently an integer of 0, 1 or 2.

Solution 29: the compound or the pharmaceutically acceptable salt, ester, deuterated compound or stereoisomer thereof according to any of the preceding solutions, has a structure of general formula (IV-1'):

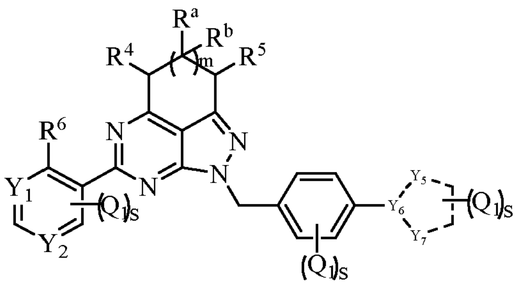

formula (IV-1')

wherein, $Y_1$ and $Y_2$ are each independently selected from N, C or CH;
$Y_5$ and $Y_7$ are each independently selected from NH, N, C, CH or $CH_2$;
$Y_6$ is selected from N, CH or C;
$R^4$ and $R^5$ are each independently selected from deuterium, hydrogen, cyano, halogen, or $C_{1-6}$ alkyl, $C_{1-6}$ alkoxy, $C_{1-6}$ alkylamino, di($C_{1-6}$ alkyl)amino, halogenated $C_{1-6}$ alkyl, hydroxy $C_{1-6}$ alkyl, amino $C_{1-6}$ alkyl, carboxyl $C_{1-6}$ alkyl or halogenated $C_{1-6}$ alkoxy, each of which is optionally deuterated;
$R^6$ is selected from hydrogen and deuterated $C_{1-6}$ alkoxy;
each $Q_1$ is independently selected from deuterium, halogen, or from $C_{1-6}$ alkyl, $C_{1-6}$ alkoxy, $C_{1-6}$ alkylamino, di($C_{1-6}$ alkyl)amino, halogenated $C_{1-6}$ alkyl, halogenated $C_{1-6}$ alkoxy, hydroxy $C_{1-6}$ alkyl, amino $C_{1-6}$ alkyl, carboxyl $C_{1-6}$ alkyl, $-(L)_m$-3-6 membered cycloalkyl or $-(L)_m$-3-6 membered heterocyclyl, each of which is optionally substituted by 1-3 substituents $Q_2$;
$R^a$ and $R^b$ are each independently selected from hydrogen;
each $Q_2$ is independently selected from deuterium, hydrogen, $C_{1-6}$ alkyl, $C_{1-6}$ alkoxy, halogenated $C_{1-6}$ alkyl and halogenated $C_{1-6}$ alkoxy;
-- and - is each independently selected from a single bond or a double bond, and two adjacent bonds are incapable of being double bonds at the same time; and
each m and each s are independently 0, 1 or 2.

Solution 30: the compound or the pharmaceutically acceptable salt, ester, deuterated compound or stereoisomer thereof according to any of the preceding solutions, has a structure of general formula (VIII-1'):

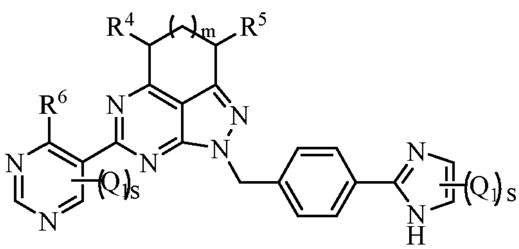

formula (VIII-1')

each $Q_1$ is independently selected from deuterium, halogen, or from $C_{1-4}$ alkyl, $C_{1-4}$ alkoxy, $C_{1-4}$ alkylamino, di($C_{1-4}$ alkyl)amino, halogenated $C_{1-4}$ alkyl, halogenated $C_{1-4}$ alkoxy, hydroxy $C_{1-4}$ alkyl, amino $C_{1-4}$ alkyl, carboxyl $C_{1-4}$ alkyl or 3-6 membered cycloalkyl, each of which is optionally substituted by 1-3 substituents $Q_2$;

each $Q_2$ is independently selected from deuterium, hydrogen, $C_{1-4}$ alkyl, $C_{1-4}$ alkoxy, halogenated $C_{1-4}$ alkyl or halogenated $C_{1-4}$ alkoxy;

$R^4$ and $R^5$ are each independently selected from deuterium, hydrogen, cyano, halogen, or from $C_{1-6}$ alkyl, $C_{1-6}$ alkoxy, halogenated $C_{1-6}$ alkyl or halogenated $C_{1-6}$ alkoxy, each of which is optionally deuterated;

$R^6$ is selected from hydrogen and deuterated $C_{1-4}$ alkoxy;

m is 1 or 2; and

each s is independently 0, 1 or 2.

Solution 31: the compound or the pharmaceutically acceptable salt, ester, deuterated compound or stereoisomer thereof according to any of the preceding solutions, wherein $R^6$ is selected from hydrogen, methyl, ethyl, propyl, isopropyl, butyl, isobutyl, sec-butyl, tert-butyl, methoxy, ethoxy, propoxy, isopropoxy, monofluoromethyl, difluoromethyl, trifluoromethyl, monofluoromethoxy, difluoromethoxy, trifluoromethoxy, deuterated methyl, deuterated ethyl, deuterated propyl, deuterated isopropyl, deuterated butyl, deuterated isobutyl, deuterated sec-butyl, deuterated tert-butyl, deuterated methoxy, deuterated ethoxy, deuterated propoxy or deuterated isopropoxy, and a number of deuteration is 1, 2, 3 or 4.

Solution 32: the compound or the pharmaceutically acceptable salt, ester, deuterated compound or stereoisomer thereof according to any of the preceding solutions, wherein:

$R^6$ is selected from deuterated methoxy, deuterated ethoxy, deuterated propoxy or deuterated isopropoxy, a number of deuteration is 1, 2 or 3.

The selection of any substituent in any embodiment of the present invention may be combined with each other, and the combined new technical solution is encompassed within the protection scope of the present invention.

In some embodiments of the present invention, the structure of the compound of general formula (I) or the pharmaceutically acceptable salt, ester, deuterated compound or stereoisomer thereof is shown in Table 1:

Table 1

| No. | Structure | No. | Structure |
|---|---|---|---|
| Compound 1 | 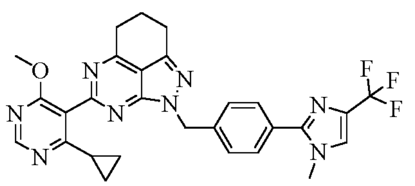 | Compound 2 | 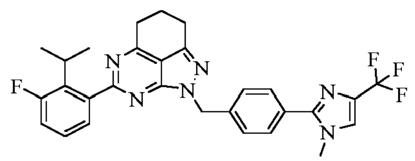 |
| Compound 3 | 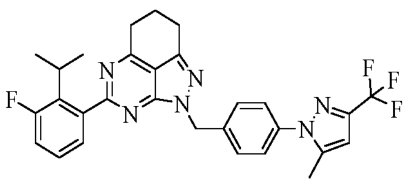 | Compound 4 | 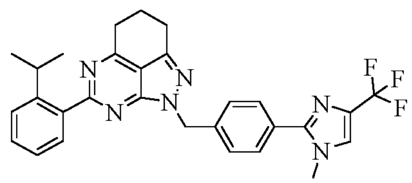 |
| Compound 5 | 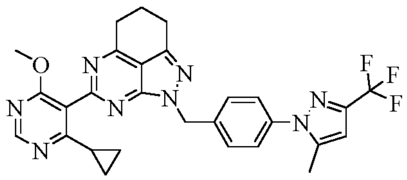 | Compound 6 | 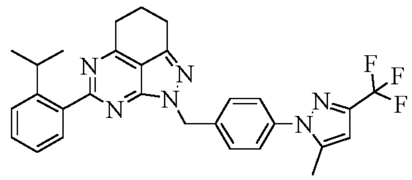 |
| Compound 7 | 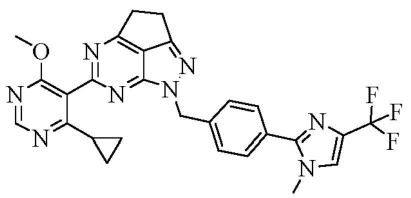 | Compound 8 | 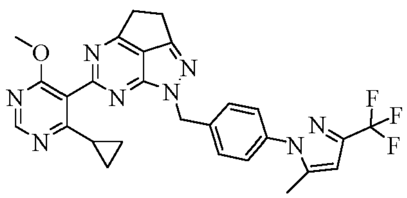 |

(continued)

| No. | Structure | No. | Structure |
|---|---|---|---|
| Compound 9 | 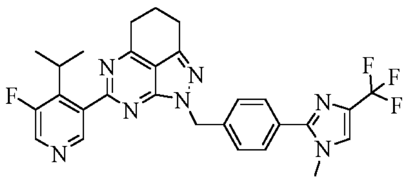 | Compound 10 | 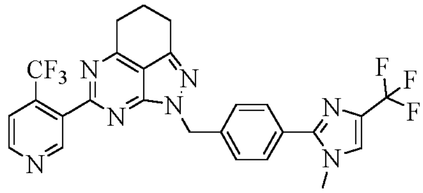 |
| Compound 11 | 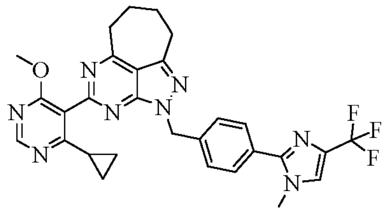 | Compound 12 | 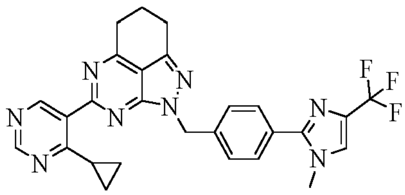 |
| Compound 13 | 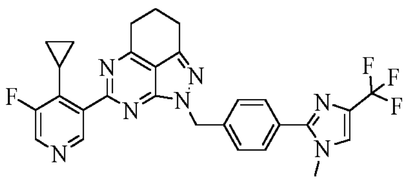 | Compound 14 | 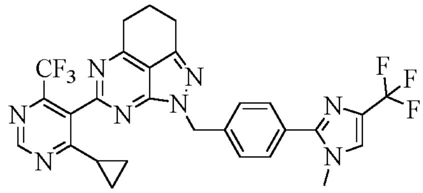 |
| Compound 15 | 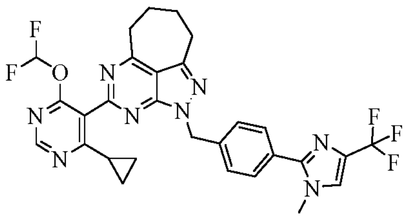 | Compound 16 | 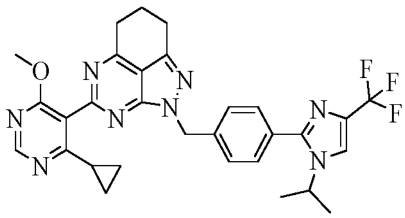 |
| Compound 17 | 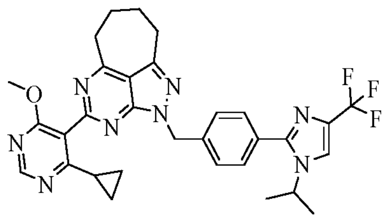 | Compound 18 | 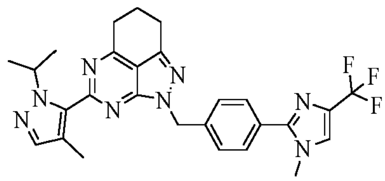 |
| Compound 19 | 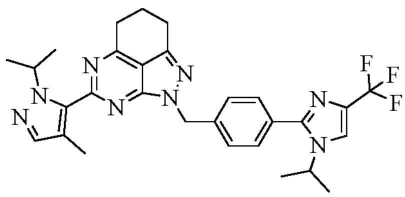 | Compound 20 | 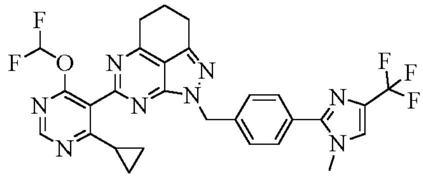 |
| Compound 1-1 | 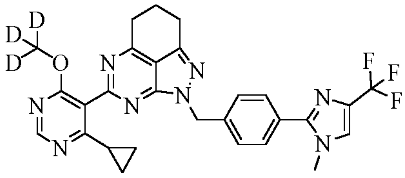 | Compound 5-1 | 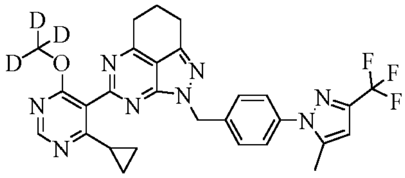 |

(continued)

| No. | Structure | No. | Structure |
|---|---|---|---|
| Compound 7-1 | 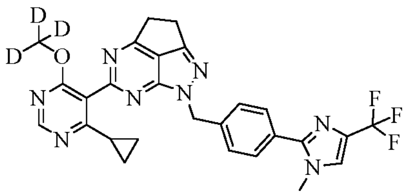 | Compound 8-1 | 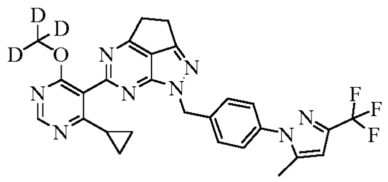 |
| Compound 11-1 | 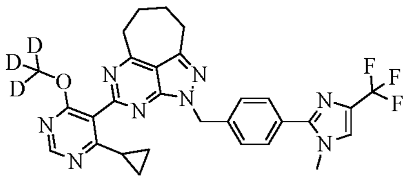 | Compound 16-1 | 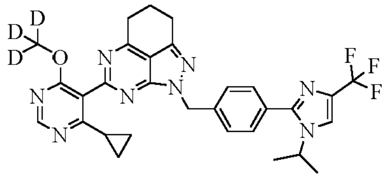 |
| Compound 17-1 | 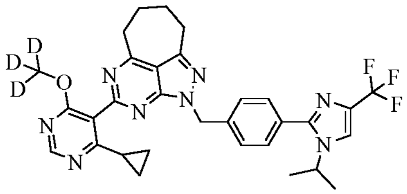 | | |

The "pharmaceutically acceptable salt" according to the present invention refers to an addition salt of a pharmaceutically acceptable acid and a base, such as a metal salt, an ammonium salt, a salt formed with an organic acid, a salt formed with an organic base, a salt formed with an inorganic acid, a salt formed with an acidic amino acid or a basic amino acid, etc.

The "ester" according to the present invention refers to a pharmaceutically acceptable ester, in particular to the following ester which is hydrolyzed in vivo and includes an ester of a compound or a salt thereof that is easily decomposed in a human body to leave a parent compound (the compound of general formula (I)) or a salt thereof. The "ester" according to the present invention, for example, may be selected from the following groups: (1) a carboxylic ester obtained by esterification with a carboxylic acid compound, where a non-carbonyl part of the carboxylic acid compound is selected from, for example, $C_{1-20}$ linear or branched alkyl, $C_{1-12}$ linear or branched alkyl, $C_{1-8}$ linear or branched alkyl, $C_{1-6}$ linear or branched alkyl (such as methyl, ethyl, n-propyl, tert-butyl or n-butyl), $C_{1-6}$ alkoxy $C_{1-6}$ alkyl (such as methoxymethyl) and $C_{6-10}$ aryl $C_{1-6}$ alkyl (such as benzyl), $C_{6-10}$ aryloxy $C_{1-6}$ alkyl (such as phenoxymethyl), and $C_{6-10}$ aryl (such as phenyl, optionally substituted by, for example, halogen, $C_{1-4}$ alkyl or $C_{1-4}$ alkoxy or amino); (2) a sulfonic acid ester, such as alkylsulfonyl or aralkylsulfonyl (such as methylsulfonyl); (3) an amino acid ester (such as L-valinyl or L-isoleucyl); (4) mono-,di-or triphosphate, and the like; and (5) an ester obtained by esterification with an alcohol compound, where a non-hydroxyl part of the alcohol compound is selected from, for example, $C_{1-20}$ linear or branched alkyl, $C_{1-12}$ linear or branched alkyl, $C_{1-8}$ linear or branched alkyl, $C_{1-6}$ linear or branched alkyl (such as methyl, ethyl, n-propyl, tert-butyl or n-butyl), $C_{1-6}$ alkoxy $C_{1-6}$ alkyl (such as methoxymethyl) and $C_{6-10}$ aryl $C_{1-6}$ alkyl (such as benzyl), $C_{6-10}$ aryloxy $C_{1-6}$ alkyl (such as phenoxymethyl), and $C_{6-10}$ aryl (such as phenyl, optionally substituted by, for example, halogen, $C_{1-4}$ alkyl or $C_{1-4}$ alkoxy or amino).

The "stereoisomer" of the compound of general formula (I) in the present invention refers to an enantiomer generated when the compound of formula (I) has an asymmetric carbon atom; a cis and trans isomer generated when the compound has a carbon-carbon double bond or a ring structure; and a tautomer generated when the compound has ketone or oxime. In some embodiments of the present invention, the stereoisomer includes, but is not limited to, an enantiomer, a diastereomer, a racemate, a cis and trans isomer, a tautomer, a geometric isomer, an epimer and a mixture thereof.

The present invention also provides a pharmaceutical composition including the compound of general formula (I) or the pharmaceutically acceptable salt, ester, deuterated compound or stereoisomer thereof, and one or more second therapeutic active agents, and optionally, the pharmaceutical composition further includes one or more pharmaceutically acceptable carriers and/or diluents.

The present invention also provides a pharmaceutical formulation including the compound of general formula (I) or the pharmaceutically acceptable salt, ester, deuterated compound or stereoisomer thereof, and one or more pharmaceutically acceptable carriers and/or diluents. The pharmaceutical formulation is any dosage form that is clinically or pharmaceutically acceptable.

In some embodiments of the present invention, the pharmaceutical formulation described above may be administered to a patient or subject in need of such treatment in oral, parenteral, rectal, or pulmonary administration. When used for oral administration, the pharmaceutical composition may be made into an oral formulation, for example, may be made into a conventional oral solid formulation, such as a tablet, a capsule, a pill, a granule and the like; may also be made into an oral liquid formulation, such as an oral solution, an oral suspension, a syrup, and the like. When the pharmaceutical composition is made into an oral formulation, an appropriate filler, binder, disintegrant, lubricant, and the like, may be added. When used for parenteral administration, the above pharmaceutical formulation may also be made into an injection preparation, including an injection, a sterile powder for injection and a concentrated solution for injection. When the pharmaceutical formulation is made into an injection preparation, the preparation may be produced by a conventional method in the existing pharmaceutical field. When preparing the injection preparation, no additives need to be added, or appropriate additives may be added according to the properties of the drug. When used for rectal administration, the pharmaceutical composition may be made into a suppository, and the like. When used for pulmonary administration, the pharmaceutical composition may be made into an inhalant or a spray, and the like.

The pharmaceutically acceptable carriers and/or diluents that may be used in the pharmaceutical composition or the pharmaceutical formulation of the present invention may be any conventional carrier and/or diluent in the field of pharmaceutical formulations, and the selection of specific carrier and/or diluent will depend on the mode of administration or the type and state of diseases used for treating specific patients. Preparation methods of suitable pharmaceutical compositions for specific modes of administration are completely within the knowledge of those skilled in the pharmaceutical field. For example, the pharmaceutical carriers and/or diluents may include solvents, diluents, dispersants, suspending agents, surfactants, isotonic agents, thickeners, emulsifiers, binders, lubricants, stabilizers, hydration agents, emulsification accelerators, buffers, absorbents, colorants, ion exchangers, release agents, coating agents, correctants, antioxidants and the like, which are common in the pharmaceutical field. When necessary, flavoring agents, preservatives, sweeteners, and the like, may also be added to the pharmaceutical composition.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present invention also provides a use of the compound of general formula (I), the pharmaceutically acceptable salt, ester, deuterated compound or stereoisomer thereof, the above pharmaceutical formulation or the above pharmaceutical composition in the manufacture of a medicament for treating and/or preventing a disease mediated by USP1 and a disease related thereto. The disease mediated by USP1 and the disease related thereto are selected from cancer or benign tumor.

The present invention also provides a use of the compound of general formula (I), the pharmaceutically acceptable salt, ester, deuterated compound or stereoisomer thereof, the above pharmaceutical formulation or the above pharmaceutical composition for treating and/or preventing a disease mediated by USP1 and a disease related thereto. The disease mediated by USP1 and the disease related thereto are selected from cancer or benign tumor.

The present invention also disclose a method for treating a disease, but not forming part of the claimed invention. The method includes administering to a patient in need a therapeutically effective amount of the preceding compound of general formula (I), the pharmaceutically acceptable salt, ester, deuterated compound or stereoisomer thereof, the preceding pharmaceutical formulation or the preceding pharmaceutical composition, where the disease refers to a disease mediated by USP1 and a disease related thereto. The disease mediated by USP1 and the disease related thereto are selected from cancer or benign tumor.

The present invention provides a preparation method of the above compound, which includes the following method, and reaction equations of the preparation method are as follows:

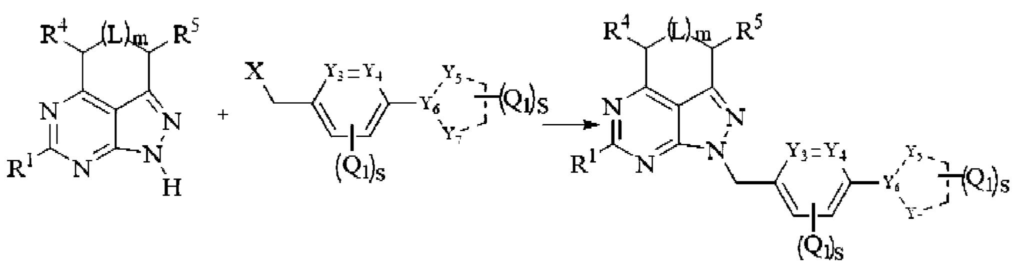

formula (II-2)-1 formula (II-2)-2 formula (II-2)

The compound of formula (II-2)-1 is dissolved in an appropriate solvent (such as DMF), added with an alkaline reagent (such as NaH, potassium carbonate, cesium carbonate **and** sodium carbonate), and then added with the compound of formula (II-2)-2 and reacted for 1-15 hours. The reaction system is added with water for quenching, extracted

with an appropriate organic solvent (such as dichloromethane **and** ethyl acetate), and spin-dried to obtain the compound of general formula (II-2) by an appropriate method (such as silica gel column chromatography).

The preparation method above may be simply summarized as: reacting the compound of formula (II-2)-1 with the compound of formula (II-2)-2 to obtain the compound of general formula (II-2);
wherein, X is halogen; and each $Q_1$, each $Q_2$, each L, m, $R^1$, $R^4$, $R^5$, $Y_3$, $Y_4$, $Y_5$, $Y_6$, $Y_7$, each $R^a$, each $R^b$ and each $R^c$ are as defined in any of the preceding technical solutions.

The present invention also provides an intermediate for preparing compounds of general formula (II-1) to general formula (IX-1), pharmaceutically acceptable salts, esters thereof, deuterated compounds thereof or stereoisomers thereof, which has the following structural formula:

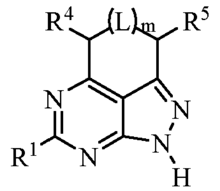

formula (II-2)-1

wherein, $R^1$, $R^4$, $R^5$, each $Q_1$, each $Q_2$, each L, each $R^a$, each $R^b$, each $R^c$ and m are as defined in any of the preceding technical solutions.

The present invention also provides an intermediate for preparing compounds of general formula (III) to general formula (IX-1), pharmaceutically acceptable salts, esters thereof, deuterated compounds thereof or stereoisomers thereof, which has the following structural formula:

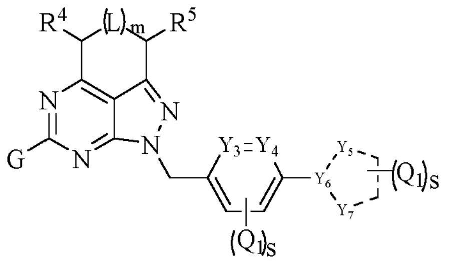

formula (II-2)-3

wherein, G is selected from halogen, hydroxyl, amino, $C_{1-6}$ alkylthio or $C_{1-6}$ alkylsulfonyl; and
each $Q_1$, each $Q_2$, each L, m, $R^1$, $R^4$, $R^5$, $Y_3$, $Y_4$, $Y_5$, $Y_6$, $Y_7$, each $R^a$, each $R^b$ and each $R^c$ are as defined in any of the preceding technical solutions.

In some embodiments, G is selected from halogen, hydroxyl, amino, methylthio or methylsulfonyl.

In the specification and claims of the present application, the compounds are named according to the chemical structural formulae, and if it is indicated that the name and chemical structural formula of the compound are inconsistent when the same compound is represented, the chemical structural formula is prevailing.

In the present application, the scientific and technical nouns used herein have the meanings commonly understood by those skilled in the art, unless otherwise stated, however, in order to better understand the present invention, definitions of some terms are provided below. When the definitions and explanations of terms provided in the present application are inconsistent with those commonly understood by those skilled in the art, the definitions and explanations of terms provided in the present application are prevailing.

The "halogen" in the present invention refers to fluorine, chlorine, bromine and iodine, preferably fluorine and chlorine.

"Halogenation" in the present invention means that any hydrogen in the substituent can be substituted by one or more identical or different halogens. "Halogen" is as defined above.

The "$C_{1-6}$ alkyl" in the present invention refers to a linear or branched alkyl containing 1-6 carbon atoms, including, for example, "$C_{1-5}$ alkyl", "$C_{1-4}$ alkyl", "$C_{1-3}$ alkyl", "$C_{1-2}$ alkyl", "$C_{2-6}$ alkyl", "$C_{2-5}$ alkyl", "$C_{2-4}$ alkyl", "$C_{2-3}$ alkyl", "$C_{3-6}$ alkyl", "$C_{3-5}$ alkyl", "$C_{3-4}$ alkyl", "$C_{4-6}$ alkyl", "$C_{4-5}$ alkyl", "$C_{5-6}$ alkyl", and the like. Specific examples include but are not limited to: methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, and the like. The "$C_{1-4}$ alkyl" according to the present invention refers to a specific example of $C_{1-6}$ alkyl containing 1-4

carbon atoms.

The "$C_{1-6}$ alkylene" in the present invention refers to a group formed by removing one hydrogen atom from the $C_{1-6}$ alkyl, including, for example, "$C_{1-5}$ alkylene", "$C_{1-4}$ alkylene", "$C_{1-3}$ alkylene", "$C_{1-2}$ alkylene", "$C_{2-6}$ alkylene", "$C_{2-5}$ alkylene", "$C_{2-4}$ alkylene", "$C_{2-3}$ alkylene", "$C_{3-6}$ alkylene", "$C_{3-5}$ alkylene", "$C_{3-4}$ alkylene", "$C_{4-6}$ alkylene", "$C_{4-5}$ alkylene", "$C_{5-6}$ alkylene", and the like. Specific examples include but are not limited to: methylene, ethylene, propylene, butylene, pentylene, hexylene, and the like. The "$C_{1-4}$ alkylene" according to the present invention refers to a specific example of $C_{1-6}$ alkylene containing 1-4 carbon atoms.

The "$C_{2-6}$ alkenyl" according to the present invention refers to a linear or branched or cyclic alkenyl with 2-6 carbon atoms and containing at least one double bond, including, for example, "$C_{2-5}$ alkenyl", "$C_{2-4}$ alkenyl" and "$C_{2-3}$ alkenyl". Specific examples include, but are not limited to, ethenyl, 1-propenyl, 2-propenyl, 2-butenyl, 3-butenyl, 2-methyl-1-propenyl, 1-methyl-2-propenyl, 1-pentenyl, 2-pentenyl, 3-pentenyl, 2-methyl-1-butenyl, 3-methyl-1-butenyl, 2-methyl-3-butenyl, 1,1-dimethyl-2-propenyl, 1-ethyl-2-propenyl, 2-hexenyl, 3-hexenyl, 2-methyl-1-pentenyl, 3-methyl-1-pentenyl, 1-methyl-2-pentenyl, 3-methyl-2-pentenyl, 2-methyl-3-pentenyl, 1-methyl-4-pentenyl, 3-methyl-4-pentenyl, 1,1-dimethyl-3-butenyl, 1,2-dimethyl-3-butenyl, 1,3-dimethyl-2-butenyl, 2,2-dimethyl-3-butenyl, 2,3-dimethyl-2-butenyl, 2,3-dimethyl-1-butenyl, 2-ethyl-1-butenyl, 2-ethyl-3-butenyl, etc.

The "$C_{2-6}$ alkynyl" according to the present invention refers to a linear or branched or cyclic alkynyl with 2-6 carbon atoms and containing a triple bond, including, for example, "$C_{2-5}$ alkynyl", "$C_{2-4}$ alkynyl" and "$C_{2-3}$ alkynyl". Specific examples include, but are not limited to, ethynyl, 1-propynyl, 2-butynyl, 1-methyl-2-propynyl, 2-pentynoyl, 3-pentynoyl, 1-methyl-2-butynyl, 2-methyl-3-butynyl, 1,1-dimethyl-2-propynyl, 1-ethyl-2-propynyl, 2-hexynyl, 3-hexynyl, 1-methyl-2-pentynoyl, 1-methyl-3-pentynoyl, 2-methyl-3-pentynoyl, 1,1-dimethyl-3-butynyl, 2-ethyl-3-butynyl, etc.

As used herein, the "$C_{1-6}$ alkoxy, $C_{1-6}$ alkylamino, di($C_{1-6}$ alkyl)amino, $C_{1-6}$ alkylaminoacyl, $C_{1-6}$ alkylamido, $C_{1-6}$ alkylsulfonyl, $C_{1-6}$ alkylsulfonamido, $C_{1-6}$ alkylaminosulfonyl, $C_{1-6}$ alkylcarbonyl, $C_{1-6}$ alkoxycarbonyl and $C_{1-6}$ alkylthio" refer to groups formed in the form of $C_{1-6}$ alkyl-O-, $C_{1-6}$ alkyl-NH-, ($C_{1-6}$ alkyl)$_2$-N-, $C_{1-6}$ alkyl-NH-C(O)-, $C_{1-6}$ alkyl-C(O)-NH-, $C_{1-6}$ alkyl-S(O)$_2$-, $C_{1-6}$ alkyl-S(O)$_2$-NH-, $C_{1-6}$ alkyl-NH-S(O)$_2$-, $C_{1-6}$ alkyl-C(O)-, $C_{1-6}$ alkyl-O-C(O)- and $C_{1-6}$ alkyl-S-, where the "$C_{1-6}$ alkyl" is as defined above.

As used herein, the "$C_{1-4}$ alkoxy, $C_{1-4}$ alkylamino, di($C_{1-4}$ alkyl)amino, $C_{1-4}$ alkylaminoacyl, $C_{1-4}$ alkylamido, $C_{1-4}$ alkylsulfonyl, $C_{1-4}$ alkylsulfonamido, $C_{1-4}$ alkylaminosulfonyl, $C_{1-4}$ alkylcarbonyl, $C_{1-4}$ alkoxycarbonyl and $C_{1-4}$ alkylthio" refer to groups formed in the form of $C_{1-4}$ alkyl-O-, $C_{1-4}$ alkyl-NH-, ($C_{1-4}$ alkyl)$_2$-N-, $C_{1-4}$ alkyl-NH-C(O)-, $C_{1-4}$ alkyl-C(O)-NH-, $C_{1-4}$ alkyl-S(O)$_2$-, $C_{1-4}$ alkyl-S(O)$_2$-NH-, $C_{1-4}$ alkyl-NH-S(O)$_2$-, $C_{1-4}$ alkyl-C(O)-, $C_{1-4}$ alkyl-O-C(O)- and $C_{1-4}$ alkyl-S-, wherein the "$C_{1-4}$ alkyl" is as defined above.

As used herein, the "halogenated $C_{1-6}$ alkyl, hydroxy $C_{1-6}$ alkyl, amino $C_{1-6}$ alkyl, carboxyl $C_{1-6}$ alkyl, halogenated $C_{1-6}$ alkylene and halogenated $C_{1-6}$ alkoxy" refer to groups formed by one or more (for example, 1-4, 1-3 and 1-2) halogen atoms, hydroxyls, aminos and carboxyls substituting hydrogen atoms in $C_{1-6}$ alkyl, $C_{1-6}$ alkylene and $C_{1-6}$ alkoxy respectively.

As used herein, the "halogenated $C_{1-4}$ alkyl, hydroxy $C_{1-4}$ alkyl, amino $C_{1-4}$ alkyl, carboxyl $C_{1-4}$ alkyl, halogenated $C_{1-4}$ alkylene and halogenated $C_{1-4}$ alkoxy" refer to groups formed by one or more (for example, 1-4, 1-3 and 1-2) halogen atoms, hydroxyls, aminos and carboxyls substituting hydrogen atoms in $C_{1-4}$ alkyl, $C_{1-4}$ alkylene and $C_{1-4}$ alkoxy respectively.

The "3-12 membered cycloalkyl" according to the present invention refers to a saturated or partially saturated cyclic alkyl containing 3-12 carbon atoms and having no aromaticity, including "monocycloalkyl" and "fused cycloalkyl".

The "monocycloalkyl" according to the present invention refers to a saturated or partially saturated monocyclic alkyl and having no aromaticity, including "3-8 membered saturated cycloalkyl" and " 3-8 membered partially saturated cycloalkyl"; preferably "3-4 membered cycloalkyl", "3-5 membered cycloalkyl", "3-6 membered cycloalkyl", "3-7 membered cycloalkyl", "4-5 membered cycloalkyl", "4-6 membered cycloalkyl", "4-7 membered cycloalkyl", "4-8 membered cycloalkyl", "5-6 membered cycloalkyl", "5-7 membered cycloalkyl", "5-8 membered cycloalkyl", "6-7 membered cycloalkyl", "6-8 membered cycloalkyl", "7-8 membered cycloalkyl", "3-6 membered saturated cycloalkyl", "4-7 membered saturated cycloalkyl", "4-8 membered saturated cycloalkyl", "5-8 membered saturated cycloalkyl", "5-7 membered saturated cycloalkyl", "5-6 membered saturated cycloalkyl", "3-6 membered partially saturated cycloalkyl", "4-7 membered partially saturated cycloalkyl", "4-8 membered partially saturated cycloalkyl", "5-8 membered partially saturated cycloalkyl", "5-7 membered partially saturated cycloalkyl", and "5-6 membered partially saturated cycloalkyl", and the like. Specific examples of the "3-8 membered saturated cycloalkyl" include, but are not limited to: cyclopropyl, cyclobutyl, cyclopentyl, cyclohexyl, cycloheptyl, cyclooctyl and the like; and specific examples of the "3-8 membered partially saturated cycloalkyl" include, but are not limited to, cyclopropenyl, cyclobutenyl, cyclopentenyl, cyclopentadienyl, cyclohexenyl, cyclohex-1,3-dienyl, cyclohex-1,4-dienyl, cycloheptenyl, cyclohept-1,3-dienyl, cyclohept-1,4-dienyl, cyclohept-1,3,5-trienyl, cyclooctenyl, cyclooct-1,3-dienyl, cyclooct-1,4-dienyl, cyclooct-1,5-dienyl, cyclooct-1,3,5-trienyl, cyclooctatetraenyl and the like.

The "fused cycloalkyl" according to the present invention refers to a saturated or partially saturated and non-aromatic cycloalkyl, which is formed by two or more cyclic structures sharing two adjacent carbon atoms, where one ring of

the fused cycloalkyl may be an aromatic ring, but the fused cycloalkyl as a whole is non-aromatic. The fused cycloalkyl may be fused in a manner as 5-6 membered cycloalkano 5-6 membered cycloalkyl, benzo 5-6 membered cycloalkyl, benzo 5-6 membered saturated cycloalkyl and the like. Examples of the fused cycloalkyl include, but are not limited to, bicyclo[3.1.0]hexyl, bicyclo[4.1.0]heptyl, bicyclo[2.2.0]hexyl, bicyclo[3.2.0]heptyl, bicyclo[4.2.0]octyl, octahydro cyclopentadienyl, octahydro-1H-indenyl, decahydronaphthyl, tetradecahydrophenanthryl, bicyclo[3.1.0]hex-2-enyl, bicyclo[4.1.0]hept-3-enyl, bicyclo[3.2.0]hept-3-enyl, bicyclo[4.2.0]oct-3-enyl, 1,2,3,3a-tetrahydrocyclopentadienyl, 2,3,3a,4,7,7a-hexahydro-1H-indenyl, 1,2,3,4,4a,5,6,8a-octahydronaphthyl, 1,2,4a,5,6,8a-hexahydronaphthyl, 1,2,3,4,5,6,7,8,9,10-decahydrophenanthryl, benzocyclopentyl, benzocyclohexyl, benzocyclohexenyl, benzocyclopentenyl and the like.

The "3-12 membered heterocyclyl" according to the present invention refers to a saturated or partially saturated and non-aromatic monocyclic or fused cycloalkyl containing at least one heteroatom (for example, containing 1, 2, 3, 4 or 5) and having 3-12 ring atoms. The heteroatom is a nitrogen atom, an oxygen atom and/or a sulfur atom, and optionally, a ring atom in a ring structure (for example, a carbon atom, a nitrogen atom or a sulfur atom) can be substituted by oxygen. The "3-12 membered heterocyclyl" according to the present invention includes "3-12 membered saturated heterocyclyl" and "3-12 membered partially saturated heterocyclyl". Preferably, the "3-12 membered heterocyclyl" according to the present invention includes 1-3 heteroatoms; preferably, the "3-12 membered heterocyclyl" according to the present invention contains 1-2 heteroatoms, and the heteroatoms are selected from nitrogen atoms and/or oxygen atoms; and preferably, the "3-12 membered heterocyclyl" according to the present invention contains 1-2 nitrogen atoms. The "3-12 membered heterocyclyl" is preferably "3-10 membered heterocyclyl", "3-8 membered heterocyclyl", "4-8 membered heterocyclyl", "3-6 membered heterocyclyl", "3-6 membered saturated heterocyclyl", "3-6 membered nitrogen-containing heterocyclyl", "3-6 membered saturated nitrogen-containing heterocyclyl", "5-6 membered heterocyclyl", "5-6 membered saturated heterocyclyl", "5-6 membered nitrogen-containing heterocyclyl", etc. Specific examples of the "3-12 membered heterocyclyl" include, but are not limited to: aziridinyl, 2H-aziridinyl, diazaziridinyl, 3H-diazacyclopropenyl, azetidinyl, 1,4-dioxanyl, 1,3-dioxanyl, 1,3-dioxolanyl, 1,4-dioxadienyl, tetrahydrofuranyl, tetrahydropyranyl, dihydropyrrolidyl, pyrrolidinyl, imidazolyl, 4,5-dihydroimidazolyl, pyrazolidinyl, 4,5-dihydropyrazolyl, 2,5-dihydrothienyl, tetrahydrothiophenyl, 4,5-dihydrothiazolyl, piperidinyl, piperazinyl, morpholinyl, 4,5-dihydrooxazolyl, 4,5-dihydroisoxazolyl, 2,3-dihydroisoxazolyl, *2H-1,2-oxazinyl,* 6H-1,3-oxazinyl, *4H-1,3-thiazinyl,* 6H-1,3-thiazinyl, 2H-pyranyl, 2H-pyran-2-onyl, 3,4-dihydro-2H-pyranyl, pyrrolidinocyclopropyl, cyclopentazacyclopropyl, pyrrolidinocyclobutyl, pyrrolidinopyrrolidinyl, pyrrolidinopiperidinyl, pyrrolidinopiperazinyl, pyrrolidinomorpholinyl, piperidinomorpholinyl, benzopyrrolidinyl, benzocyclopentyl, benzocyclohexyl, benzotetrahydrofuranyl, benzopyrrolidinyl, benzoimidazolyl, benzoxazolidinyl, benzothiazolidinyl, benzoisoxazolidinyl, benzoisothiazolidinyl, benzopiperidinyl, benzomorpholinyl, benzopiperazinyl, benzotetrahydropyranyl, pyridinocyclopentyl, pyridinocyclohexyl, pyridinotetrahydrofuranyl pyridinopyrrolidinyl, pyridinoimidazolyl, pyridoxazolidinyl, pyridinothiazolidinyl, pyridinosatoisothiazolidinyl, pyridinoisothiazolidinyl, pyridinopiperidinyl, pyridinomorpholinyl, pyridinopiperazinyl, pyridinotetrahydropyranyl, pyrimidinocyclopentyl, pyrimidinocyclohexyl, pyrimidinotetrahydrofuranyl, pyrimidinopyrrolidinyl, pyrimidinoimidazolyl, pyrimidinothiazolidinyl, pyrimidinoisothiazolidinyl, pyrimidinoisothiazolidinyl, pyrimidinopiperidiny, pyrimidinomorpholinyl, pyrimidinopiperazinyl, pyrimidinotetrahydropyranyl, tetrahydroimidazo[4,5-c]pyridinyl, 3,4-dihydroquinazolinyl, 1,2-dihydroquinoxalinyl, benzo[d][1,3]dioxolyl, 2*H*-chromenyl, 2*H*-chromen-2-onyl, 4*H*-chromenyl, 4*H*-chromen-4-onyl, 4*H*-1,3-benzoxazinyl, 4,6-dihydro-1*H*-furo[3,4-d]imidazolyl, 3a,4,6,6a-tetrahydro-1*H*-furo[3,4-d]imidazolyl, 4,6-dihydro-1*H*-thieno[3,4-d]imidazolyl, 4,6-dihydro-1*H*-pyrrolo[3,4-d]imidazolyl, octahydrobenzo octahydro-benzo[d]imidazolyl, decahydroquinolinyl, hexahydrothienoimidazolyl, hexahydrofuroimidazolyl, 4,5,6,7-tetrahydro-1*H*-benzo[d]imidazolyl, octahydrocyclopenta[c]pyrrolyl, 4*H*-1,3-benzoxazinyl and the like.

The "6-10 membered aryl" in the present invention refers to an aromatic ring group which contains 6 to 10 cyclic carbon atoms, including "6-8 membered monocycloaryl " and "8-10 membered fused ring aryl".

The "6-8 membered monocycloaryl" in the present invention refers to monocycloaryl which contains 6 to 8 cyclic carbon atoms, and examples thereof comprise, but are not limited to, phenyl, cyclooctatetraenyl and the like; and phenyl is preferred.

The "8-10 membered fused ring aryl" in the present invention refers to an unsaturated aromatic ring group which is formed by two or more ring structures sharing two adjacent atoms with each other and contains 8 to 10 cyclic carbon atoms, and "9-10 membered fused ring aryl" is preferred, with specific examples such as naphthyl and the like.

The "5-12 membered heteroaryl" in the present invention refers to an aromatic ring group which contains 5 to 12 ring atoms (where at least one ring atom is a heteroatom, such as a nitrogen atom, an oxygen atom or a sulfur atom), such as 5-12 membered nitrogen-containing heteroaryl, 5-12 membered oxygen-containing heteroaryl, 5-12 membered sulfur-containing heteroaryl and the like, including "5-8 membered monoheteroaryl" and "8-10 membered fused heteroaryl".

The "5-8 membered monoheteroaryl" in the present invention refers to an aromatic monocyclic ring group which contains 5 to 8 ring atoms (where at least one ring atom is a heteroatom, such as a nitrogen atom, an oxygen atom or a sulfur atom). Optionally, ring atoms (such as carbon atoms, nitrogen atoms or sulfur atoms) in a ring structure may be substituted by oxygen atoms. The "5-8 membered monoheteroaryl" includes, for example, "5-7 membered monoheteroaryl", "5-6 membered monoheteroaryl", "5-6 membered nitrogen-containing monoheteroaryl", "5 membered nitrogen-containing monoheteroaryl" and the like. Specific examples of "5-8 membered monocyclic heteroaryl" comprise, but are not limited to, furyl, thienyl, pyrrolyl, thiazolyl, isothiazolyl, thiadiazole, oxazolyl, isoxazole, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, pyridazinyl, pyrazinyl, 1,2,3-triazinyl, 1,3,5-triazinyl, 1,2,4,5-tetrazinyl, aza-cycloheptatrienyl, 1,3-diaza-cycloheptatrienyl, aza-cyclooctatetraenyl. The "5-6 membered heteroaryl" refers to a specific example in which 5-8 member heteroaryl contains 5 -6 ring atoms.

The "8-10 membered fused heteroaryl" in the present invention refers to an unsaturated aromatic ring structure which is formed by two or more ring structures sharing two adjacent atoms with each other and contains 8 to 10 ring atoms (where at least one ring atom is a heteroatom, such as a nitrogen atom, an oxygen atom or a sulfur atom). Optionally, ring atoms (such as carbon atoms, nitrogen atoms or sulfur atoms) in a ring structure may be substituted by oxygen atoms, including "9-10 membered fused heteroaryl", "8-9 membered fused heteroaryl" and the like, and a fusion mode may be benzo 5-6 membered heteroaryl, 5-6 membered heteroaro 5-6 membered heterocyclyl and the like; and specific examples comprise, but are not limited to, pyrrolopyrrole, pyrrolofuran, pyrazolopyrrole, pyrazolothiophene, furanothiophene, pyrazoloxazole, benzofuryl, benzoisofuryl, benzothiophenyl, indolyl, isoindolyl, benzoxazolyl, benzimidazolyl, indazolyl, benzotriazolyl, quinolyl, 2-quinolinonyl, 4-quinolinonyl, 1-isoquinolinonyl, isoquinolinyl, acridinyl, phenanthridinyl, benzopyridazinyl, phthalazinyl, quinazolinyl, quinoxalinyl, purinyl, naphthyridinyl and the like.

The "optionally substituted by ..." in the present invention includes two conditions of being "substituted" and "unsubstituted".

The-- and - in the present invention is selected from a single-bond or a double-bond.

Unless otherwise specified, any atom of the compounds of the present application may represent any stable isotope of the atom. Unless otherwise specified, when a certain site in a structure is defined as H, which is namely deuterium (H-1), this site only contains naturally occurring isotopes. Similarly, unless otherwise specified, when a certain site in a structure is defined as D, which is namely deuterium (H-2), an isotope content at this site is at least 3,340 folds greater than the naturally occurring isotope content (0.015%) (which means that at least 50.1% deuterium isotopes are contained), and when one or more sites in structures of the compounds of the present application are defined as D, which is namely deuterium (H-2), contents of the compounds represented by the structures may be at least 52.5%, at least 60%, at least 67.5%, at least 75%, at least 82.5%, at least 90%, at least 95%, at 97%, at least 98.5%, at least 99% and at least 99.5%.

Deuteration rates of the compounds of the present application refer to ratios of labeled-synthesized isotope contents to naturally occurring isotope contents. A deuteration rate of each designated deuterium atom in the compounds of the present application may be at least 3,500 folds (52.5%), at least 4,000 folds (60%), at least 4,500 folds (67.5%), at least 5,000 folds (75%), at least 5,500 folds (82.5%), at least 6,000 folds (90%), at least 6,333.3 folds (95%), at least 6,466.7 folds (97%), at least 6,566.7 folds (98.5%), at least 6,600 folds (99%) and at least 6,633.3 folds (99.5%).

Isotopologues in the present application refer to compounds that differ only in isotopic composition in chemical structure. The compounds containing deuterium at specific sites in the present application may also contain very few hydrogen isotopologues at the sites, and an amount of hydrogen isotopologues at deuterated sites in the deuterated compounds of the present application depends on many factors, including a deuterium isotope purity of a deuterated reagent ($D_2O$, $D_2$, $NaBD_4$, $L1AlD_4$, etc.) and an effectiveness of a synthetic method of introducing deuterium isotopes. However, as mentioned above, a total amount of hydrogen isotopologues at deuterated sites will be less than 49.9%. The total amount of hydrogen isotopologues at deuterated sites in the deuterated compounds of the present application will be less than 47.5%, 40%, 32.5%, 25%, 17.5%, 10%, 5%, 3%, 1% or 0.5%.

In the present application, any atom not designated as deuterium exists in the natural isotope abundance.

The "deuterated" in the present invention refers to replacing one or more hydrogen atoms on a deuterated group by one or more deuterium atoms, which may be partially deuterated or completely deuterated. For example, a deuterated compound may contain only one deuterium atom. In some embodiments, the deuterated compound contains only two deuterium atoms. In some embodiments, the deuterated compound contains only three deuterium atoms. In some embodiments, the deuterated compound contains only four deuterium atoms.

The "optionally deuterated" as mentioned in the present invention includes two conditions that a group is deuterated and non-deuterated, where the "deuterated" is as defined above.

The "therapeutically effective amount" as mentioned in the present invention refers to amounts of the aforementioned compound, the pharmaceutical formulation and the pharmaceutical composition capable of at least alleviating symptoms of a disease of a patient when administered to the patient. An actual amount including the "therapeutically effective amount" may vary according to various conditions, including but being not limited to a specific disease treated, a severity of disease, a physical and health status of patient and a route of administration. A skilled medical practitioner may easily determine an appropriate amount by methods known in the medical field.

Beneficial effects of the present invention

(1) The compound, the pharmaceutically acceptable salt, ester and deuterated compound thereof, or the stereoisomer thereof of the present invention have excellent inhibitory activity on USP1, and can treat and/or prevent the disease mediated by USP1 and the disease related thereto;
(2) the compound of the present invention has a good inhibitory effect on tumor cells;
(3) the compound, the pharmaceutically acceptable salt, ester and deuterated compound thereof, or the stereoisomer thereof of the present invention have good pharmacokinetic properties, more lasting action and high bioavailability;
(4) the compound, the pharmaceutically acceptable salt, esters and deuterated compound thereof, or the stereoisomer thereof of the present invention have good safety; and
(5) the compound of the present invention has simple preparation process, high drug purity and stable quality, and are easy for large-scale industrial production.

The beneficial effects of the compound provided by the examples of the present invention are further illustrated by experiments, which should not be understood that the compounds provided by the examples of the present invention only have the following beneficial effects.

**Experimental Example 1 In-vitro enzymatic activities of compounds of** the **present invention**

Test compounds: the compounds synthesized in the examples of the present invention, with structural formulas shown in Table 1.

Experimental reagents:

| Reagent | Vendor | Cat No. |
|---|---|---|
| Recombinant Human His6-USP1/His6-UAF1 Complex Protein | R&D | E-568-050 |
| Ubiquitin Rhodamine 110 Protein, CF (Ub-Rho) | R&D | U-555-050 |

Experimentalcomsumables:

| Consumables | Vendor | Cat No. |
|---|---|---|
| 384-Well plate | Perkin Elmer | 6007279 |

First experimental method:

1. Compound dilution

1) The compounds of the present invention were prepared to 10 mM with DMSO, and used as test stock solutions.
2) The stock solutions of the compounds of the present invention were diluted in a 4-fold gradient for 10 concentrations, with a maximum concentration of 10 mM.
3) The diluted compounds of the present invention were respectively transferred to 384-well plates (diluted by 1,000 folds) by Echo550, 2 multiple wells were set for each concentration, and a final concentration of DMSO was 1%.
4) The final concentrations of test compounds were 10,000 nM, 2,500 nM, 625 nM, 156 nM, 39 nM, 9.8 nM, 2.4 nM, 0.61 nM, 0.15 nM and 0.038 nM.

2. Enzyme reaction experiment

1) An enzyme solution was prepared in 1×test buffer.
2) Ubiquitin Rhodamine 110 protein, CF (UB-Rho), was added into 1×determination buffer to prepare a substrate solution.
3) 10 μL of enzyme solution and 1×reaction buffer were transferred to a 384-well plate, and
4) incubated at room temperature for 15 minutes.
5)10 μL of substrate solution was added into each well to start the reaction, centrifuged for 30 seconds, and shaken for 30 seconds.

3. Result detection

1) Plate reading was carried out on SpectraMax Paradigm for 30 minutes, with an excitation wavelength of 480 nm and an emission wavelength of 540 nm.
2) Data on SpectraMax Paradigm were collected.
4. Data analysis

An inhibition (% inh) was calculated by the following formula:

$$\text{inhibition}(\%) = 100\% \times \frac{Max - Signal}{Max - Min}$$

wherein, Max represented: a luminous signal intensity of a positive control well without adding the compound;
Min represented: a luminous signal intensity of a negative control well without adding the enzyme; and
Signal represented: a luminous signal intensity of the compound of the test sample.
$IC_{50}$ was calculated by the following formula:

$$\text{Y} = \text{Bottom} + \frac{\text{Top} - \text{Bottom}}{1 + (IC50 \div X) \times HillSlope}$$

wherein, Y represented: %inhibition; and
X represented: the concentration of the compound.

Experimental results:

Table 2 Inhibitory activities of compounds of the present invention on USP-1 [00359]

| Compounds | $IC_{50}$ (nM) |
|---|---|
| Compound 1 | 28.0 |
| Compound 2 | 5.0 |
| Compound 3 | 18 |
| Compound 11 | 22 |

It can be seen from the above experimental results that the compounds prepared by the present invention can effectively inhibit the activity of USP1, thus being effective USP1 inhibitors.

Second experimental method:

In the enzyme reaction experiment, the incubation time at room temperature in the step 4) was changed to 60 minutes, other conditions were the same as those in the first experimental method, and the following test results were obtained:

Table 3 Inhibitory activities of compounds of the present invention on USP-1 [00364]

| Compounds | $IC_{50}$ (nM) |
|---|---|
| Compound 16 | 8.00 |
| Compound 18 | 3.98 |
| Compound 19 | 4.36 |
| Compound 1-1 | 12.88 |
| Compound 11-1 | 15.0 |
| Compound 16-1 | 6.67 |
| Compound 17-1 | 6.66 |

It can be seen from the above experimental results that the compounds prepared by the present invention can effectively inhibit the activity of USP1, thus being effective USP1 inhibitors.

**Experimental Example 2 First experiment of in-vitro cytological inhibitory activities of compounds of the** present **invention**

Test compounds: some compounds of the present invention, with chemical names and structures shown in the preparation examples.

A cell line used in the following experiment was as follows: MDA-MB-436: human breast cancer cells.

The definitions represented by the following abbreviations were as follows:

FBS: fetal bovine serum
ITS-G: insulin-transferrin-selenium additive
Glutathione: GSH
Experimental method (CelltiterGlo assay)

1 Cell preparation

1.1 Cell culture:

All cells were adherent cells, the medium was DMEM+10% FBS+1% ITS-G+16 μg/ml glutathione, and the cells were tested in a logarithmic growth phase.

1.2 Preparation of cell suspension:

Cells in the logarithmic growth phase were collected and counted by a platelet counter. The cell viability was detected by a trypan blue exclusion method, and the cell viability was kept over 90%. The cell concentration was adjusted to be in a proper range, and 90 μL of cell suspension was added into a 96-well plate respectively.

Table 4 Number of cell inoculation [00380]

| Cell line | Number of cells inoculated |
|---|---|
| MDA-MB-436 | 4,000 cells/well |

2 Preparation of test compounds

2.1 DMSO stock solutions of the test compounds were prepared, and concentrations of the stock solutions of the test compounds were 10 mM respectively.

2.2 Preparation of working stock solutions of test compounds

The stock solutions of the test compounds were serially diluted from 10 mM with DMSO in a 3-fold gradient for a total of 8 concentrations. Then, 2 μL of DMSO-gradient diluted compounds were respectively added into 198 μL of culture solutions to give the working stock solutions of the test compounds (a compound concentration was 10-fold higher than the final concentration, and a maximum concentration was 10 μM).

2.3 Compound treatment

10 μL of compound working stock solution was added into each well in the 96-well plate inoculated with cells (10-fold dilution, a final DMSO concentration was 0.1%).

The final concentrations of the test compounds were: 10,000.00 nM, 3,333.33 nM, 1,111.11 nM, 370.37 nM, 123.46 nM, 41.15 nM, 13.72 nM and 4.57 nM.

2.4 Setting of control well

Solvent control: 0.1% DMSO.

Blank control: only a medium was added, and cells were not inoculated.

2.5 The 96-well plate was placed in a 5% $CO_2$ cell incubator at 37°C to culture for 7 days.

3 Detection

A CTG reagent was thawed and the 96-well plate was equilibrated to room temperature for 30 minutes, 60 μL of reagent (Celititer Glo assay kit) was added into each well, shaking was carried out for 2 minutes with a shaker (in the absence of light), and the plate was incubated at room temperature for 20 minutes (in the absence of light). Light signal values were read on a multi-functional microplate reader.

4 Data processing

1) inhibition (%)=(DMSO solvent control well reading-test compound well reading)/(DMSO solvent control well reading-blank control well reading)×100%;

2) GraphPad Prism was input for drawing to obtain a curve and $IC_{50}$.

**Experimental result and conclusion**

Table 5 In-vitro cytological activities of compounds of the present invention ($IC_{50}$, nM) [00399]

| Test compound | MDA-MB-436 |
|---|---|
| Compound 1 | 68.05 |
| Compound 2 | 88.12 |
| Compound 11 | 100.95 |

It can be seen from Table 5 that the compounds of the present invention can effectively inhibit the proliferation of MDA-MB-436 cells, indicating that the compounds of the present invention have the potential of clinical application in treating cancerous diseases with BRCA1 gene mutation.

**Experimental Example 3 Second experiment of in-vitro cytological inhibitory activities of compounds** of the **present invention**

Test compounds: some compounds of the present invention, with chemical names and structures shown in the preparation examples.

Cell lines used in the following experiment were as follows: MDA-MB-436: human breast cancer cells; and Caov-3: Human ovarian cancer cells

**First experimental method** (CelltiterGlo assay)

1 Cell preparation

1.1 Cell culture:

All cells were adherent cells, the MDA-MB-436 cell medium was DMEM+10% FBS+1% ITS-G+16 μg/ml glutathione, the Caov-3 cell medium was DMEM+10% FBS, and cells were tested in a logarithmic growth phase.

1.2 Preparation of cell suspension:

Cells in the logarithmic growth phase were collected and counted by a platelet counter. The cell viability was detected by a trypan blue exclusion method, and the cell viability was kept over 90%. The cell concentration was adjusted to be in a proper range, and 90 μL of cell suspension was added into a 96-well plate respectively.

Table 6 Number of cell inoculation

| Cell line | Number of cells inoculated |
|---|---|
| MDA-MB-436 | 3,000 cells/well |
| Caov-3 | 2,000 cells/well |

2 Preparation of test compounds

2.1 DMSO stock solutions of the test compounds were prepared, and concentrations of the stock solutions of the test compounds were 10 mM respectively.

2.2 Preparation of working stock solutions of test compounds

The stock solutions of the test compounds were serially diluted from 10 mM with DMSO in a 3-fold gradient for a total of 8 concentrations. Then, 2 μL of DMSO-gradient diluted compounds were respectively added into 198 μL of culture solutions to give the working stock solutions of the test compounds (a compound concentration was 10-fold higher than the final concentration, and a maximum concentration was 100 μM).

2.3 Compound treatment

10 μL of compound working stock solution was added into each well in the 96-well plate inoculated with cells (10-fold dilution, a final DMSO concentration was 0.1%).

The final concentrations of the test compounds were: 10,000.00 nM, 3,333.33 nM, 1,111.11 nM, 370.37 nM, 123.46 nM, 41.15 nM, 13.72 nM and 4.57 nM.

2.4 Setting of control well

Solvent control: 0.1% DMSO.

Blank control: 0 hour after dosing, the 96-well plate was detected and read.

2.5 The 96-well plate was placed in a 5% $CO_2$ cell incubator at 37°C to culture for 7 days.

3 Detection

A CTG reagent was thawed and the 96-well plate was equilibrated to room temperature for 30 minutes, 60 μL of reagent (Celititer Glo assay kit) was added into each well, shaking was carried out for 2 minutes with a shaker (in the absence of light), and the plate was incubated at room temperature for 20 minutes (in the absence of light). Light signal values were read on a multi-functional microplate reader.

4. Data processing

1) inhibition (%)=(DMSO solvent control well reading-test compound well reading)/(DMSO solvent control well reading-blank control well reading)×100%;
2) GraphPad Prism was input for drawing to obtain a curve and $IC_{50}$.

Experimental result and conclusion

Table 7 In-vitro cytological activities of compounds of the present invention ($IC_{50}$, nM)

| Test compound | MDA-MB-436 |
|---|---|
| Compound 16 | 42.31 |
| Compound 18 | 29.67 |
| Compound 19 | 49.79 |
| Compound 1-1 | 42.43 |
| Compound 11-1 | 121.69 |
| Compound 16-1 | 55.32 |
| Compound 17-1 | 110.41 |

The $IC_{50}$ values of the inhibitory activities of the compounds 11, 16, 18, 19, 1-1, 11-1, 16-1 and 17-1 in the examples of the present invention on Caov-3 cells range from 1 nM to 600 nM, indicating that the compounds of the present invention can effectively inhibit the proliferation of the MDA-MB-436 cells and the Caov-3 cells, so that the compounds of the present invention have the clinical application potential for treating HRD positive (homologous recombination defect) cancer diseases.

**Second experimental method:**

The concentrations of the compound stock solutions prepared were 5 mM.
The stock solutions of the test compounds were serially diluted from 5 mM with DMSO in a 3-fold gradient for a total of 9 concentrations.
The final concentrations of the test compounds were: 10,000.00 nM, 3,333.33 nM, 1,111.11 nM, 370.37 nM, 123.46 nM, 41.15 nM, 13.72 nM, 4.57 nM and 1.52 nM.
Other conditions were the same as those in the first experimental method, and the $IC_{50}$ value of the inhibitory activity of the compound 20 on the MDA-MB-436 cells was 55.7 nM; and the $IC_{50}$ value of the inhibitory activity of the compound 20 on the Caov-3 cells was 1 nM to 100 nM.

**Experiment Example 4 Pharmacokinetic experiment of compounds of the present invention**

1. Test compounds: the compounds in the examples of the present invention, prepared according to the examples in the specification of the present application.

| Number of animals | Sex | Administration route | Sample |
|---|---|---|---|
| 9 | Female | Intravenous injection (iv) | Plasma |
| 9 | Female | Oral administration (po) | Plasma |

2. Preparation of test compound solution

(1) Intravenous administration (iv): 2.54 mg of compound 11 was taken, added with 0.495 ml of DMSO solution to be dissolved by vortex and ultrasound, added with 0.495 ml of PEG400 to be mixed by vortex, and then added with 1.484 ml of 28% HP-β-CD to be mixed by vortex to obtain a clear solution, with a concentration of 1 mg/ml.
(2) Oral administration (po): 3.33 mg of compound 11 was taken, added with 3.243 ml of solvent (2% HPC+0.1% Tween 80), and ground evenly to obtain a uniform suspension, with a concentration of 1 mg/ml.

3. Experimental method

(1) Administration

The test compounds were administered intravenously (iv), with an administration dosage of 5 mg/kg and an administration volume of 5 ml/kg.

The test compounds were administered orally (po), with an administration dosage of 10 mg/kg and an administration volume of 10 ml/kg.

(2) Blood sampling

0.083 hour, 0.25 hour, 0.5 hour, 1 hour, 2 hours, 3 hours, 4 hours, 6 hours, 8 hours and 24 hours after administration, tail vein blood sampling was carried out, about 100 μl of whole blood was taken at each time point, plasma was centrifugally separated in a high-speed centrifuge at 8,000 rpm for 6 minutes, and the plasma was frozen in a refrigerator at -80°C.

(3) Analysis of plasma sample

A protein precipitation method was adopted: 20 μl of plasma was added into a 96-well deep-well plate, added with 200 μl of internal standard solution (tolbutamide-200 ng/ml) to be processed by vortex for 10 minutes, and then centrifuged at 4,000 rpm for 20 minutes, and 100 μl of supernatant was taken, and added with 100μL of water to be processed by vortex for 3 minutes. LC-MS/MS was to be analyzed.

**4. Experimental result and conclusion**

Experimental data show that intravenous injection or oral administration of the compounds of the present invention has high exposure in vivo and suitable half-life and clearance rate, showing good pharmacokinetic properties. For example, the exposure of the compound 11 administered intravenously in mice is more than 6,000 h*ng/ml, indicating that the compounds in the examples of the present invention have good clinical application prospects.

**DETAILED DESCRIPTIONS OF THE EMBODIMENTS**

The following clearly and completely describes the technical solutions of the present invention with reference to the embodiments of the present invention. Apparently, the described embodiments are merely some but not all of the embodiments of the present invention. Based on the embodiments of the present invention, all other embodiments obtained by those of ordinary skills in the art without going through any creative work shall fall within the protection scope of the present invention.

The abbreviations used in the following experiments have the following meanings:

Xphos-Pd-G2: Chloro(2-dicyclohexylphosphino-2',4',6'-triisopropyl-1,1'-biphenyl)[2-(2'-amino-1,1'-biphenyl)]palladium (II); Xphos: 2-dicyclohexylphosphino-2',4',6'-triisopropylbiphenyl; Pd(dppf)$Cl_2$: dichloro[1,1'-bis(diphenylphosphino)ferrocene]palladium; DMF: N,N-dimethylformamide EA: ethyl acetate; PE: petroleum ether; DCM: dichloromethane; NMP: N-methylpyrrolidone; and DIBAl-H: diisobutylaluminum hydride

**Example 1 Preparation of 4-(4-cyclopropyl-6-methoxypyrimidine-5-yl)-2-(4-(1-methyl-4-(trifluoromethyl)-1H-imidazol-2-yl)benzyl)-2,6,7,8-tetrahydropyrazolo[3,4,5-de]quinazoline (Compound 1)**

**(1) Preparation of 2-(bis(methylthio)methylene)cyclohexane-1,3-dione**

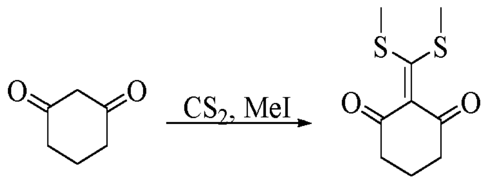


1-3-cyclohexanedione (20 g, 178.4 mmol) was dissolved to DMF (200 mL), added with potassium carbonate (74 g, 535.2 mmol), and reacted at 20°C for 0.5 h. Carbon disulphid (20 g, 267.6 mmol) was added and reacted at 20°C for 1 h. Methyl iodide (76 g, 535.2 mmol) was added and reacted at 20°C for 1 h. The reaction mixture was concentrated to give a

crude product used directly in next step.

(2) Preparation of 2-amino-4-(methylthio)-7,8-dihydroquinazoline-5(6H)-one

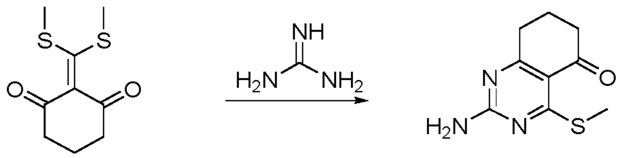

2-(bis(methylthio)methylene)cyclohexane-1,3-dione (crude product from the above step) was dissolved in DMF (200 mL), added with guanidine hydrochloride (17 g, 178.4 mmol) and potassium carbonate (49.3 g, 356.8 mmol), reacted at 100°C for 16 h, cooled to 25°C, added with water (300 mL), and filtered to obtain a filter cake, and then dried in vacuum to give 16.3 g of the target compound, with a yield of 44%.

**(3) Preparation of 2-chloro-4-(methylthio)-7,8-dihydroquinazolin-5(6H)-one**

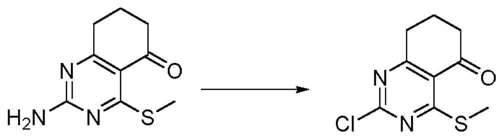

2-amino-4-(methylthio)-7,8-dihydroquinazoline-5(6H)-one (16.3 g, 78 mmol) was dissolved to dichloromethane (150 mL), added with titanium tetrachloride (14.8 g, 78 mmol) and tert-butyl nitrite (48 g, 468 mmol), and reacted at 25°C for 3 h. Water was added for quenching, and the reaction mixture was filtered to obtain a filtrate, concentrated and subjected to column chromatography (ethyl acetate/petroleum ether = 0-40%) to give 6.1 g of the target compound, with a yield of 34%.

**(4) Preparation of 2-(4-cyclopropyl-6-methoxypyrimidin-5-yl)-4-(methylthio)-7,8-dihydroquinazolin-5(6H)-**one

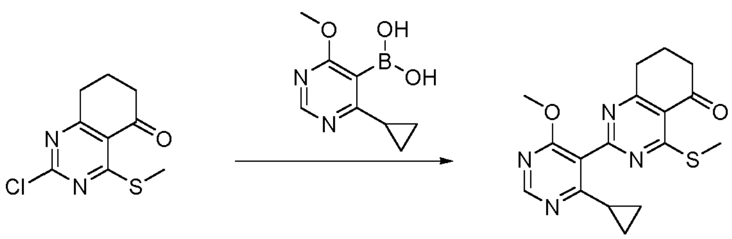

2-chloro-4-(methylthio)-7,8-dihydroquinazolin-5(6H)-one (1.2 g, 5.15 mmol) was dissolved to 1,4-dioxane (30 mL), added with (4-cyclopropyl-6-methoxypyrimidin-5-yl)boric acid (1 g, 5.15 mmol), Xphos-Pd-G2 (408 mg, 0.52 mmol), Xphos (496 mg, 1.04 mmol), $K_3PO_4$ (1.2 g, 5.7mmol), and water (10 mL), and reacted under the protection of $N_2$ at 90°C for 3 h. After the reaction, the solvent was spin-dried to give 830 mg of the target compound by normal phase preparation and separation (ethyl acetate/petroleum ether = 0-50%) with a yield of 47%.

(5) **Preparation of 2-(4-cyclopropyl-6-methoxypyrimidin-5-yl)-4-hydrazino-7,8-dihydroquinazolin-5(6H)-**one

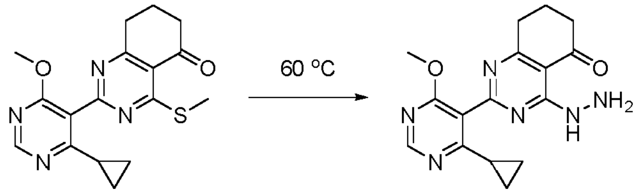


2-(4-cyclopropyl-6-methoxypyrimidin-5-yl)-4-(methylthio)-7,8-dihydroquinazolin-5(6H)-one (830 mg, 2.4 mmol) was dissolved to ethanol (20 mL), added with hydrazine hydrate (184 mg, 3.6 mmol), and reacted at 70°C for 3 h. After the reaction, the reaction mixture was concentrated to give 700 mg of the target compound with a yield of 89%.

(6) **Preparation of 4-(4-cyclopropyl-6-methoxypyrimidin-5-yl)-2,6,7,8-tetrahydropyrazolo[3,4,5-de]quinazoline**

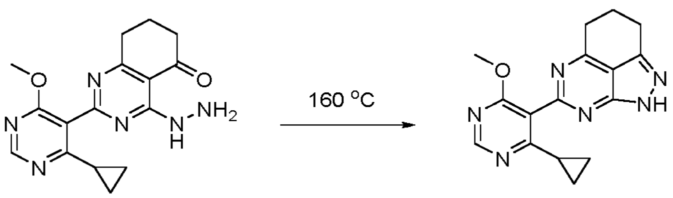


2-(4-cyclopropyl-6-methoxypyrimidin-5-yl)-4-hydrazino-7,8-dihydroquinazolin-5(6H)-one (300 mg, 0.92 mmol) was dissolved to N-methylpyrrolidone (5 mL) and ethanol (60 mL), and reacted at 160°C for 1 h with microwave. The reaction mixture was subjected to column chromatography separation (methanol/water = 0-60%) to give 150 mg of the target compound with a yielding of 53%.

(7) **Preparation of 4-(4-cyclopropyl-6-methoxypyrimidine-5-yl)-2-(4-(1-methyl-4-(trifluoromethyl)-1H-imidazol-2-yl)benzyl)-2,6,7,8-tetrahydropyrazolo [3,4,5-*de*]quinazoline**

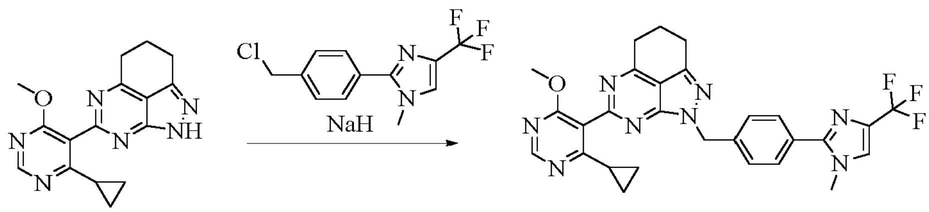


4-(4-cyclopropyl-6-methoxypyrimidin-5-yl)-2,6,7,8-tetrahydropyrazolo[3,4,5-de]quinazoline (130 mg, 0.43 mmol) was dissolved to N,N-dimethylformamide (4 mL), added with sodium hydride (60%, 34 mg, 0.85 mmol), and reacted at 20°C for 10 min, then added with 2-(4-(chloromethyl)phenyl)-1-methyl-4-(trifluoromethyl)-1H-imidazole (139 mg, 0.51 mmol), and reacted at 20°C for 3 h. After the reaction, the reaction mixture was subjected to column chromatography separation (methanol/water = 0-40%) to give 18.5 mg of the target compound with a yield of 8%.

Molecular formula: $C_{28}H_{25}F_3N_8O$,

Molecular weight: 546.6, LC-MS (M/e): 547.2(M+H$^+$)

$^1$H-NMR (400 MHz, $CDCl_3$) $\delta$: 8.65(s, 1H), 7.60-7.45 (m, 4H), 7.29(s, 1H), 5.67(s, 2H), 3.91(s,3H), 3.72(s,3H), 3.15-3.02 (m, 4H), 2.45-2.32 (m, 2H), 1.69-1.55 (m, 1H), 1.25-1.19 (m, 2H), 0.92-0.75 (m, 2H).

**Example 2 Preparation of** 4-(3-fluoro-2-isopropylphenyl)-2-(4-(1-methyl-4-(trifluoromethyl)-1H-imidazol-**2-yl)benzyl)-2,6,7,8-tetrahydropyrazolo[3,4,5-de]quinazoline (Compound 2)**

(1) **Preparation** of 3-fluoro-2-(prop-1-en-2-yl)phenol

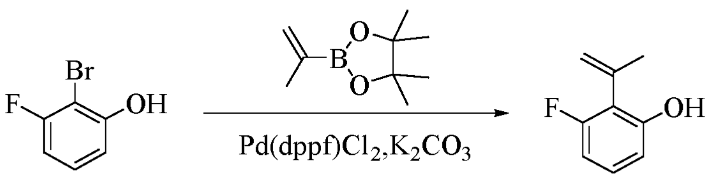


2-bromo-3-fluorophenol (11 g, 57.6 mmol), 4,4,5,5-tetramethyl-2-(prop-l-en-2-yl)-1,3,2-dioxolane (13.2 g, 78.6 mmol), $K_2CO_3$ (15.9 g, 115.2 mmol) and Pd(dppf)$Cl_2$ (4.2 g, 5.7 mmol) were dissolved in dioxane (120 mL) and water (24 mL), reacted at 80°C for 16 h under the protection of nitrogen. After the completion of the reaction by LCMS detection, the reaction solution was poured into water (200 mL), extracted with EA, then organic phases were dried with anhydrous sodium sulfate, spin-dried and separated by silica gel column chromatography (EA: PE = 1:5) to give 5.6 g of the product with a yield of 63.9%.

(2) Preparation of 3-fluoro-2-isopropylphenol

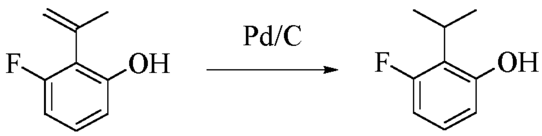


3-fluoro-2-(prop-1-en-2-yl)phenol (5.6 g, 36.8 mmol) was dissolved to methanol (60 mL), added with Pd/C (1.2 g), and reacted at 25°C for 4 h. After the completion of the reaction by LCMS detection, the reaction mixture was subjected to suction filtration, and the filtrate was spin-dried to give 5 g of the product with a yield of 88.2%.

**(3) Preparation of 3-fluoro-2-isopropylphenyl triflate**

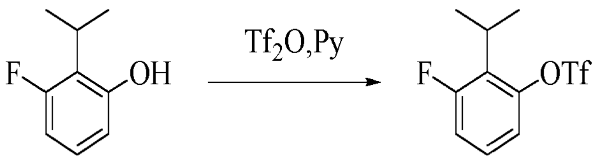


3-fluoro-2-isopropylphenol (1 g, 6.49 mmol) was dissolved to dichloromethane (20 mL), dropwise added with pyridine (1.0 g, 12.6 mmol) and $Tf_2O$ (2.7 g, 9.6 mmol) at -10°C, and then continuously reacted for 1 h. After the completion of the reaction by LCMS detection, the reaction solution was poured into water (50 mL) for quenching, and extracted with DCM. Organic phases were dried with anhydrous sodium sulfate, spin-dried, and separated by silica gel column chromatography (EA: PE = 1:5) to give 1.6 g of the product with a yield of 86.1%.

**(4) Preparation of 2-(3-fluoro-2-isopropylphenyl)-4,4,5,5-tetramethyl-1,3,2-dioxolane**

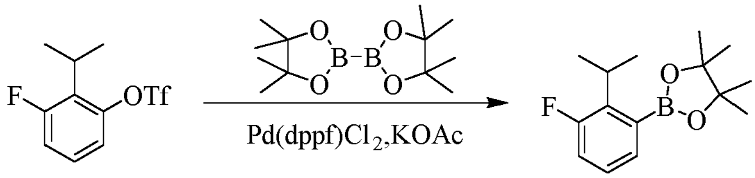


3-fluoro-2-isopropylphenyl triflate (1.6 g, 5.59 mmol), bis(pinacolato)diboron (2.8 g, 11.18 mmol), Pd(dppf)$Cl_2$ (409 mg, 0.56 mmol) and potassium acetate (1.7 g,17.32 mmol) were dissolved to dioxane (40 mL), reacted under the protection of nitrogen at 80°C for 8 h. After the completion of the reaction by LCMS detection, the reaction solution was spin-dried, and extracted with EA. Organic phases were spin-dried and separated by silica gel column chromatography

(EA: PE = 1:5) to give 1 g of the product with a yield of 67.8%.

**(5) Preparation of 2-(3-fluoro-2-isopropylphenyl)-4-(methylthio)-7,8-dihydroquinazolin-5(6H)-one**

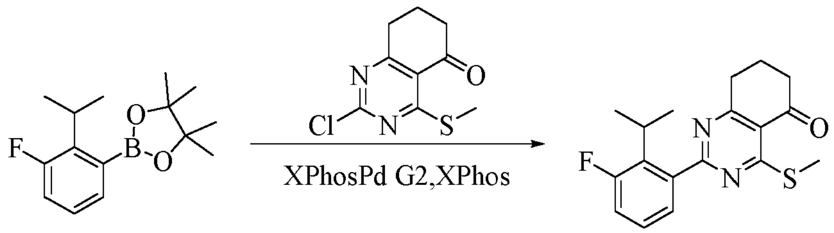


2-(3-fluoro-2-isopropylphenyl)-4,4,5,5-tetramethyl-1,3,2-dioxolane (1.0 g, 3.79 mmol), 2-chloro-4-(methylthio)-7,8-dihydroquinazolin-5(6H)-one (870 mg, 3.79 mmol), XPhosPdG2 (299 mg, 0.38 mmol), XPhos (362 mg, 0.76 mmol) and potassium phosphate (892 mg, 4.2 mmol) were dissolved to dioxane (26 mL) and water (7 mL), and reacted under the protection of nitrogen at 90°C for 2 h. After the completion of the reaction by LCMS detection, the reaction solution was spin-dried and separated by silica gel column chromatography (EA: PE = 1: 2) to give 350 mg of the product with a yield of 28.0%.

**(6) Preparation of 2-(3-fluoro-2-isopropylphenyl)-4-hydrazino-7,8-dihydroquinazolin-5(6H)-one**

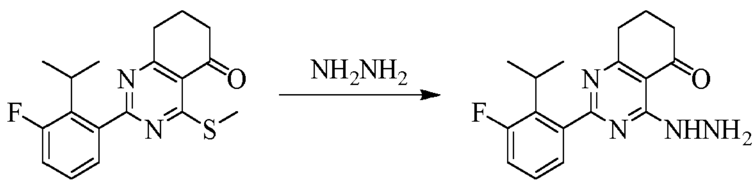


2-(3-fluoro-2-isopropylphenyl)-4-(methylthio)-7,8-dihydroquinazolin-5(6H)-one (350 mg, 1.06 mmol) was dissolved to ethanol (10 mL), added with hydrazine hydrate (64 mg, 1.27 mmol), and reacted at 70°C for 8 h. After the completion of the reaction by LCMS detection, the reaction solution was spin-dried and directly used for next reaction.

**(7) Preparation of 4-(3-fluoro-2-isopropylphenyl)-2,6,7,8-tetrahydropyrazolo[3,4,5-de]quinazoline**

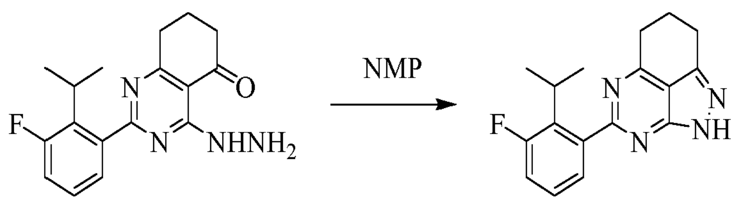


2-(3-fluoro-2-isopropylphenyl)-4-hydrazino-7,8-dihydroquinazolin-5(6*H*)-one (crude product) was dissolved to NMP (2 mL), and reacted at 160°C for 1 h with microwaves. After the completion of the reaction by LCMS detection, the reaction mixture was subjected to reversed-phase chromatography purification (MeOH: $H_2O$ = 0-50) to give 100 mg of the product with a two-step yield of 31.8%.

**(8) Preparation of 4-(3-fluoro-2-isopropylphenyl)-2-(4-(1-methyl-4-(trifluoromethyl)-1*H*-imidazol-2-yl)benzyl)-2,6,7,8-tetrahydropyrazolo[3,4,5-*de*]quinazoline**

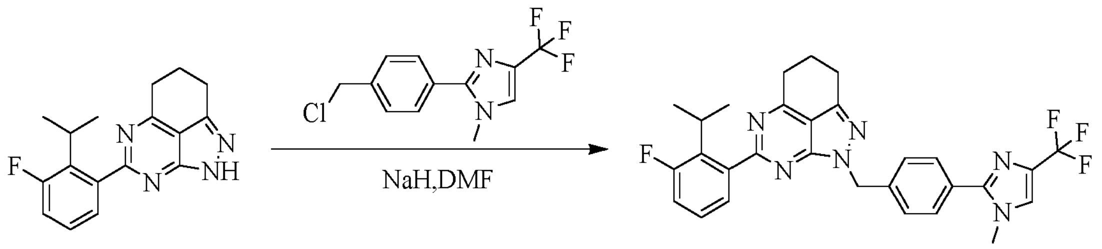


4-(3-fluoro-2-isopropylphenyl)-2,6,7,8-tetrahydropyrazolo[3,4,5-*de*]quinazoline (70 mg, 0.24 mmol) was dissolved to DMF (3 mL), added with NaH (60%, 19 mg, 0.48 mmol) in batches at 25°C, added with 2-(4-(chloromethyl) phenyl)-1-methyl-4-(trifluoromethyl)-1*H*-imidazole (77 mg, 0.28 mmol) after 10 min, and continuously reacted for 4 h. The reaction was completed by LCMS detection. The reaction solution was added with water (20 mL) for quenching, and extracted with dichloromethane. Organic phases were spin-dried, and the crude product was separated by silica gel column chromatography (EA: PE = 1: 1) to give 16 mg of the product with a yield of 12.5%.

Molecular formula: $C_{29}H_{26}F_4N_6$ Molecular weight: 534.6 LC-MS (M/e): 535.0 ($M+H^+$)

$^1$H-NMR(400MHz,DMSO) $\delta$: 7.90 (s, 1H), 7.60-7.70 (m, 2H), 7.40-7.50 (m,2H), 7.18-7.35 (m, 3H), 5.63 (s, 2H), 3.73 (s, 3H), 3.15-3.22 (m, 1H), 2.95-3.05 (m,4H), 2.20-2.30 (m, 2H), 1.25 (d, *J* = 6.8 Hz, 6H).

**Example 3 Preparation of 4-(3-fluoro-2-isopropylphenyl)-2-(4-(5-methyl-3-(trifluoromethyl)-1*H*-pyrazol-1-yl) benzyl)-2,6,7,8-tetrahydropyrazolo[3,4,5-*de*]quinazoline (Compound 3)**

**(1) Preparation of 4-(5-methyl-3-(trifluoromethyl)-1*H*-pyrazol-1-yl)methyl benzoate**

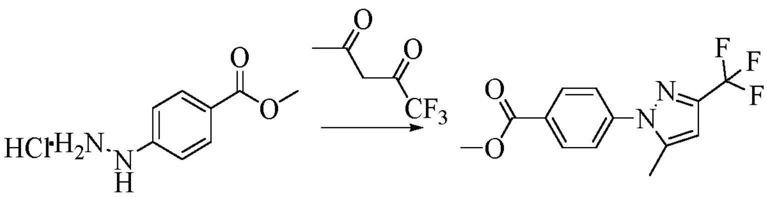

4-hydrazino-methylbenzoate hydrochloride (9.0 g, 44.4 mmol) and 1,1,1-trifluoropropane-2,4-dione (6.8 g, 44.1 mmol) were dissolved to hexafluoroisopropanol (70 mL), dropwise added with triethylamine (8.9 g, 87.9 mmol) at 0°C, and then reacted at 20°C for 2 h. The system was spin-dried, and the residue was purified by silica gel column chromatography (PE: EA = 10: 1) to give the product (9.0 g, with a yield of 71.3%).

**(2) Preparation of (4-(5-methyl-3-(trifluoromethyl)-1*H*-pyrazol-1-yl)phenyl)methanol**

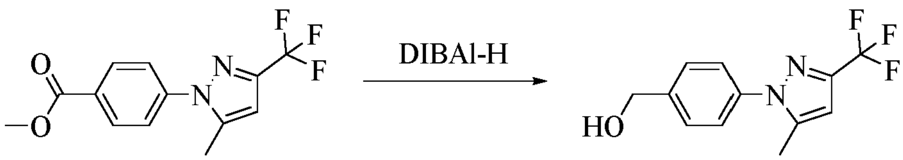


4-(5-methyl-3-(trifluoromethyl)-1*H*-pyrazol-1-yl)methyl benzoate (5.0 g, 17.6 mmol) was dissolved in THF (60 mL), dropwise added with DIBAl-H (1.5 mol/L, 46.9 mL, 70.4 mmol), and reacted at 20°C for 2 h after the addition was completed. Water was slowly added for quenching, added with saturated aqueous ammonium chloride solution (50 mL) and EA (150 mL) to extract for solution separation. Organic phases were spun-dried, and the residue was purified by silica gel column chromatography (PE: EA = 3: 1) to give the product (4.0 g, with a yielding of 88.7%).

**(3) Preparation of 1-(4-(chloromethyl)phenyl)-5-methyl-3-(trifluoromethyl)-1*H*-pyrazole**

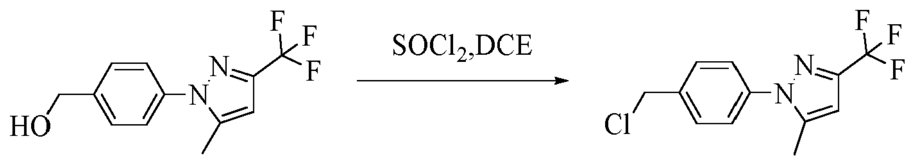


(4-(5-methyl-3-(trifluoromethyl)-1*H*-pyrazol-1-yl)phenyl)methanol (3.7 g, 14.4 mmol) was dissolved to dichloroethane (30 mL), added with sulfur dichloride (3 mL), and then reacted at 50°C for 1 h. The system was spin-dried, and the residue was purified by silica gel column chromatography (PE: EA = 5: 1) to give the product (3.6 g, with a yielding of 91.0%).

**(4) Preparation of 4-(3-fluoro-2-isopropylphenyl)-2-(4-(5-methyl-3-(trifluoromethyl)-1*H*-pyrazol-1-yl)benzyl)-2,6,7,8-tetrahydropyrazolo[3,4,5-*de*]quinazoline**

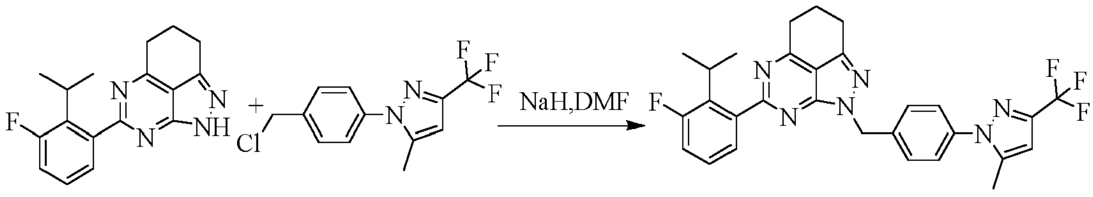


4-(3-fluoro-2-isopropylphenyl)-2,6,7,8-tetrahydropyrazolo[3,4,5-*de*]quinazoline (100 mg, 0.34 mmol) was dissolved to DMF (5 mL), and added with NaH (60%, 27mg, 0.68mmol) in batches at 25°C, then added with 1-(4-(chloromethyl)phenyl)-5-methyl-3-(trifluoromethyl)-1*H*-pyrazole (139 mg, 0.51 mmol) after 30 min, and continuously reacted for 2 h. Water (20 mL) was added to the reaction solution for quenching, then added with EA (30 mL) for extraction and solution separation. Organic phases were spin-dried, and the residue was purified by silica gel column chromatography (PE: EA = 3: 1) to give 20 mg of crude product, which was subjected to C18 reversed purification (water/methanol = 2/8) and freeze-dried to give the product (6 mg, with a yield of 3.3%).

Molecular formula: $C_{29}H_{26}F_4N_6$

Molecular weight: 534.6 LC-MS (M/e): 535.0 ($M+H^+$)

$^1$H-NMR(400MHz, $CDCl_3$) $\delta$: 7.65-7.61(m,2H), 7.42-7.38(m,2H), 7.36-7.33 (m, 1H), 7.27-7.23(m,1H), 7.13-7.08(m,1H), 6.43(s,1H), 5.63(m,2H), 3.18-3.11(m,1H), 3.12-3.03 (m, 4H), 2.44-2.38(m,2H), 2.32(s,3H), 1.36 (d, *J* = 6.8 Hz, 6H).

**Example 4 Preparation of 2-(4-(1-methyl-4-(trifluoromethyl)-1*H*-imidazol-2-yl)benzyl)-4-(4-(trifluoromethyl)pyridin-3-yl)-2,6,7,8-tetrahydropyrazolo[3,4,5-*de*]quinazoline (Compound 10)**

**(1) Preparation of (4-(trifluoromethyl)pyridin-3-yl)boric acid**

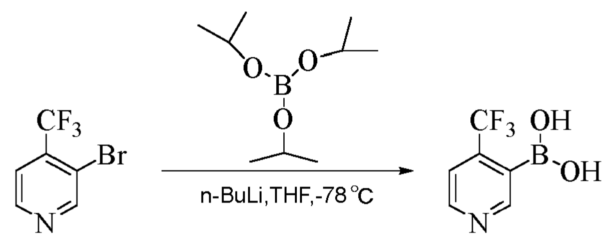

3-bromo-4-(trifluoromethyl)pyridine (9.2 g, 0.041 mol) was dissolved to THF (200 mL), added with boron isopropoxide (10.8 g, 0.057 mol), added with n-BuLi (2.5 M) (22.9 mL, 0.057 mol) at -78°C and reacted for 3 h. After 50.0 mL of water was added for quenching, the reaction mixture was adjusted to a pH of 4 by 4N hydrochloric acid, then adjusted to a pH of 8 by saturated sodium hydrogen carbonate solution and extracted with ethyl acetate. Organic phases were concentrated to give the crude product directly used in next reaction.

**(2) Preparation of 4-(methylthio)-2-(4-(trifluoromethyl)pyridin-3-yl)-7,8-dihydroquinazolin-5(6*H*)-one**

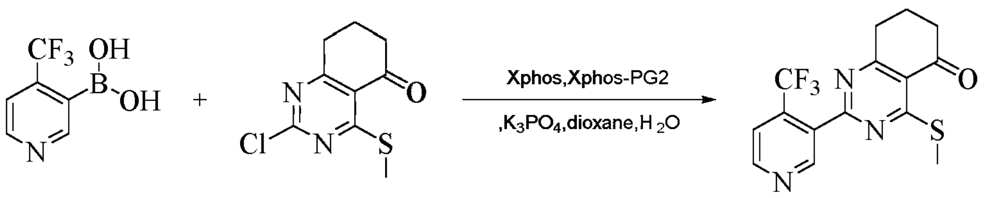


2-chloro-4-(methylthio)-7,8-dihydroquinazolin-5(6*H*)-one (1.5 g, 6.6 mmol) was dissolved to 1,4-dioxane (75 mL), added with (4-(trifluoromethyl)pyridin-3-yl)boric acid (crude product from the above step), Xphos-Pd-G2 (514.7 mg, 0.66 mmol), Xphos (625.3 mg, 1.3 mmol), $K_3PO_4$ (1.5 g, 7.2 mmol), and water (25 mL), and reacted under the protection of $N_2$ at 90°C for 3 h. After the reaction, the solvent was spin-dried, and the residue was subjected to normal phase preparation and separation (ethyl acetate/petroleum ether = 0-50%) to give 535.0 mg of the target product.

**(3) Preparation of 4-hydrazino-2-(4-(trifluoromethyl)pyridin-3-yl)-7,8-dihydroquinazolin-5(6*H*)-one**

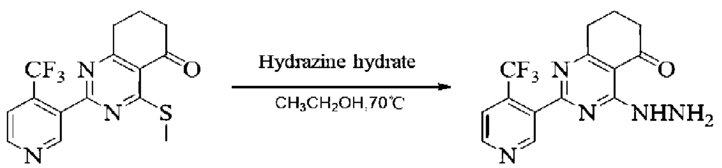


4-(methylthio)-2-(4-(trifluoromethyl)pyridin-3-yl)-7,8-dihydroquinazolin-5(6*H*)-one (0.48 g, 1.4 mmol) was dissolved to ethanol (20.0 mL), added with hydrazine hydrate (248.0 mg, 5.0 mmol) and reacted at 80°C for 3 h. After the reaction, the solvent was spin-dried and the residue was subjected to normal phase preparation and separation (ethyl acetate/petroleum ether = 0-70%) to give 240.0 mg of the target product with a yielding of 52.2%.

**(4) Preparation of 4-(4-(trifluoromethyl)pyridin-3-yl)-2,6,7,8-tetrahydropyrazolo[3,4,5-*de*]quinazoline**

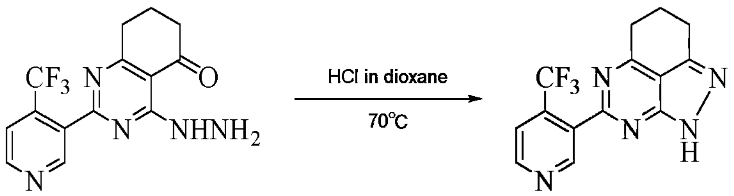


4-hydrazino-2-(4-(trifluoromethyl)pyridin-3-yl)-7,8-dihydroquinazolin-5(6*H*)-one (200.0 mg, 0.62 mmol) was dissolved to HCL 1,4-dioxane solution (4 M,18 mL), and reacted at 70°C for 1 h. After the reaction, the solvent was spin-dried, and the reaction mixture was added with a saturated sodium bicarbonate solution to adjust a pH to neutrality, and then extracted with ethyl acetate. Organic phases were subjected to normal phase preparation and separation (ethyl acetate/petroleum ether = 0-50%) to give the product (130.0 mg, with a yield of 68.8%).

**(5) Preparation of 2-(4-(1-methyl-4-(trifluoromethyl)-1*H*-imidazol-2-yl)benzyl)-4-(4-(trifluoromethyl)pyridin-3-yl)-2,6,7,8-tetrahydropyrazolo[3,4,5-*de*]quinazoline**

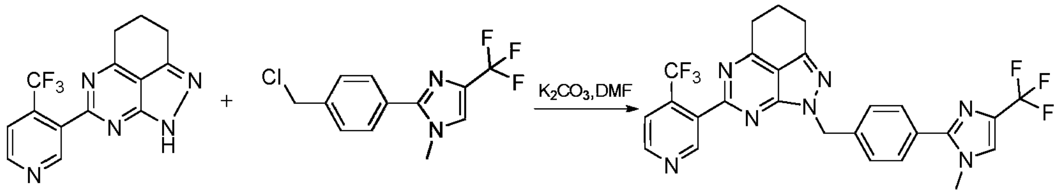


4-(4-(trifluoromethyl)pyridin-3-yl)-2,6,7,8-tetrahydropyrazolo[3,4,5-*de*]quinazoline (105 mg, 0.34 mmol) was dissolved to DMF (4 mL), added with potassium carbonate (143 mg, 1.03 mmol), then added with 2-(4-(chloromethyl) phenyl)-1-methyl-4-(trifluoromethyl)-1*H*-imidazole (71 mg, 0.26 mmol), and reacted at 50°C for 5 h. After the reaction, the reaction solution was subjected to normal phase column chromatography separation (methanol/water = 0-10%) to give 8.8 mg of the product with a yield of 4.7%.

Molecular formula: $C_{26}H_{19}F_6N_7$

Molecular weight: 543.48 LC-MS (M/e): 544.2(M+H$^+$)

$^1$H-NMR (400 MHz, $CDCl_3$) $\delta$: 9.13(s, 1H), 7.97(d, 1H), 7.69 (d, 1H), 7.6(d, 2H), 7.5(d, 2H), 7.28(s,1H), 5.63(d,2H), 3.73 (s, 3H), 3.12-3.08 (m, 4H), 2.43-2.37 (m, 2H).

**Example 5 Preparation of 4-(4-cyclopropyl-6-methoxypyrimidine-5-yl)-2-(4-(1-methyl-4-(trifluoromethyl)-1*H*-imidazol-2-yl)benzyl)-6,7,8,9-tetrahydro-2*H*-1,2,3,5-tetraazabenzo[*cd*]pyrene (Compound 11)**

**(1) Preparation of 2-(bis(methylthio)methylene)cycloheptane-1,3-dione**

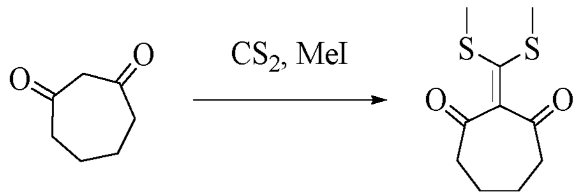


1,3-cycloheptanedione (9 g, 71.4 mmol) was dissolved to DMF (200 mL), added with potassium carbonate (30 g, 214.2 mmol), and reacted at 20°C for 0.5 h. Carbon disulphid (8.1 g, 106.4 mmol) was added and reacted at 20°C for 1 h. Methyl iodide (30.4 g, 214.2 mmol) was added and reacted at 20°C for 1 h. The reaction mixture was concentrated to give a crude product used directly in next step.

**(2) Preparation of 2-amino-4-(methylthio)-6,7,8,9-tetrahydro-5*H*-cyclohepta[*d*]pyrimidin-5-one**

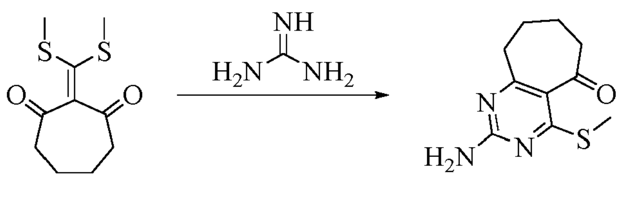

2-(bis(methylthio)methylene)cycloheptane-1,3-dione (crude product from the above step) was dissolved in DMF (200 mL), and added with guanidine hydrochloride (6.8 g, 71.4 mmol) and potassium carbonate (14.8 g, 107.1 mmol), reacted at 100°C for 16 h, cooled to 25°C, added with water (300 mL), and filtered to obtain a filter cake, and then dried in vacuum to give 10.4 g of the product with a two-step yielding of 65.0%.

**(3) Preparation of 2-chloro-4-(methylthio)-6,7,8,9-tetrahydro-5*H*-cyclohepta[*d*]pyrimidin-5-one**

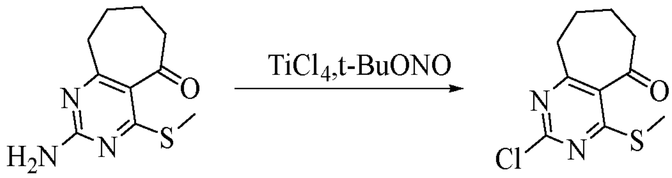


2-amino-4-(methylthio)-6,7,8,9-tetrahydro-5H-cyclohepta[d]pyrimidin-5-one (10.4 g, 46.6 mmol) was dissolved to dichloromethane (150 mL), added with titanium tetrachloride (8.8 g, 46.4 mmol) and tert-butyl nitrite ( (28.7 g, 278.6 mmol), and reacted at 25°C for 3 h. Water was added for quenching, and the reaction mixture was filtered to obtain a filtrate, concentrated and subjected to column chromatography (ethyl acetate/petroleum ether = 0-30%) to give 1.2 g of the product with a yield of 10.6%.

**(4) Preparation of 2-(4-cyclopropyl-6-methoxypyrimidin-5-yl)-4-(methylthio)-6,7,8,9-tetrahydro-5*H*-cyclohepta [*d*]pyrimidin-5-one**

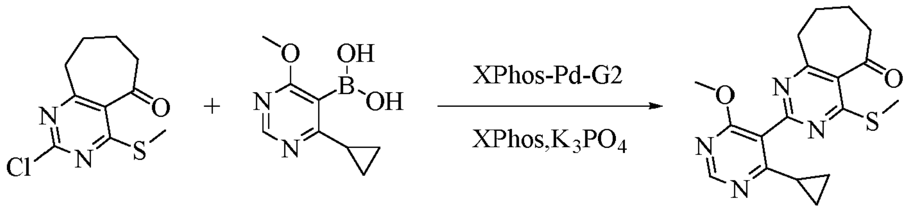


2-chloro-4-(methylthio)-6,7,8,9-tetrahydro-5*H*-cyclohepta[d]pyrimidin-5-one (800 mg, 3.3 mmol) was dissolved to 1,4-dioxane (18 mL), added with (4-cyclopropyl-6-methoxypyrimidin-5-yl)boric acid (640 mg, 3.3 mmol), Xphos-Pd-G2 (259 mg, 0.33 mmol), Xphos (315 mg, 0.66 mmol), potassium phosphate (770 mg, 3.6 mmol) and water (6 mL), and reacted under the protection of nitrogen at 90°C for 3 h. After the reaction, the solvent was spin-dried, and the residue was subjected to normal phase preparation and separation (ethyl acetate/petroleum ether = 0-50%) to give 400 mg of the product with a yield of 34.0%.

**(5) Preparation of 4-(4-cyclopropyl-6-methoxypyrimidine-5-yl)-6,7,8,9-tetrahydro-2*H*-1,2,3,5-tetraazabenzo[*cd*] pyrene**

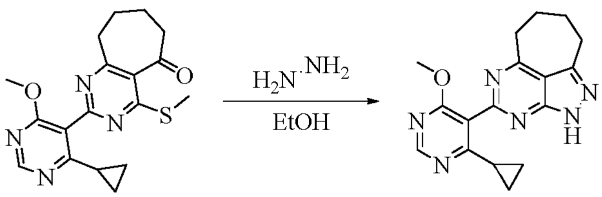


2-(4-cyclopropyl-6-methoxypyrimidin-5-yl)-4-(methylthio)-6,7,8,9-tetrahydro-5*H*-cyclohepta[*d*]pyrimidin-5-one (200 mg, 0.56 mmol) was dissolved to ethanol (5 mL), added with hydrazine hydrate (84 mg, 1.69 mmol), and reacted at 80°C for 3 h. After the reaction, the solvent was spin-dried and the residue was subjected to normal phase preparation and separation (ethyl acetate/petroleum ether = 0-70%) to give 150 mg of the product with a yield of 82.9%.

**(6) Preparation of 4-(4-cyclopropyl-6-methoxypyrimidine-5-yl)-2-(4-(1-methyl-4-(trifluoromethyl)-1*H*-imidazol-2-yl)benzyl)-6,7,8,9-tetrahydro-2*H*-1,2,3,5-tetraazabenzo[*cd*]pyrene**

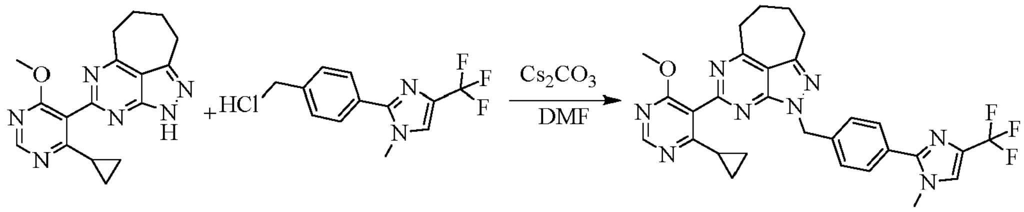


4-(4-cyclopropyl-6-methoxypyrimidine-5-yl)-6,7,8,9-tetrahydro-2*H*-1,2,3,5-tetraazabenzo[*cd*]pyrene (70 mg, 0.22 mmol) was dissolved to DMF (4 mL), added with cesium carbonate (143 mg, 0.44 mmol), and reacted at 20°C for 10 min, then added with 2-(4-(chloromethyl)phenyl)-1-methyl-4-(trifluoromethyl)-1H-imidazole (71 mg, 0.26 mmol), and reacted at 20°C for 3 h. After the reaction, the reaction solution was subjected to reversed column chromatography separation (methanol/water = 0-70%) to give 25 mg of the product with a yield of 20.2%.

Molecular formula: $C_{29}H_{27}F_3N_8O$

Molecular weight: 560.6 LC-MS (M/e): 561.2(M+H$^+$)

$^1$H-NMR (400 MHz, $CDCl_3$) $\delta$: 8.70(s, 1H), 7.97(s, 1H), 7.92-7.89 (m, 2H), 7.43-7.41(m, 2H), 5.67(s, 2H), 3.92(s,3H), 3.75(s,3H), 3.25-3.08 (m, 4H), 2.07-2.00 (m, 4H), 1.69-1.55 (m, 1H), 1.22-1.18 (m, 2H), 0.95-0.85 (m, 2H).

**Example 6 Preparation of 4-(4-cyclopropyl-6-methoxypyrimidine-5-yl)-2-(4-(1-isopropyl-4-(trifluoromethyl)-1H-imidazol-2-yl)benzyl)-2,6,7,8-tetrahydropyrazolo[3,4,5-*de*]quinazoline (Compound 16)**

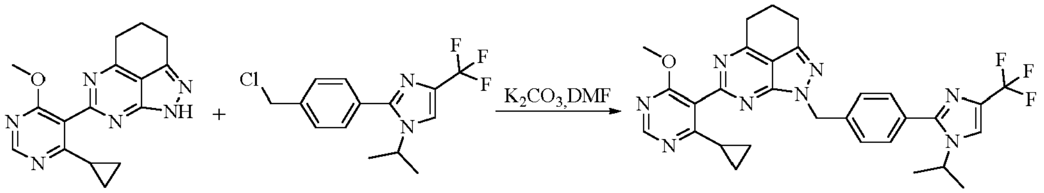


4-(4-cyclopropyl-6-methoxypyrimidin-5-yl)-2,6,7,8-tetrahydropyrazolo[3,4,5-*de*]quinazoline (100 mg, 0.32 mmol) was dissolved to DMF (4 mL), added with potassium carbonate (88 mg, 0.64 mmol) and 2-(4-(chloromethyl) phenyl)-1-isopropyl-4-(trifluoromethyl)-1*H*-imidazole (98 mg, 0.32 mmol), and reacted at 70°C for 5 h. After the reaction, the reaction mixture was subjected to column chromatography separation (methanol/water = 0-40%) to give 21 mg of the product with a yield of 11.4%.

Molecular formula: $C_{30}H_{29}F_3N_8O$

Molecular weight: 574.6 LC-MS (M/e): 575.2(M+H$^+$)

$^1$H-NMR (400 MHz, DMSO): 8.67(s, 1H), 7.53-7.51 (m, 4H), 7.48 (s, 1H), 5.68(s, 2H), 4.56-4.55(m, 1H), 3.95(s,3H), 3.15-3.00 (m, 4H), 2.45-2.40(m,2H), 1.69-1.55 (m, 1H), 1.53-1.32 (m, 6H), 1.42-1.21 (m, 2H) , 0.96-0.87 (m, 2H).

**Example 7 Preparation of 4-(1-isopropyl-4-methyl-1H-pyrazol-5-yl)-2-(4-(1-methyl-4-(trifluoromethyl)-1*H*-imidazol-2-yl)benzyl)-2,6,7,8-tetrahydropyrazolo[3,4,5-*de*]quinazoline (Compound 18)**

**(1) Preparation of 2-(1-isopropyl-4-methyl-1H-pyrazol-5-yl)-4-(methylthio)-7,8-dihydroquinazolin-5(6*H*)-one**

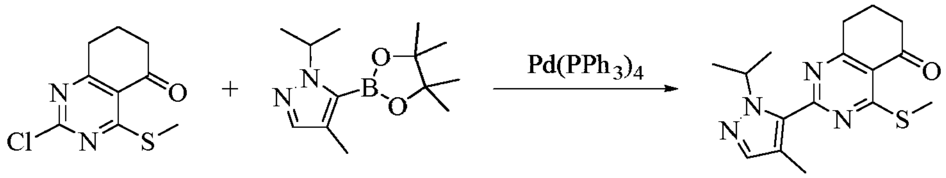


2-chloro-4-(methylthio)-7,8-dihydroquinazolin-5(6*H*)-one (1.0 g, 4.4 mmol) and 1-isopropyl-4-methyl-5-(4,4,5,5-tetramethyl-1,3,2-dioxaborolan-2-yl)-1H-pyrazole (1.3 g, 5.2 mmol) were dissolved to 1,4-dioxane (20 mL) and water (4 mL), then added with tetratriphenylphosphine palladium (760 mg, 0.66 mmol) and sodium carbonate (950 mg, 9.0 mmol). Under the protection of $N_2$, the reaction mixture reacted at 90°C for 3 h. After the reaction, the reaction mixture was concentrated and purified by silica gel column chromatography (ethyl acetate: normal heptane = 35%) to give 700 mg of the product with a yield of 50.6%.

**(2) Preparation of 4-hydrazino-2-(1-isopropyl-4-methyl-1*H*-pyrazol-5-yl)-7,8-dihydroquinazolin-5(6*H*)-one**

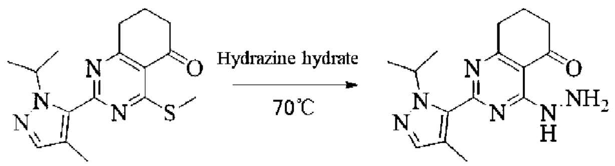


2-(1-isopropyl-4-methyl-1*H*-pyrazol-5-yl)-4-(methylthio)-7,8-dihydroquinazolin-5(6*H*)-one (500 mg, 1.6 mmol) was dissolved to anhydrous ethanol (10 mL), added with 98%hydrazine hydrate (180 mg, 3.5 mmol), and reacted at 70°C for 7 h. After the reaction, the reaction mixture was concentrated to give crude product directly used for next reaction.

**(3) Preparation of 4-(1-isopropyl-4-methyl-1*H*-pyrazol-5-yl)-2,6,7,8-tetrahydropyrazolo[3,4,5]quinazoline**

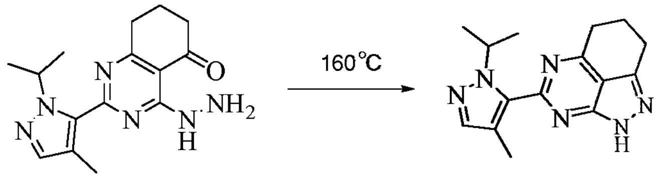


The crude product from the above step was dissolved to NMP (10 mL), added with p-toluenesulfonic acid (50 mg), and the system reacted at 160°C for 1.5 h with microwaves. After the reaction, the reaction mixture was washed with water, extracted with ethyl acetate, subjected to solution separation and concentrated, and purified by silica gel column chromatography (normal heptane: ethyl acetate = 1: 1) to give 399 mg of the target product with a two-step yield of 89.4%.

**(4) Preparation of 4-(1-isopropyl-4-methyl-1H-pyrazol-5-yl)-2-(4-(1-methyl-4-(trifluoromethyl)-1*H*-imidazol-2-yl) benzyl)-2,6,7,8-tetrahydropyrazolo[3,4,5-*de*]quinazoline**

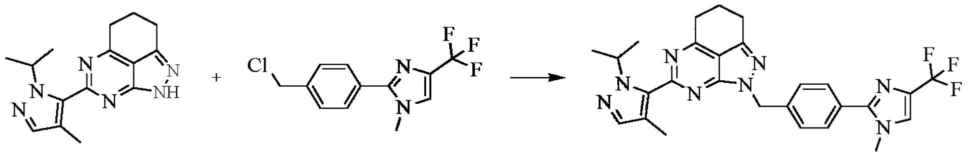

4-(1-isopropyl-4-methyl-1*H*-pyrazol-5-yl)-2,6,7,8-tetrahydropyrazolo[3,4,5-*de*]quinazoline (150 mg, 0.53 mmol), 2-(4-(chloromethyl)phenyl)-1-methyl-4-(trifluoromethyl)-1*H*-imidazole (218 mg, 0.79 mmol), cesium carbonate (518 mg, 1.6 mmol) and DMF (10 mL) were weighed in turn, and reacted at 50°C for 13 h. After LC-MS showed that the

reaction was completed, the reaction solution was diluted with water, extracted with EA. Organic phases were spin-dried, and the residue was separated by Prep-TLC ($SiO_2$, PE: EA = 1: 2, Rf = 0.5), and then separated by reversed column chromatography (C18, $H_2O$: MeOH = 90:10 to 65:35) to give 150 mg of the product with a yield of 54.2%.

Molecular formula: $C_{27}H_{27}F_3N_8$

Molecular weight: 520.6 LC-MS (m/z): 521.3 ($M+H^+$)

$^1$H-NMR(400 MHz, $CDCl_3$): 7.52 (d, *J*=8.4, 2H), 7.61 (d, *J*=8.4, 2H), 7.47 (s, 1H), 7.30 (s, 1H), 5.65 (s, 2H), 5.44-5.37 (m, 1H), 3.75 (s, 3H), 3.11-3.07 (m, 4H), 2.43-2.36 (m, 2H), 2.32 (s, 3H), 1.56-1.53 (m, 6H).

**Example 8 Preparation of 2-(4-(1-isopropyl-4-(trifluoromethyl)-1*H*-imidazol-2-yl)benzyl)-4-(1-isopropyl-4-methyl-1*H*-pyrazol-5-yl)-2,6,7,8-tetrahydropyrazolo[3,4,5-*de*]quinazoline (Compound 19)**

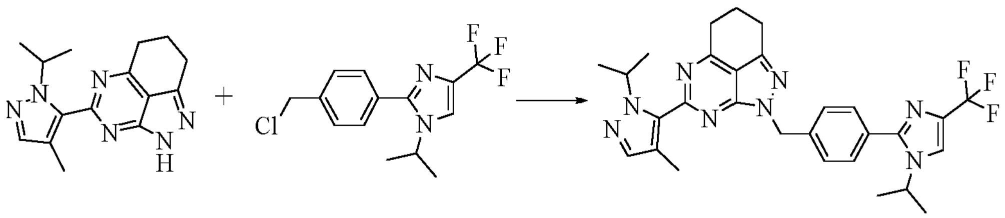

4-(1-isopropyl-4-methyl-1*H*-pyrazol-5-yl)-2,6,7,8-tetrahydropyrazolo[3,4,5-*de*]quinazoline (120 mg, 0.42 mmol) was dissolved to DMF (5 mL), and added with 2-(4-(chloromethyl)phenyl)-1-isopropyl-4-(trifluoromethyl)-1*H*-imidazole (120 mg, 0.40 mmol) and potassium carbonate (150 mg, 1.1 mmol). The reaction mixture reacted at 50°C for 2 h. After the reaction, the reaction mixture was filtered, concentrated and purified by silica gel plate (ethyl acetate: normal heptane = 2: 1) to give 23 mg of the target product with a yield of 10.5%.

Molecular formula: $C_{29}H_{31}F_3N_8$

Molecular weight: 548.6 LC-MS (M/e): 549.4($M+H^+$)

$^1$H-NMR (400 MHz, $CDCl_3$) $\delta$:7.92-7.69(m, 4H), 7.47-7.28 (m,2H), 5.89 (s, 2H), 5.41 (s, 1H), 4.54 (s,1H), 3.19-3.01 (m,4H), 2.5 (s,2H), 2.40 (s,3H), 1.72-1.5 (m, 6H), 1.55-1.43 (m, 6H).

**Example 9 Preparation of 4-(4-cyclopropyl-6-(difluoromethoxy)pyrimidin-5-yl)-2-(4-(1-methyl-4-(trifluoromethyl)-1*H*-imidazol-2-yl)benzyl)-2,6,7,8-tetrahydropyrazolo[3,4,5-*de*]quinazoline (Compound 20)**

**(1) Preparation of 2-(bis(methylthio)methylene)cyclohexane-1,3-dione**

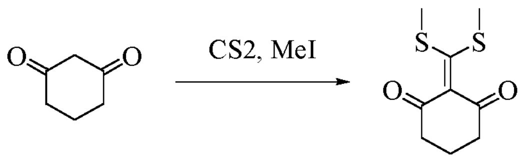


1-3-cyclohexanedione (12.5 g, 0.11 mol) was dissolved to DMF (100 mL), added with potassium carbonate (45.6 g, 0.33 mol) and reacted at 20°C for 0.5 h. Carbon disulphid (12.9 g, 0.17 mol) was added and reacted at 20°C for 1 h. Methyl iodide (46.8 g, 0.33 mol) was added and reacted at 20°C for 1 h. The reaction mixture was concentrated to give the product used directly in next step.

**(2) Preparation of 2,4-bis(methylthio)-7,8-dihydroquinazolin-5(6*H*)-one**

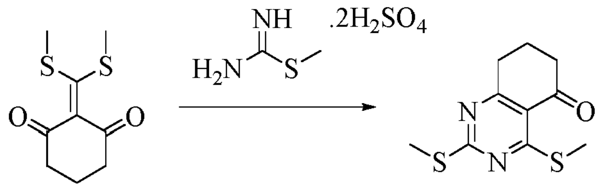


2-(bis(methylthio)methylene)cyclohexane-1,3-dione (crude product from the above step) was dissolved to DMF (200 mL), added with methylisothiourea disulfate (11.5 g, 40.1 mmol) and potassium carbonate (18.5 g, 133.8 mmol), reacted at 100°C for 16 h, cooled to 25°C, added with water (300 mL), filtered to obtain a filter cake. The filter cake was dried in vacuum to give 2.48 g of the product with a yield of 26%.

**(3) Preparation of 4-hydrazino-2-(methylthio)-7,8-dihydroquinazolin-5(6*H*)-one**

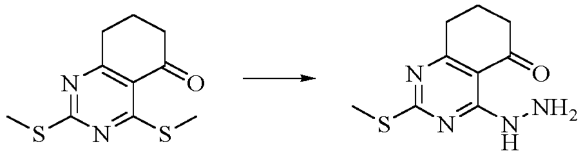

2,4-bis(methylthio)-7,8-dihydroquinazolin-5(6*H*)-one (2.0 g, 8.3 mmol) was dissolved to ethanol (20 mL), added with hydrazine hydrate (1.0 g, 20.0 mmol), and reacted at 70°C for 3 h. After the reaction, the reaction mixture was concentrated to give crude product.

**(4) Preparation of 4-(methylthio)-2,6,7,8-tetrahydropyrazolo[3,4,5-*de*]quinazoline**

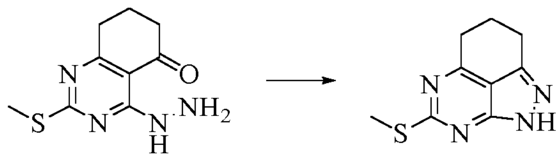

4-hydrazino-2-(methylthio)-7,8-dihydroquinazolin-5(6*H*)-one (crude product from the above step) was dissolved to DMAc (20 ml), added with p-toluenesulfonic acid (0.25 g, 1.5 mmol), stirred and heated to 160°C, and reacted for 2 h. Then, the reaction was stopped, and the DMAc was spin-evaporated to give the crude product, which was separated by normal-phase chromatographic column (petroleum ether: ethyl acetate = 10:0 to 4:6) to give 0.45 g of the product with a yield of 26.3% (two-step).

**(5) Preparation of 2-(4-(1-methyl-4-(trifluoromethyl)-1*H*-imidazol-2-yl)benzyl)-4-(methylthio)-2,6,7,8-tetrahydropyrazolo[3,4,5-*de*]quinazoline**

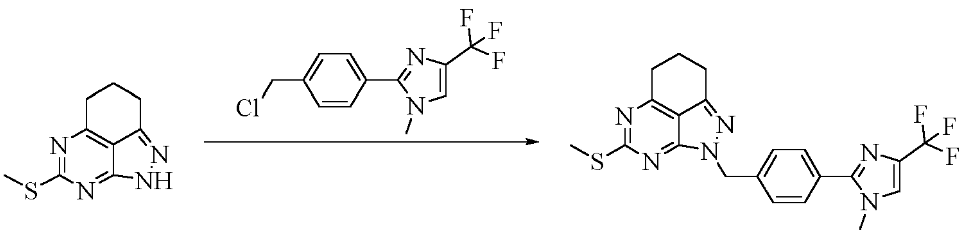

4-(methylthio)-2,6,7,8-tetrahydropyrazolo[3,4,5-*de*]quinazoline (206 mg, 1.0 mmol), 2-(4-(chloromethyl)phenyl)-1-methyl-4-(trifluoromethyl)-1*H*-imidazole (411 mg, 1.5 mmol) and potassium carbonate (414 mg, 3.0 mmol) were

added into DMF (20 ml), and heated to 80°C. After 4 hours of reaction, the reaction was stopped, the reaction mixture was filtered, and the filtrate was spin-evaporated to give a crude product, which was separated by normal phase column (petroleum ether: ethyl acetate = 10:0 to 4:6) to give 200 mg of the product with a yield of 45%.

**(6) Preparation of 2-(4-(1-methyl-4-(trifluoromethyl)-1*H*-imidazol-2-yl)benzyl)-4-(methylsulfonyl)-2,6,7,8-tetrahydropyrazolo[3,4,5-*de*]quinazoline**

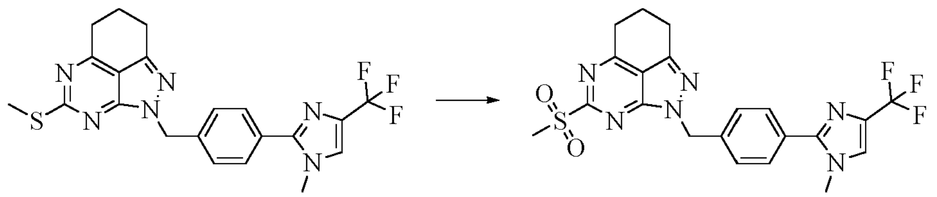

2-(4-(1-methyl-4-(trifluoromethyl)-1*H*-imidazol-2-yl)benzyl)-4-(methylthio)-2,6,7,8-tetrahydropyrazolo[3,4,5-de]quinazoline (200 mg, 0.45 mmol) was dissolved to dichloromethane (10 mL), added with metachloroperbenzoic acid (155 mg, 0.90 mmol), reacted at 20°C for 1 h. Then, the reaction was stopped, and the reaction mixture was added with 50 ml of dichloromethane, 50 ml of saturated aqueous sodium carbonate solution and stirred for 5 min, stood for solution separation, and organic phases were collected and spin-evaporated to give a crude product, which was directly used in the next step.

**(7) Preparation of 2-(4-(1-methyl-4-(trifluoromethyl)-1*H*-imidazol-2-yl)benzyl)-2,6,7,8-tetrahydropyrazolo[3,4,5-*de*]quinazoline-4-ol**

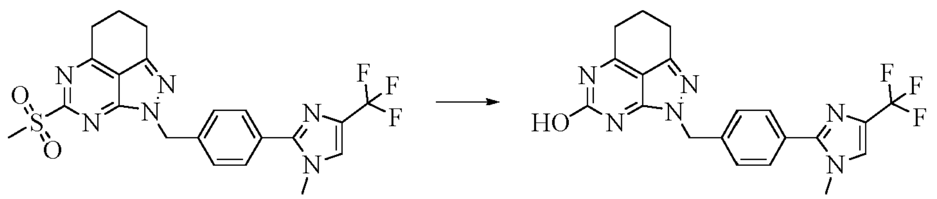

2-(4-(1-methyl-4-(trifluoromethyl)-1*H*-imidazol-2-yl)benzyl)-4-(methylsulfonyl)-2,6,7,8-tetrahydropyrazolo [3,4,5-de]quinazoline (crude product from the above step) was dissolved to tetrahydrofuran (20 mL), added with 10 ml of 10% KOH aqueous solution, stirred at 20 °C for 4 h, then the reaction was stopped, and a pH was adjusted to 4-5 by adding 1M HCl. The solvent in the reaction solution was spin-evaporated, and 200 ml of mixed solvent of dichloromethane and methanol (10:1) was added to elute the product, which was spin-evaporated to give a crude product. 2 ml of ethyl acetate was added into the reaction solution, stirred evenly, and filtered to give 200 mg of the product (crude product).

**(8) Preparation of 4-chloro-2-(4-(1-methyl-4-(trifluoromethyl)-1*H*-imidazol-2-yl)benzyl)-2,6,7,8-tetrahydropyrazolo[3,4,5-*de*]quinazoline**

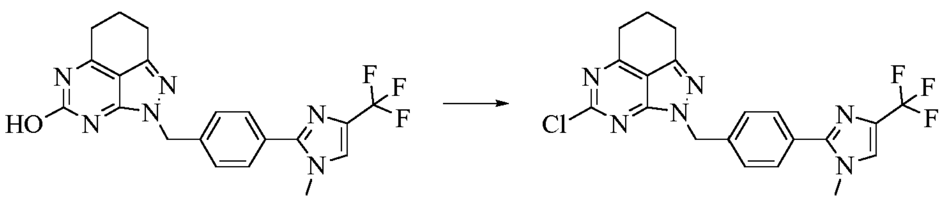

2-(4-(1-methyl-4-(trifluoromethyl)-1*H*-imidazol-2-yl)benzyl)-2,6,7,8-tetrahydropyrazolo[3,4,5-*de*]quinazoline-4-ol (200 mg of crude product) was dissolved to phosphorus oxychloride (6 mL), reacted at 110°C for 4 h. LCMS showed that the reaction was completed. The reaction solution was spin-dried, the residue was diluted with saturated

sodium bicarbonate solution, and extracted with EA. Organic phases were spin-dried, and the residue was separated by column chromatography ($SiO_2$, PE: EA = 4:1 to 2:3) to give 100 mg of the product.

**(9) Preparation of 4-(4-cyclopropyl-6-(difluoromethoxy)pyrimidin-5-yl)-2-(4-(1-methyl-4-(trifluoromethyl)-1*H*-imidazol-2-yl)benzyl)-2,6,7,8-tetrahydropyrazolo[3,4,5-*de*]quinazoline**

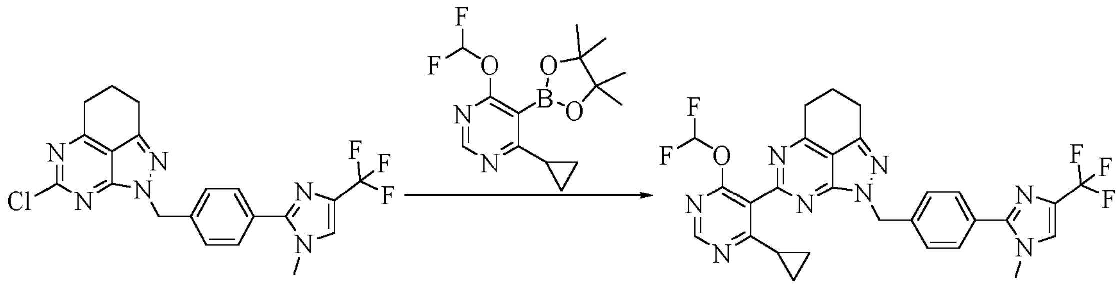

4-chloro-2-(4-(1-methyl-4-(trifluoromethyl)-1*H*-imidazol-2-yl)benzyl)-2,6,7,8-tetrahydropyrazolo[3,4,5-*de*]quinazoline (90 mg, 0.21 mmol), 4-cyclopropyl-6-(difluoromethoxy)-5-(4,4,5,5-tetramethyl-1,3,2-dioxobenzofuran-2-yl)pyrimidine (330 mg of crude product), XPhos-Pd-G2 (17 mg, 0.022 mmol), XPhos (10 mg, 0.021 mmol), potassium phosphate (134 mg, 0.63 mmol), dioxane (5 mL) and water (1 mL) were weighed, and then reacted at 90°C for 2 h. LCMS showed that the reaction was completed, the reaction solution was spin-dried, and the residue was separated by column chromatography ($SiO_2$, PE: EA=4: 1 to 2: 3) to give 40 mg of the product with a yield of 33.0%.

Molecular formula: $C_{28}H_{23}F_5N_8O$

Molecular weight: 582.5 LC-MS (m/z):583.1 ($M+H^+$)

$^1$H-NMR(400 MHz, $CDCl_3$) $\delta$: 8.66 (s, 1H), 7.72-7.20 (m, 6H), 5.64 (s, 2H), 3.72 (s, 3H), 3.15-3.05 (m, 4H), 2.45-2.35 (m, 2H), 1.85-1.75 (m, 1H), 1.30-1.20 (m, 2H), 1.05-0.95 (m, 2H).

**Example 10 Preparation of 4-(4-cyclopropyl-6-(methoxy-*d*$_3$)pyrimidin-5-yl)-2-(4-(1-methyl-4-(trifluoromethyl)-1*H*-imidazol-2-yl)benzyl)-2,6,7,8-tetrahydropyrazolo[3,4,5-de]quinazoline (Compound 1-1)**

**(1) Preparation of 2-(4-cyclopropyl-6-(methoxy-*d*$_3$)pyrimidin-5-yl)-4-(methylthio)-7,8-dihydroquinazolin-5(6*H*)-one**

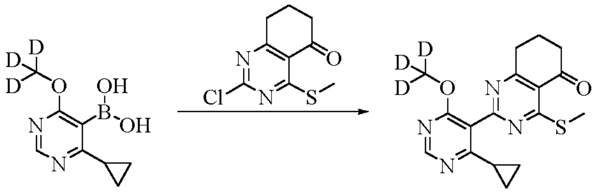

2-chloro-4-(methylthio)-7,8-dihydroquinazolin-5(6H)-one (1.2 g, 5.2 mmol) was dissolved to 1,4-dioxane (30 mL), added with (4-cyclopropyl-6-(methoxy-*d*$_3$)pyrimidin-5-yl)boronic acid (1 g, 5.1 mmol), Xphos-Pd-G2 (408 mg, 0.52 mmol), Xphos (496 mg, 1.04 mmol), $K_3PO_4$ (1.2 g, 5.7 mmol) and water (10 mL), and reacted at 90°C for 3 h under the protection of $N_2$. After the reaction, the solvent was spin-dried to give 600 mg of the product by normal phase preparation and separation (ethyl acetate/petroleum ether = 0-50%) with a yield of 34.1%.

**(2) Preparation of 2-(4-cyclopropyl-6-(methoxy-*d*$_3$)pyrimidin-5-yl)-4-hydrazino-7,8-dihydroquinazolin-5(6*H*)-one**

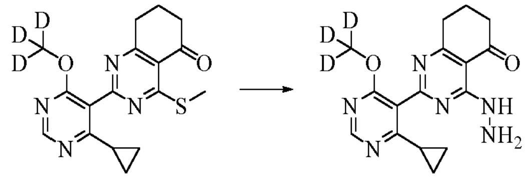

2-(4-cyclopropyl-6-(methoxy-*d*$_3$)pyrimidin-5-yl)-4-(methylthio)-7,8-dihydroquinazolin-5(6*H*)-one (500 mg, 1.4 mmol) was dissolved to ethanol (10 mL), added with hydrazine hydrate (87 mg, 1.7 mmol), and reacted at 70°C for 3 h. After the reaction, the reaction mixture was concentrated to give 400 mg of the product with a yielding of 86.8%.

**(3) Preparation of 4-(4-cyclopropyl-6-(methoxy-*d*$_3$)pyrimidin-5-yl)-2,6,7,8-tetrahydropyrazolo[3,4,5-*de*]quinazoline**

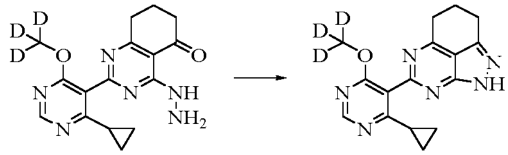

2-(4-cyclopropyl-6-(methoxy-*d*$_3$)pyrimidin-5-yl)-4-hydrazino-7,8-dihydroquinazolin-5(6*H*)-one (300 mg, 0.92 mmol) was dissolved to NMP (5 mL) and ethanol (60 mL), and reacted at 160°C for 1 h with microwaves. 150 mg of the product was given by column chromatography separation (methanol/water=0-60%) with a yield of 53%.

**(4) Preparation of 4-(4-cyclopropyl-6-(methoxy-*d*$_3$)pyrimidin-5-yl)-2-(4-(1-methyl-4-(trifluoromethyl)-1*H*-imidazol-2-yl)benzyl)-2,6,7,8-tetrahydropyrazolo[3,4,5-*de*]quinazoline**

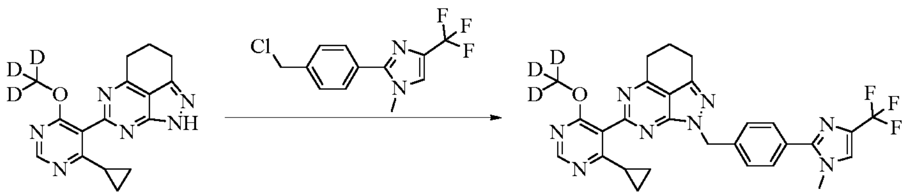

4-(4-cyclopropyl-6-(methoxy-*d*$_3$)pyrimidin-5-yl)-2,6,7,8-tetrahydropyrazolo[3,4,5-*de*]quinazoline (100 mg, 0.32 mmol) was dissolved to DMF (4 mL), added with potassium carbonate (88 mg, 0.64 mmol) and 2-(4-(chloromethyl) phenyl)-1-methyl-4-(trifluoromethyl)-1*H*-imidazole (88 mg, 0.32 mmol), and reacted at 20°C for 3 h. After the reaction, 42 mg of the product was given by column chromatography separation (methanol/water = 0-40%) with a yield of 24%.

Molecular formula: $C_{28}H_{22}D_3F_3N_8O$

Molecular weight: 549.58 LC-MS (M/e): 550.2(M+H$^+$)

$^1$H-NMR (400 MHz, DMSO) $\delta$: 8.68(s, 1H), 7.92(s, 1H), 7.76-7.68 (m, 2H), 7.44-7.42 (m, 2H), 5.79(s, 2H), 3.59(s,3H), 3.05-3.00 (m, 4H), 2.45-2.32 (m, 2H), 1.69-1.55 (m, 1H), 1.25-1.19 (m, 2H), 0.92-0.75 (m, 2H).

**Example 11 Preparation of 4-(4-cyclopropyl-6-(methoxy-*d*$_3$)pyrimidin-5-yl)-2-(4-(1-methyl-4-(trifluoromethyl)-1*H*-imidazol-2-yl)benzyl)-6,7,8,9-tetrahydro-2*H*-1,2,3,5-tetraazabenzo[cd]azulene (Compound 11-1)**

**(1) Preparation of 2-(di-tert-butoxycarbonyl)amino-4-(methylthio)-6,7,8,9-tetrahydro-5*H*-cyclohepta[*d*]pyrimidin-5-one**

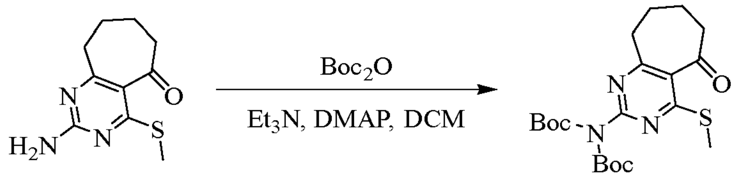


A DCM (60 mL) solution of 2-amino-4-(methylthio)-6,7,8,9-tetrahydro-5*H*-cyclohepta[d]pyrimidin-5-one (2.0 g, 9.0 mmol) was added with triethylamine (4.6 g, 45.5 mmol), p-dimethylaminopyridine (110 mg, 0.9 mmol) and $Boc_2O$ (7.9 g, 36.2 mmol) in turn, and reacted at 25°C for 1 h. LC-MS showed that the reaction was completed, the reaction solution was spin-dried and the residue was separated by column chromatography ($SiO_2$, PE: EA = 10:1 to 2:1) to give 3.4 g of the product with a yield of 89.6%.

**(2) Preparation of tert-butyl(6,7,8,9-tetrahydro-2*H*-1,2,3,5-tetraazabenzo[*cd*]azulene -4-yl)carbamate**

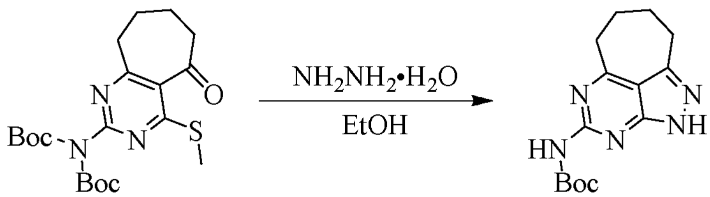


An ethanol (50 mL) solution of 2-(di-tert-butoxycarbonyl)amino-4-(methylthio)-6,7,8,9-tetrahydro-5*H*-cyclohepta[*d*]pyrimidin-5-one (3.2 g, 7.6 mmol) was added with hydrazine hydrate (1.9 g, 37.9 mmol), and reacted at 80°C for 13 h. LC-MS showed that the reaction was completed, the reaction solution was spin-dried and the residue was separated by column chromatography ($SiO_2$, DCM: MeOH = 50: 1 to 10: 1) to give 1.5 g of the product with a yield of 68.6%.

**(3) Preparation of tert-butyl(2-(4-(1-methyl-4-(trifluoromethyl)-1*H*-imidazol-2-yl)benzyl)-6,7,8,9-tetrahydro-2*H*-1,2,3,5-tetraazabenzo[*cd*]azulene-4-yl)carbamate**

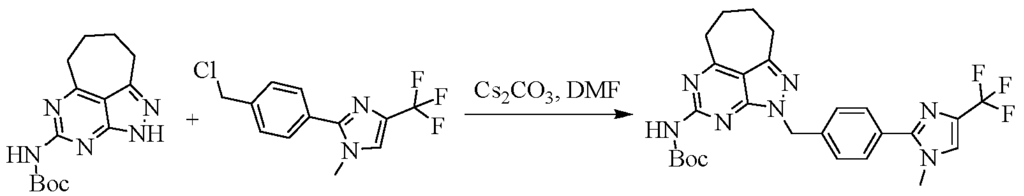


Tert-butyl(6,7,8,9-tetrahydro-2*H*-1,2,3,5-tetraazabenzo[*cd*] azulene-4-yl)carbamate (1.4 g, 4.8 mmol), 2-(4-(chloromethyl)phenyl)-1-methyl-4-(trifluoromethyl)-1*H*-imidazole (1.6 g, 5.8 mmol), cesium carbonate (3.1 g, 9.5 mmol) and DMF (30 mL) were weighed in turn, and reacted at 70°C for 6 h. LC-MS showed that the reaction was completed, the reaction solution was diluted with water, and extracted with ethyl acetate. Organic phases were dried, and spin-dried. The residue was separated by column chromatography ($SiO_2$, PE: EA = 2:1 to 1:1) to give 1.9 g of the product with a yield of 74.4%.

**(4) Preparation of 2-(4-(1-methyl-4-(trifluoromethyl)-1*H*-imidazol-2-yl)benzyl)-6,7,8,9-tetrahydro-2*H*-1,2,3,5-tetraazabenzo[*cd*]azulene-4-amine**

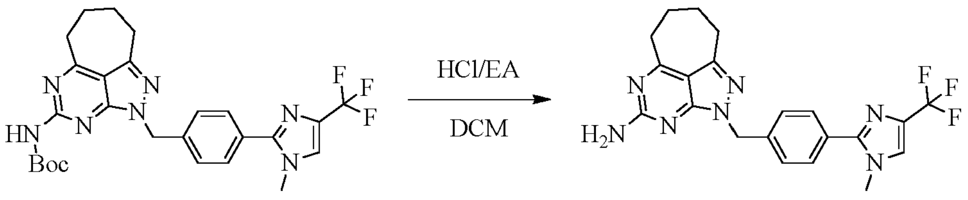


A DCM (10 mL) solution of tert-butyl(2-(4-(1-methyl-4-(trifluoromethyl)-1*H*-imidazol-2-yl)benzyl)-6,7,8,9-tetrahydro-2*H*-1,2,3,5-tetraazabenzo[*cd*]azulene-4-yl)carbamate (1.9 g, 3.6 mmol) was added with HCl/EA (20 mL, 80.0 mmol) and reacted at 25°C for 2 h. LC-MS showed that the reaction was completed, the reaction solution was diluted with water, adjusted with ammonia water till a pH was 9, and then extracted with DCM. Organic phases were dried, and spin-dried. The residue was separated by column chromatography ($SiO_2$, DCM: MeOH = 50: 1 to 30: 1) to give 1.2 g of the product with a yield of 77.9%.

**(5) Preparation of 4-chloro-2-(4-(1-methyl-4-(trifluoromethyl)-1*H*-imidazol-2-yl)benzyl)-6,7,8,9-tetrahydro-2*H*-1,2,3,5-tetraazabenzo[*cd*]azulene**

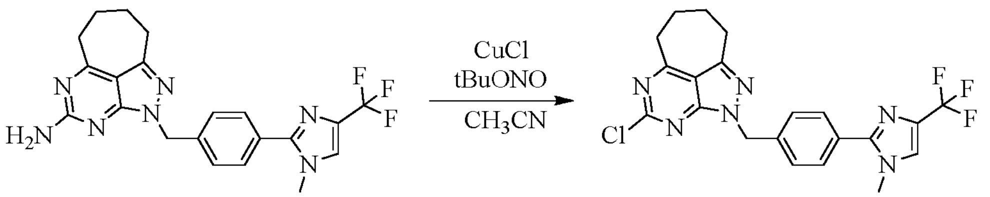


At 0°C, an acetonitrile (3 mL) solution of 2-(4-(1-methyl-4-(trifluoromethyl)-1*H*-imidazol-2-yl)benzyl)-6,7,8,9-tetrahydro-2*H*-1,2,3,5-tetraazabenzo[*cd*]azulene-4-amine (500 mg, 1.2 mmol) was added with cuprous chloride (238 mg, 2.4 mmol) and tert-butyl nitrite (247 mg, 2.4 mmol), and reacted at 60°C for 4 h. LC-MS showed that the reaction was completed, the reaction solution was filtered, and the filtrate was spin-dried. The residue was separated by column chromatography ($SiO_2$, DCM: MeOH = 50:1 to 30:1) to give 100 mg of the product with a yield of 19.1%.

**(6) Preparation of 4-(4-cyclopropyl-6-(methoxy-*d*$_3$)pyrimidin-5-yl)-2-(4-(1-methyl-4-(trifluoromethyl)-1*H*-imidazol-2-yl)**benzyl**)-6,7,8,9-tetrahydro-2*H*-1,2,3,5-tetraazabenzo[*cd*]azulene**

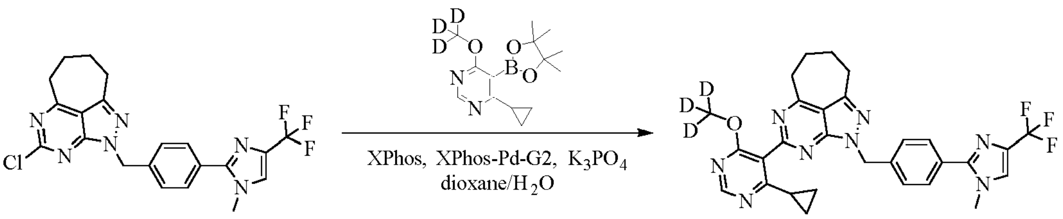


4-chloro-2-(4-(1-methyl-4-(trifluoromethyl)-1*H*-imidazol-2-yl)benzyl)-6,7,8,9-tetrahydro-2*H*-1,2,3,5-tetraazabenzo[*cd*]azulene (100 mg, 0.22 mmol), 4-cyclopropyl-6-(methoxy-*d*$_3$)-5-(4,4,5,5-tetramethyl-1,3,2-dioxobenzaldehyde-2-yl)pyrimidine (50 mg of crude product), XPhos (10 mg, 0.021 mmol), XPhos-Pd-G2 (17 mg, 0.022 mmol), potassium phosphate (140 mg, 0.66 mmol), dioxane (5 mL) and water (1 mL) were weighed in turn, subjected to nitrogen replacement for 3 times, and reacted at 90°C for 6 h. LC-MS showed that the reaction was completed, the reaction solution was filtered, and the filtrate was spin-dried. The residue was separated by thin layer chromatography ($SiO_2$, DCM: MeOH = 30:1, Rf-0.4) to give 16 mg of the product with a yield of 12.7%.

Molecular formula: $C_{29}H_{24}D_3F_3N_8O$

Molecular weight: 563.6 LC-MS (m/z): 564.2 ($M+H^+$)

$^1$H-NMR(400 MHz, $CDCl_3$) $\delta$: 8.68 (s, 1H), 7.59 (d, 2H, *J*=8.0), 7.53 (d, 2H, *J*=8.0), 7.31 (s, 1H), 5.69 (s, 2H), 3.75

(s, 3H), 3.43-3.33 (m, 2H), 3.24-3.14 (m, 2H), 2.33-2.12 (m, 5H), 1.20-0.90 (m, 4H).

**Example 12 Preparation of 4-(4-cyclopropyl-6-(methoxy-*d*$_3$)pyrimidin-5-yl)-2-(4-(1-isopropyl-4-(trifluoromethyl)-1*H*-imidazol-2-yl)benzyl)-2,6,7,8-tetrahydropyrazolo[3,4,5-*de*]quinazoline (Compound 16-1)**

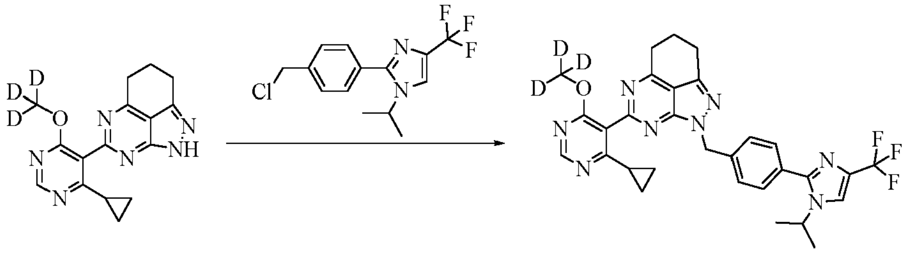

4-(4-cyclopropyl-6-(methoxy-*d*$_3$)pyrimidin-5-yl)-2,6,7,8-tetrahydropyrazolo[3,4,5-*de*]quinazoline (93 mg, 0.3 mmol) was dissolved to DMF (5 mL), added with potassium carbonate (83 mg, 0.6 mmol) and 2-(4-(chloromethyl) phenyl)-1-isopropyl-4-(trifluoromethyl)-1*H*-imidazole (100 mg, 0.3 mmol), and reacted at 70°C for 2 h. After the reaction, the reaction mixture was subjected to column chromatography separation (PE/EA = 3:1 to 1:1) to give 43 mg of the product with a yield of 24.2%.

Molecular formula: $C_{30}H_{26}D_3F_3N_8O$

Molecular weight: 577.6 LC-MS (M/e): 578.3(M+H$^+$)

$^1$H-NMR (400 MHz, DMSO) $\delta$: 8.69(s, 1H), 8.16(s, 1H), 7.55-7.49 (d, 2H), 7.44-7.39 (d, 2H), 5.65(s, 2H), 4.45-4.35(m,1H), 3.09-3.00 (m, 4H), 2.30-2.22 (m, 2H), 1.65-1.55 (m, 1H), 1.38-1.32 (m, 6H), 1.09-1.01 (m, 2H), 0.86-0.80 (m, 2H).

**Example 13 Preparation of 4-(4-cyclopropyl-6-(methoxy-*d*$_3$)pyrimidin-5-yl)-2-(4-(1-isopropyl-4-(trifluoromethyl)-1*H*-imidazol-2-yl)benzyl)-6,7,8,9-tetrahydro-2*H*-1,2,3,5-tetraazabenzo[*cd*]azulene(Compound 17-1)**

**(1) Preparation** of **2-(4-cyclopropyl-6-(methoxy-*d*$_3$)pyrimidin-5-yl)-4-(methylthio)-6,7,8,9-tetrahydro-5*H*-cyclohepta[*d*]pyrimidin-5-one**

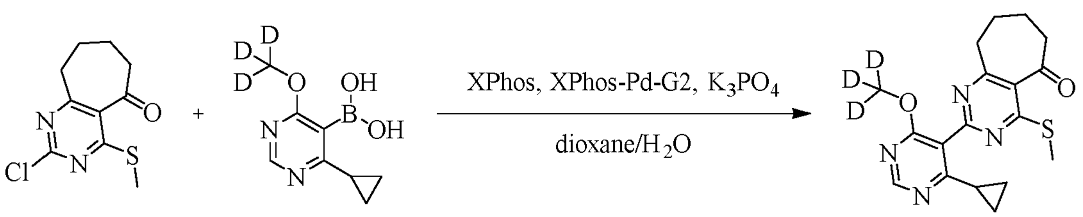


2-chloro-4-(methylthio)-6,7,8,9-tetrahydro-5*H*-cyclohepta[d]pyrimidin-5-one (400 mg, 1.6 mmol), (4-cyclopropyl-6-(methoxy-*d*$_3$)pyrimidin-5-yl)boronic acid (347 mg, 1.8 mmol), XPhos (76 mg, 0.16 mmol), XPhos-Pd-G2 (126 mg, 0.16 mmol), potassium phosphate (1.0 g, 4.7 mmol), dioxane (20 mL) and water (4 mL) were weighed in turn, subjected to nitrogen replacement for three times, and reacted at 90°C for 4 h. LC-MS showed that the reaction was completed, the reaction solution was filtered, and the filtrate was spin-dried. The residue was separated by column chromatography ($SiO_2$, PE: EA = 5:1 to 3:1) to give 260 mg of the product with a yield of 43.9%.

**(2) Preparation of 4-(4-cyclopropyl-6-(methoxy-*d*$_3$)pyrimidin-5-yl)-6,7,8,9-tetrahydro-2*H*-1,2,3,5-tetraazabenzo [*cd*]azulene**

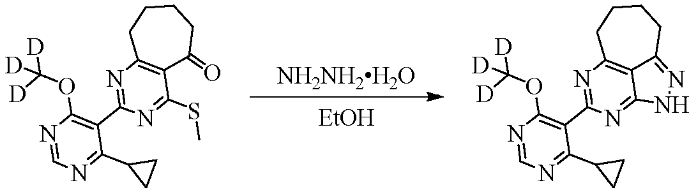


An ethanol (6 mL) solution of 2-(4-cyclopropyl-6-(methoxy-*d*$_3$)pyrimidin-5-yl)-4-(methylthio)-6,7,8,9-tetrahydro-5*H*-cyclohepta[d]pyrimidin-5-one (240 mg, 0.67 mmol) was added with hydrazine hydrate (168 mg, 3.4 mmol), and reacted at 80°C for 3 h. LC-MS showed that the reaction was completed, the reaction solution was spin-dried and the residue was separated by column chromatography ($SiO_2$, PE: EA = 3:1 to 1:1) to give 200 mg of the product with a yield of 92.0%.

**(3) Preparation of 4-(4-cyclopropyl-6-(methoxy-*d*$_3$)pyrimidin-5-yl)-2-(4-(1-isopropyl-4-(trifluoromethyl)-1*H*-imidazol-2-yl)benzyl)-6,7,8,9-tetrahydro-2*H*-1,2,3,5-tetraazabenzo[*cd*]azulene**

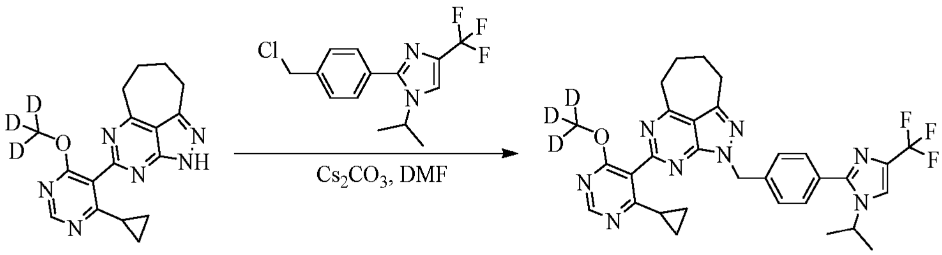


A DMF (5 mL) solution of 4-(4-cyclopropyl-6-(methoxy-*d*$_3$)pyrimidin-5-yl)-6,7,8,9-tetrahydro-2*H*-1,2,3,5-tetraazabenzo[*cd*]chamomile (200 mg, 0.61 mmol) was added with 2-(4-(chloromethyl)phenyl)-1-isopropyl-4-(trifluoromethyl)-1*H*-imidazole (203 mg, 0.67 mmol) and cesium carbonate (596 mg, 1.8 mmol), and reacted at 50°C for 13 h. LC-MS showed that the reaction was completed, the reaction solution was diluted with water, and extracted with EA. Organic phases were dried, and spin-dried. The residue was separated by column chromatography ($SiO_2$, PE: EA = 3:1 to 1:1) to give 167 mg of the product with a yield of 45.9%.

Molecular formula: $C_{31}H_{28}D_3F_3N_8O$

Molecular weight: 591.7 LC-MS (m/z): 592.3 ($M+H^+$)

$^1$H-NMR(400 MHz, $CDCl_3$) $\delta$: 8.67 (s, 1H), 7.52-7.47 (m, 4H), 7.41 (s, 1H), 5.69 (s, 2H), 4.56-4.49 (m, 1H), 3.37-3.34 (m, 2H), 3.19-3.16 (m, 2H), 2.23-2.12 (m, 4H), 1.70-1.62 (m, 1H), 1.45-1.41 (m, 6H), 1.35-1.23 (m, 2H), 0.91-0.86 (m, 2H).

The compounds shown in the following table were prepared by the same or similar methods as in the above examples:

| No. | LC-MS (M/e, M+H$^+$) | No. | LC-MS (M/e, M+H$^+$) |
|---|---|---|---|
| Compound 4 | 517.2 | Compound 5 | 547.2 |
| Compound 6 | 517.2 | Compound 7 | 533.2 |
| Compound 8 | 533.2 | Compound 9 | 536.2 |
| Compound 12 | 517.2 | Compound 13 | 534.2 |
| Compound 14 | 585.2 | Compound 15 | 597.2 |
| Compound 17 | 590.3 | Compound 5-1 | 550.2 |
| Compound 7-1 | 536.2 | Compound 8-1 | 536.2 |

## Claims

**1.** A compound of general formula (II-2) or a pharmaceutically acceptable salt, deuterated compound or stereoisomer thereof,

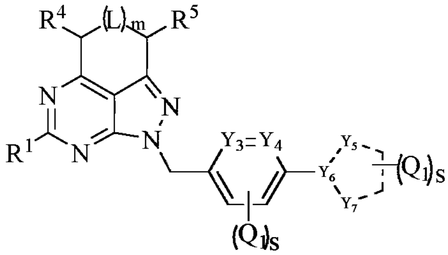

formula (II-2)

**characterized in that**:

$R^1$ is selected from phenyl or 5-6 membered heteroaryl optionally substituted by 1-4 $Q_1$;
$R^4$ and $R^5$ are each independently selected from deuterium, hydrogen, carboxyl, cyano, nitro, amino, halogen, $C_{2-6}$ alkenyl, $C_{2-6}$ alkynyl, or from $C_{1-6}$ alkyl, $C_{1-6}$ alkoxy, $C_{1-6}$ alkylamino, di($C_{1-6}$ alkyl)amino, halogenated $C_{1-6}$ alkyl, hydroxy $C_{1-6}$ alkyl, amino $C_{1-6}$ alkyl, carboxyl $C_{1-6}$ alkyl or halogenated $C_{1-6}$ alkoxy, each of which is optionally deuterated;
each $Q_1$ is independently selected from deuterium, halogen, cyano, carboxyl, hydroxyl, amino, nitro, sulfonamido, or from $C_{1-6}$ alkylamino, di($C_{1-6}$ alkyl)amino, halogenated $C_{1-6}$ alkyl, halogenated $C_{1-6}$ alkoxy, hydroxy $C_{1-6}$ alkyl, amino $C_{1-6}$ alkyl, carboxyl $C_{1-6}$ alkyl, $C_{1-6}$ alkylcarbonyl, $C_{1-6}$ alkoxycarbonyl, $C_{1-6}$ alkylaminoacyl, $C_{1-6}$ alkylamido, $C_{1-6}$ alkylsulfonyl,$C_{1-6}$ alkylsulfonamido, $C_{1-6}$ alkylaminosulfonyl, $-(L)_m-C_{1-6}$ alkyl, $-(L)_m-C_{2-6}$ alkenyl, $-(L)_m-C_{2-6}$ alkynyl, $-(L)_m-C_{1-6}$ alkoxy, $-(L)_m$-6-10 membered aryl, $-(L)_m$-5-12 membered heteroaryl, $-(L)_m$-3-8 membered cycloalkyl or $-(L)_m$-3-8 membered heterocyclyl, each of which is optionally substituted by 1-4 substituents $Q_2$; and each $Q_2$ is independently selected from deuterium, halogen, carboxyl, hydroxyl, cyano, nitro, amino, $C_{1-6}$ alkyl, hydroxy $C_{1-6}$ alkyl, carboxyl $C_{1-6}$ alkyl, $C_{1-6}$ alkylamino, di($C_{1-6}$ alkyl)amino, $-CO-C_{1-6}$ alkylene-$NH_2$, $-CO-C_{1-6}$ alkyl, $C_{1-6}$ alkoxy, halogenated $C_{1-6}$ alkyl and halogenated $C_{1-6}$ alkoxy;
each L is independently selected from $-CR^aR^b-$;
each $R^a$ and each $R^b$ are each independently selected from deuterium, hydrogen, or from $C_{1-6}$ alkyl, $C_{1-6}$ alkoxy, each of which is optionally deuterated;
$Y_3$, $Y_4$, and $Y_6$ are each independently selected from N, C or $CR^a$;
$Y_5$ and $Y_7$ are each independently selected from N, $NR^c$, C, $CR^aR^b$ or $CR^a$;
each $R^c$ is independently selected from deuterium, hydrogen, or from $C_{1-6}$ alkyl, halogenated $C_{1-6}$ alkyl, halogenated $C_{1-6}$ alkoxy, hydroxy $C_{1-6}$ alkyl, amino $C_{1-6}$ alkyl and carboxyl $C_{1-6}$ alkyl, each of which is optionally deuterated;
each m is independently an integer of 0-3;
-- and - is each independently selected from a single bond or a double bond, and two adjacent bonds are incapable of being double bonds at the same time; and
each s is independently selected from an integer of 0-2.

**2.** The compound or the pharmaceutically acceptable salt, deuterated compound or stereoisomer thereof according to claim 1,
**characterized in that**:

$R^1$ is selected from phenyl or 5-6 membered nitrogen-containing heteroaryl, each of which is optionally substituted by 1-3 $Q_1$;
$R^4$ and $R^5$ are each independently selected from deuterium, hydrogen, cyano, halogen, or from $C_{1-6}$ alkyl, $C_{1-6}$ alkoxy, $C_{1-6}$ alkylamino, di($C_{1-6}$ alkyl)amino, halogenated $C_{1-6}$ alkyl, hydroxy $C_{1-6}$ alkyl, amino $C_{1-6}$ alkyl, carboxyl $C_{1-6}$ alkyl or halogenated $C_{1-6}$ alkoxy, each of which is optionally deuterated;
each $Q_1$ is independently selected from deuterium, hydrogen, or from $C_{1-6}$ alkyl, $C_{1-6}$ alkoxy, halogenated $C_{1-6}$ alkyl, halogenated $C_{1-6}$ alkoxy, and 3-6 membered cycloalkyl, each of which is optionally substituted by 1-3 substituents $Q_2$; and each $Q_2$ is independently selected from deuterium, $C_{1-6}$ alkyl, $C_{1-6}$ alkoxy, halogenated $C_{1-6}$ alkyl and halogenated $C_{1-6}$ alkoxy;
each L is independently selected from $-CR^aR^b-$;

each $R^a$ and each $R^b$ are each independently selected from deuterium, hydrogen, or from methyl, ethyl, propyl, isopropyl, butyl, isobutyl, sec-butyl, tert-butyl, methoxy, ethoxy, propoxy or isopropoxy, each of which is optionally deuterated;

$Y_3$ and $Y_4$ are each independently selected from N, C or $CR^a$;

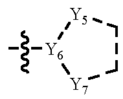

is selected from the following structures:

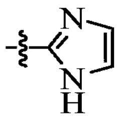 , 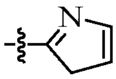 , 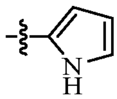 , 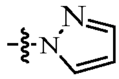 , 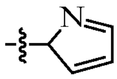 ,

and

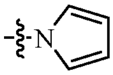 ;

each $R^c$ is independently selected from deuterium, hydrogen, or from $C_{1-6}$ alkyl, halogenated $C_{1-6}$ alkyl and halogenated $C_{1-6}$ alkoxy, each of which is optionally deuterated;

each m is independently an integer of 0-2;

-- and - is each independently selected from a single bond or a double bond, and two adjacent bonds are incapable of being double bonds at the same time; and

each s is independently selected from an integer of 0-2.

**3.** The compound or the pharmaceutically acceptable salt, deuterated compound or stereoisomer thereof according to claim 1,

**characterized in that**:

$R^1$ is selected from phenyl, furyl, thienyl, pyrrolyl, thiazolyl, isothiazolyl, thiadiazolyl, oxazolyl, isoxazolyl, oxadiazole, imidazolyl, pyrazolyl, 1,2,3-triazolyl, 1,2,4-triazolyl, pyridyl, 2-pyridone, 4-pyridone, pyrimidinyl, pyridazinyl, pyrazinyl, 1,2,3-triazolyl, 1,3,5-triazinyl and 1,2,4,5-tetrazinyl, each of which is optionally substituted by 1-3 $Q_1$;

$R^4$ and $R^5$ are each independently selected from deuterium, hydrogen, or from methyl, ethyl, propyl, isopropyl, butyl, isobutyl, sec-butyl, tert-butyl, methoxy, ethoxy, propoxy, isopropoxy, methylamino, dimethylamino, monofluoromethyl, difluoromethyl, trifluoromethyl, hydroxymethyl, hydroxyethyl, hydroxypropyl, hydroxybutyl, aminomethyl, carboxymethyl, carboxyethyl, monofluoromethoxy, difluoromethoxy and trifluoromethoxy, each of which is optionally deuterated;

each $Q_1$ is independently selected from deuterium, fluorine, chlorine, bromine, iodine, or from methoxy, ethoxy, propoxy, isopropoxy, methylamino, dimethylamino, monofluoromethyl, difluoromethyl, trifluoromethyl, monofluoromethoxy, difluoromethoxy, trifluoromethoxy, hydroxymethyl, hydroxyethyl, hydroxypropyl, hydroxybutyl, aminomethyl, carboxymethyl, carboxyethyl, $-(L)_m-C_{1-4}$ alkyl, or $-(L)_m$-3-6 membered cycloalkyl, each of which is optionally substituted by 1-3 substituents $Q_2$, and each $Q_2$ is independently selected from deuterium, halogen, carboxyl, hydroxyl, cyano, nitro, amino, methyl, ethyl, propyl, isopropyl, butyl, isobutyl, sec-butyl, tert-butyl, methoxy, ethoxy, propoxy, isopropoxy, hydroxymethyl, hydroxyethyl, hydroxypropyl, hydroxybutyl, carboxymethyl, carboxyethyl, methylamino, dimethylamino, monofluoromethyl, difluoromethyl, trifluoromethyl, monofluoromethoxy, difluoromethoxy and trifluoromethoxy;

each L is independently selected from $-CR^aR^b-$;

each $R^a$ and each $R^b$ are each independently selected from deuterium, hydrogen;

$Y_3$, $Y_4$, and $Y_6$ are each independently selected from N, C or $CR^a$;

$Y_5$ and $Y_7$ are each independently selected from N, $NR^c$, C, $CR^aR^b$ or $CR^a$;

each $R^c$ is independently selected from deuterium, hydrogen, or from $C_{1-4}$ alkyl, halogenated $C_{1-4}$ alkyl and halogenated $C_{1-4}$ alkoxy, each of which is optionally deuterated;

each m is independently 0, 1 or 2;

-- and - is each independently selected from a single bond or a double bond, and two adjacent bonds are incapable of being double bonds at the same time; and
each s is independently 0, 1 or 2.

**4.** The compound or the pharmaceutically acceptable salt, deuterated compound or stereoisomer thereof according to any one of claims 1-3,

**characterized in that**:
$R^1$ is selected from

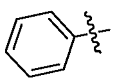, 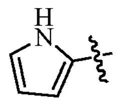, 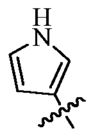, 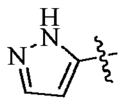, 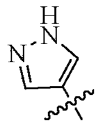, 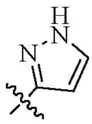, 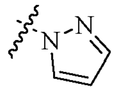, 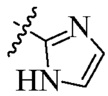,

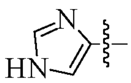 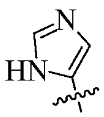 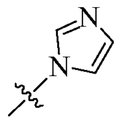 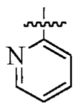 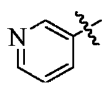 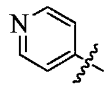 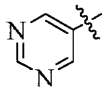 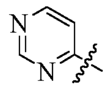 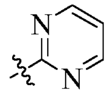 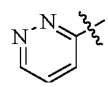

or

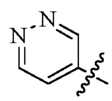

optionally substituted by 1-3 Q1.

**5.** The compound or the pharmaceutically acceptable salt, deuterated compound or stereoisomer thereof according to claim 1 or 2, **characterized by** comprising a structure of formula (II-5),

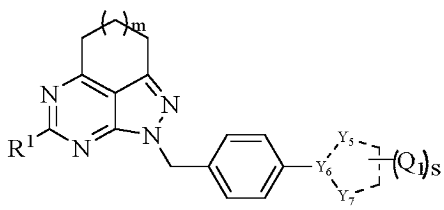

formula (II-5)

**characterized in that**:

$R^1$ is selected from phenyl or 5-6 membered nitrogen-containing heteroaryl optionally substituted by 1-3 $Q_1$; each $Q_1$ is independently selected from deuterium, hydrogen, or from $C_{1-6}$ alkyl, $C_{1-6}$ alkoxy, halogenated $C_{1-6}$ alkyl, halogenated $C_{1-6}$ alkoxy, and 3-6 membered cycloalkyl, each of which is optionally substituted by 1-3 substituents $Q_2$; and each $Q_2$ is independently selected from deuterium, $C_{1-6}$ alkyl, $C_{1-6}$ alkoxy, halogenated $C_{1-6}$ alkyl and halogenated $C_{1-6}$ alkoxy;

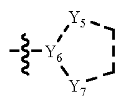

is selected from the following structures:

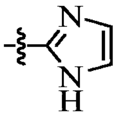, 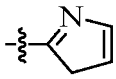, 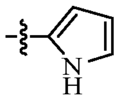, 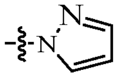, 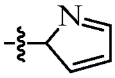,

and

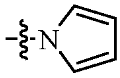;

m is an integer of 1 or 2; and
each s is independently an integer of 0, 1 or 2.

**6.** The compound or the pharmaceutically acceptable salt, deuterated compound or stereoisomer thereof according to claim 1, **characterized by** comprising a structure of formula (III-1'),

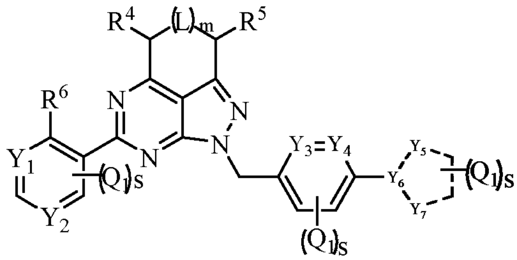

formula (III-1')

**characterized in that**:

$R^6$ is selected from hydrogen, or $C_{1-6}$ alkyl, $C_{1-6}$ alkoxy, halogenated $C_{1-6}$ alkyl and halogenated $C_{1-6}$ alkoxy, each of which is optionally deuterated;
$Y_1$, $Y_2$, $Y_3$, $Y_4$ and $Y_6$ are each independently selected from N, C or $CR^a$;
$Y_5$ and $Y_7$ are each independently selected from N, $NR^c$, C, $CR^aR^b$ or $CR^a$;
--- is each independently selected from a single bond or a double bond, and two adjacent bonds are incapable of being double bonds at the same time;
each s is independently selected from an integer of 0-2.

**7.** The compound or the pharmaceutically acceptable salt, deuterated compound or stereoisomer thereof according to claim 1, **characterized by** comprising a structure of formula (IV-1'),

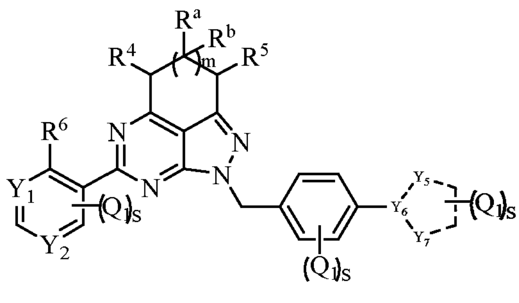

formula (IV-1')

wherein, $R^6$ is selected from hydrogen, or optionally deuterated $C_{1-6}$ alkyl, $C_{1-6}$ alkoxy, halogenated $C_{1-6}$ alkyl and halogenated $C_{1-6}$ alkoxy,
$Y_1$ and $Y_2$ are each independently selected from N, C or $CR^a$;
$Y_6$ is selected from N, C or $CR^a$;
$Y_5$ and $Y_7$ are each independently selected from N, $NR^c$, $CR^aR^b$ or $CR^a$;
-- and - is each independently selected from a single bond or a double bond, and two adjacent bonds are incapable of being double bonds at the same time; and

each s is independently selected from an integer of 0-2.

**8.** The compound or the pharmaceutically acceptable salt, deuterated compound or stereoisomer thereof according to claim 7, **characterized in that**: $R^6$ is selected from deuterated methoxy, deuterated ethoxy, deuterated propoxy or deuterated isopropoxy, and a number of deuteration is 1, 2 or 3.

**9.** The compound or the pharmaceutically acceptable salt, deuterated compound or stereoisomer thereof according to any one of claims 1-8, **characterized by** comprising the following structure:

| No. | Structure | No. | Structure |
|---|---|---|---|
| Compound 1 | 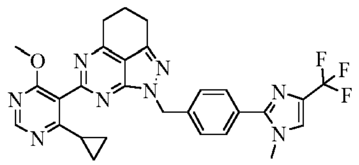 | Compound 2 | 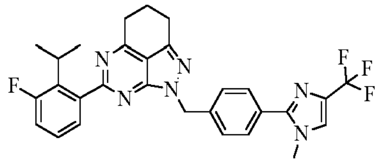 |
| Compound 3 | 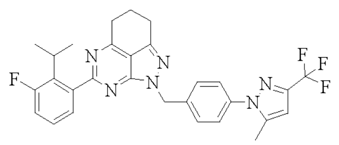 | Compound 4 | 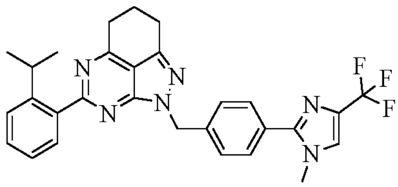 |
| Compound 5 | 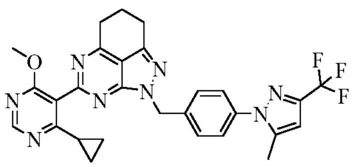 | Compound 6 | 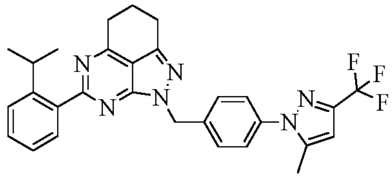 |
| Compound 7 | 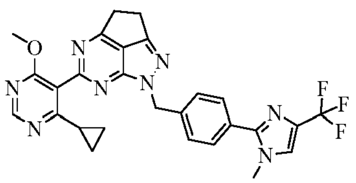 | Compound 8 | 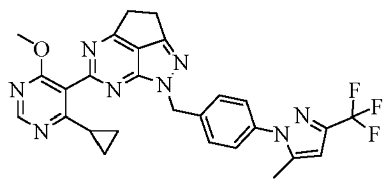 |
| Compound 9 | 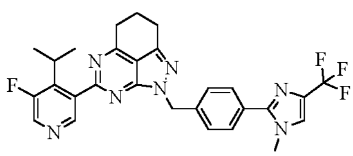 | Compound 10 | 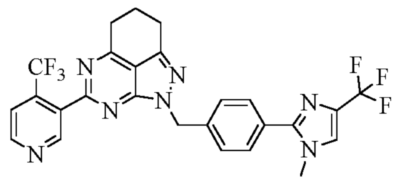 |
| Compound 11 | 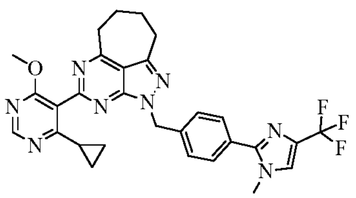 | Compound 12 | 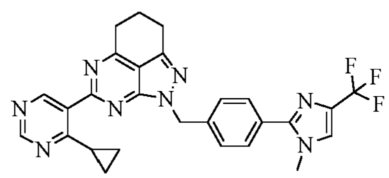 |
| Compound 13 | 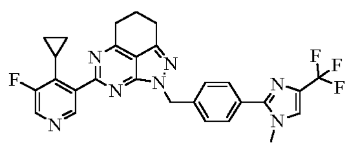 | Compound 14 | 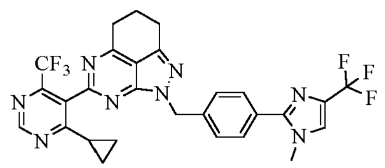 |

(continued)

| No. | Structure | No. | Structure |
|---|---|---|---|
| Compound 15 | 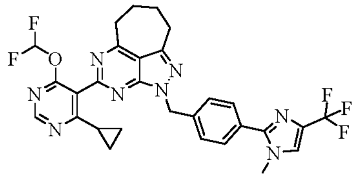 | Compound 16 | 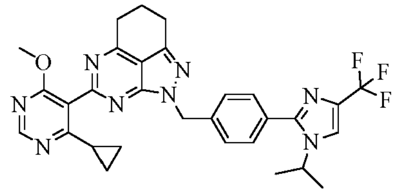 |
| Compound 17 | 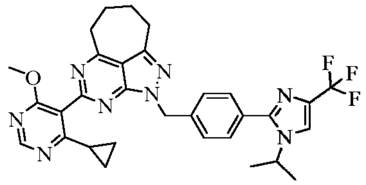 | Compound 18 | 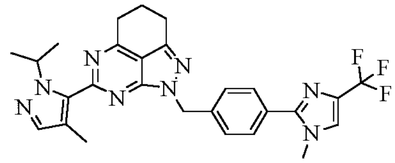 |
| Compound 19 | 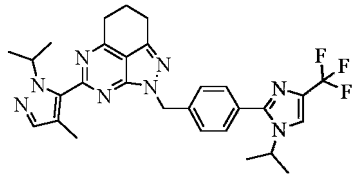 | Compound 20 | 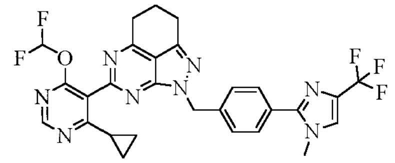 |
| Compound 1-1 | 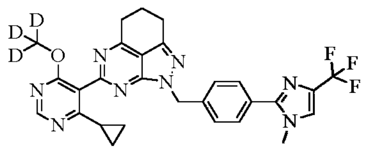 | Compound 5-1 | 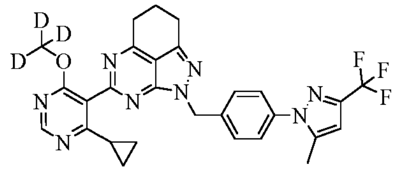 |
| Compound 7-1 | 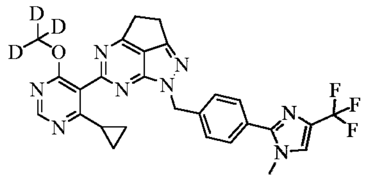 | Compound 8-1 | 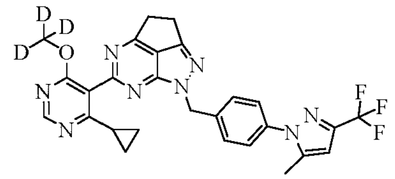 |
| Compound 11-1 | 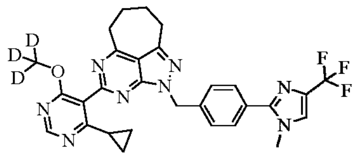 | Compound 16-1 | 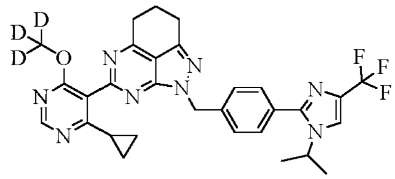 |
| Compound 17-1 | 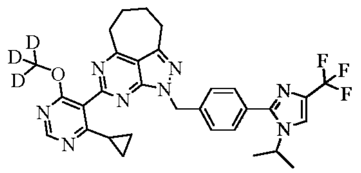 | | |

**10.** A pharmaceutical formulation, comprising the compound or the pharmaceutically acceptable salt, deuterated compound or stereoisomer thereof according to any one of claims 1-9, and one or more pharmaceutically acceptable carriers and/or diluents, **characterized in that** the pharmaceutical formulation is any dosage form that is clinically or pharmaceutically acceptable.

**11.** A pharmaceutical composition, **characterized by** comprising the compound or the pharmaceutically acceptable salt, deuterated compound or stereoisomer thereof according to any one of claims 1-9, or the pharmaceutical formulation according to claim 10, and one or more second therapeutic active agents.

**12.** The compound or the pharmaceutically acceptable salt, deuterated compound or stereoisomer thereof according to any one of claims 1-9, or the pharmaceutical formulation according to claim 10, or the pharmaceutical composition according to claim 11 for use in treating and/or preventing a disease mediated by USP1 and a disease related thereto, the disease mediated by USP1 and the disease related thereto are selected from cancer or benign tumor.

**13.** A method for preparing the compound or the pharmaceutically acceptable salt, deuterated compound or stereoisomer thereof according to any one of claims 1-9, **characterized by** comprising the following steps of:

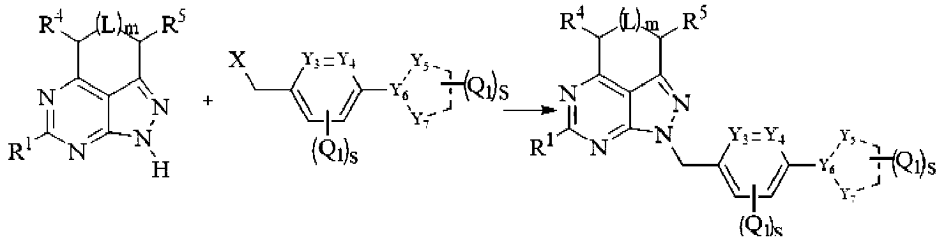

formula (II-2)-1 formula (II-2)-2 formula (II-2)

reacting the compound of formula (II-2)-1 with the compound of formula (II-2)-2 to obtain the compound of formula (II-2),
X is halogen.

**14.** An intermediate for preparing the compound or the pharmaceutically acceptable salt, deuterated compound or stereoisomer thereof according to any one of claims 1-9, **characterized by** comprising the following structure:

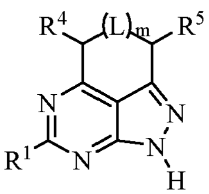 formula (II-2)-1, 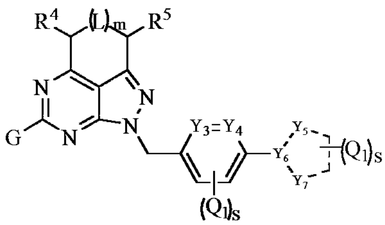 formula (II-2)-3,

wherein, G is selected from hydrogen, hydroxyl, amino, $C_{1-6}$ alkylthio or $C_{1-6}$ alkylsulfonyl.

## Patentansprüche

**1.** Eine Verbindung der allgemeinen Formel (II-2) oder ein pharmazeutisch annehmbares Salz, eine deuterierte Verbindung oder ein Stereoisomer davon,

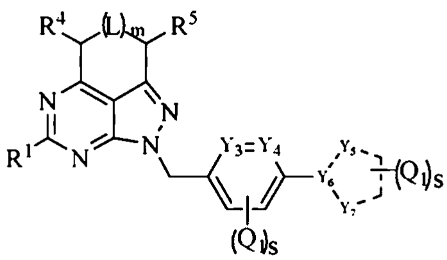

Formel (II-2),

**dadurch gekennzeichnet, dass**:

$R^1$ aus Phenyl oder 5-6-gliedrigem Heteroaryl ausgewählt ist, optional substituiert mit 1-4 $Q_1$;
$R^4$ und $R^5$ jeweils unabhängig aus Deuterium, Wasserstoff, Carboxyl, Cyan, Nitro, Amino, Halogen, $C_{2-6}$-Alkenyl, $C_{2-6}$-Alkinyl oder aus $C_{1-6}$-Alkyl, $C_{1-6}$-Alkoxy, $C_{1-6}$-Alkylamino, Di($C_{1-6}$-alkyl)amino, halogeniertem $C_{1-6}$-Alkyl,

Hydroxy-$C_{1-6}$-alkyl, Amino-$C_{1-6}$-alkyl, Carboxyl-$C_{1-6}$-alkyl oder halogeniertem $C_{1-6}$-Alkoxy ausgewählt sind, von denen jedes optional deuteriert ist;

jedes $Q_1$ unabhängig aus Deuterium, Halogen, Cyan, Carboxyl, Hydroxyl, Amino, Nitro, Sulfonamido oder aus $C_{1-6}$-Alkylamino, Di($C_{1-6}$-alkyl)amino, halogeniertem $C_{1-6}$-Alkyl, halogeniertem $C_{1-6}$-Alkoxy, Hydroxy-$C_{1-6}$-alkyl, Amino-$C_{4-6}$-alkyl, Carboxyl-$C_{1-6}$-alkyl, $C_{1-6}$-Alkylcarbonyl, $C_{1-6}$-Alkoxycarbonyl, $C_{1-6}$-Alkylaminoacyl, $C_{1-6}$-Alkylamido, $C_{1-6}$-Alkylsulfonyl, $C_{1-6}$-Alkylsulfonamido, $C_{1-6}$-Alkylaminosulfonyl, $-(L)_m-C_{1-6}$-Alkyl, $-(L)_m-C_{2-6}$-Alkenyl, $-(L)_m-C_{2-6}$-Alkinyl, $-(L)_m-C_{1-6}$-Alkoxy, $-(L)_m$-6-10-gliedrigem Aryl, $-(L)_m$-5-12-gliedrigem Heteroaryl, $-(L)_m$-3-8-gliedrigem Cycloalkyl oder $-(L)_m$-3-8-gliedrigem Heterocyclyl ausgewählt ist, von denen jedes optional mit 1-4 Substituenten $Q_2$ substituiert ist; und jedes $Q_2$ unabhängig aus Deuterium, Halogen, Carboxyl, Hydroxyl, Cyan, Nitro, Amino, $C_{1-6}$-Alkyl, Hydroxy-$C_{1-6}$-alkyl, Carboxyl-$C_{1-6}$-alkyl, $C_{1-6}$-Alkylamino, Di($C_{1-6}$-alkyl)amino, -CO-$C_{1-6}$-Alkylen-$NH_2$, -CO-$C_{1-6}$-Alkyl, $C_{1-6}$-Alkoxy, halogeniertem $C_{1-6}$-Alkyl und halogeniertem $C_{1-6}$-Alkoxy ausgewählt ist;

jeder L unabhängig aus $-CR^aR^b-$ ausgewählt ist;

jeder $R^a$ und jeder $R^b$ jeweils unabhängig aus Deuterium, Wasserstoff oder aus $C_{1-6}$-Alkyl, $C_{1-6}$-Alkoxy ausgewählt ist, von denen jedes optional deuteriert ist;

$Y_3$, $Y_4$ und $Y_6$ jeweils unabhängig aus N, C oder $CR^a$ ausgewählt sind;

$Y_5$ und $Y_7$ jeweils unabhängig aus N, $NR^c$, C, $CR^aR^b$ oder $CR^a$ ausgewählt sind;

jeder $R^c$ unabhängig aus Deuterium, Wasserstoff oder aus $C_{1-6}$-Alkyl, halogeniertem $C_{1-6}$-Alkyl, halogeniertem $C_{1-6}$-Alkoxy, Hydroxy-$C_{1-6}$-alkyl, Amino-$C_{1-6}$-alkyl und Carboxyl-$C_{1-6}$-alkyl ausgewählt ist, von denen jedes optional deuteriert ist;

jedes m unabhängig eine ganze Zahl von 0-3 ist;

-- und - jeweils unabhängig aus einer Einfachbindung oder einer Doppelbindung ausgewählt sind und zwei benachbarte Bindungen nicht gleichzeitig Doppelbindungen sein können; und

jedes s unabhängig aus einer ganzen Zahl von 0-2 ausgewählt ist.

**2.** Verbindung oder pharmazeutisch annehmbares Salz, deuterierte Verbindung oder Stereoisomer davon gemäß Anspruch 1,
**dadurch gekennzeichnet, dass**:

$R^1$ aus Phenyl oder 5-6-gliedrigem, Stickstoff enthaltendem Heteroaryl ausgewählt ist, von denen jedes optional mit 1-3 $Q_1$ substituiert ist;

$R^4$ und $R^5$ jeweils unabhängig aus Deuterium, Wasserstoff, Cyan, Halogen oder aus $C_{1-6}$-Alkyl, $C_{1-6}$-Alkoxy, $C_{1-6}$-Alkylamino, Di($C_{1-6}$-alkyl)amino, halogeniertem $C_{1-6}$-Alkyl, Hydroxy-$C_{1-6}$-alkyl, Amino-$C_{1-6}$-alkyl, Carboxyl-$C_{1-6}$-alkyl oder halogeniertem $C_{1-6}$-Alkoxy ausgewählt sind, von denen jedes optional deuteriert ist;

jedes $Q_1$ unabhängig aus Deuterium, Wasserstoff oder aus $C_{1-6}$-Alkyl, $C_{1-6}$-Alkoxy, halogeniertem $C_{1-6}$-Alkyl, halogeniertem $C_{1-6}$-Alkoxy und 3-6-gliedrigem Cycloalkyl ausgewählt ist, von denen jedes optional mit 1-3 Substituenten $Q_2$ substituiert ist; und

jedes $Q_2$ unabhängig aus Deuterium, $C_{1-6}$-Alkyl, $C_{1-6}$-Alkoxy, halogeniertem $C_{1-6}$-Alkyl und halogeniertem $C_{1-6}$-Alkoxy ausgewählt ist;

jeder L unabhängig aus $-CR^aR^b-$ ausgewählt ist;

jeder $R^a$ und jeder $R^b$ jeweils unabhängig aus Deuterium, Wasserstoff oder aus Methyl, Ethyl, Propyl, Isopropyl, Butyl, Isobutyl, sec-Butyl, tert-Butyl, Methoxy, Ethoxy, Propoxy oder Isopropoxy ausgewählt ist, von denen jedes optional deuteriert ist;

$Y_3$ und $Y_4$ jeweils unabhängig aus N, C oder $CR^a$ ausgewählt sind;

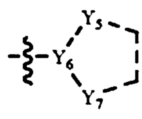;

aus den folgenden Strukturen ausgewählt ist:

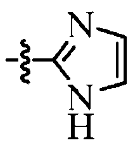, 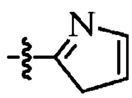,

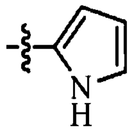, 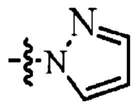, 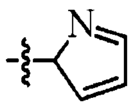 und 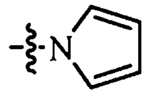;

jeder $R^c$ unabhängig aus Deuterium, Wasserstoff oder aus $C_{1-6}$-Alkyl, halogeniertem $C_{1-6}$-Alkyl und halogeniertem $C_{1-6}$-Alkoxy ausgewählt ist, von denen jedes optional deuteriert ist;
jedes m unabhängig eine ganze Zahl von 0-2 ist;
-- und - jeweils unabhängig aus einer Einfachbindung oder einer Doppelbindung ausgewählt sind und zwei benachbarte Bindungen nicht gleichzeitig Doppelbindungen sein können; und
jedes s unabhängig aus einer ganzen Zahl von 0-2 ausgewählt ist.

3. Verbindung oder pharmazeutisch annehmbares Salz, deuterierte Verbindung oder Stereoisomer davon gemäß Anspruch 1,
**dadurch gekennzeichnet, dass**:

$R^1$ aus Phenyl, Furyl, Thienyl, Pyrrolyl, Thiazolyl, Isothiazolyl, Thiadiazolyl, Oxazolyl, Isoxazolyl, Oxadiazol, Imidazolyl, Pyrazolyl, 1,2,3-Triazolyl, 1,2,4-Triazolyl, Pyridyl, 2-Pyridon, 4-Pyridon, Pyrimidinyl, Pyridazinyl, Pyrazinyl, 1,2,3-Triazolyl, 1,3,5-Triazinyl und 1,2,4,5-Tetrazinyl ausgewählt ist, von denen jedes optional mit 1-3 $Q_1$ substituiert ist;
$R^4$ und $R^5$ jeweils unabhängig aus Deuterium, Wasserstoff oder aus Methyl, Ethyl, Propyl, Isopropyl, Butyl, Isobutyl, sec-Butyl, tert-Butyl, Methoxy, Ethoxy, Propoxy, Isopropoxy, Methylamino, Dimethylamino, Monofluormethyl, Difluormethyl, Trifluormethyl, Hydroxymethyl, Hydroxyethyl, Hydroxypropyl, Hydroxybutyl, Aminomethyl, Carboxymethyl, Carboxyethyl, Monofluormethoxy, Difluormethoxy und Trifluormethoxy ausgewählt sind, von denen jedes optional deuteriert ist;
jedes $Q_1$ unabhängig aus Deuterium, Fluor, Chlor, Brom, Iod oder aus Methoxy, Ethoxy, Propoxy, Isopropoxy, Methylamino, Dimethylamino, Monofluormethyl, Difluormethyl, Trifluormethyl, Monofluormethoxy, Difluormethoxy, Trifluormethoxy, Hydroxymethyl, Hydroxyethyl, Hydroxypropyl, Hydroxybutyl, Aminomethyl, Carboxymethyl, Carboxyethyl, $-(L)_m-C_{1-4}$-Alkyl oder $-(L)_m$-3-6-gliedrigem Cycloalkyl ausgewählt ist, von denen jedes optional mit 1-3 Substituenten $Q_2$ substituiert ist, und
jedes $Q_2$ unabhängig aus Deuterium, Halogen, Carboxyl, Hydroxyl, Cyan, Nitro, Amino, Methyl, Ethyl, Propyl, Isopropyl, Butyl, Isobutyl, sec-Butyl, tert-Butyl, Methoxy, Ethoxy, Propoxy, Isopropoxy, Hydroxymethyl, Hydroxyethyl, Hydroxypropyl, Hydroxybutyl, Carboxymethyl, Carboxyethyl, Methylamino, Dimethylamino, Monofluormethyl, Difluormethyl, Trifluormethyl, Monofluormethoxy, Difluormethoxy und Trifluormethoxy ausgewählt ist;
jeder L unabhängig aus $-CR^aR^b-$ ausgewählt ist;
jeder $R^a$ und jeder $R^b$ jeweils unabhängig aus Deuterium, Wasserstoff ausgewählt ist;
$Y_3$, $Y_4$ und $Y_6$ jeweils unabhängig aus N, C oder $CR^a$ ausgewählt sind;
$Y_5$ und $Y_7$ jeweils unabhängig aus N, $NR^c$, C, $CR^aR^b$ oder $CR^a$ ausgewählt sind;
jeder $R^c$ unabhängig aus Deuterium, Wasserstoff oder aus $C_{1-4}$-Alkyl, halogeniertem $C_{1-4}$-Alkyl und halogeniertem $C_{1-4}$-Alkoxy ausgewählt ist, von denen jedes optional deuteriert ist;
jedes m unabhängig 0, 1 oder 2 ist;
-- und - jeweils unabhängig aus einer Einfachbindung oder einer Doppelbindung ausgewählt sind und zwei benachbarte Bindungen nicht gleichzeitig Doppelbindungen sein können; und
jedes s unabhängig 0, 1 oder 2 ist.

4. Verbindung oder pharmazeutisch annehmbares Salz, deuterierte Verbindung oder Stereoisomer davon gemäß einem der Ansprüche 1-3,
**dadurch gekennzeichnet, dass**:

$R^1$ aus

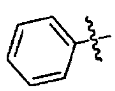, 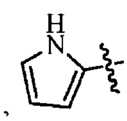, 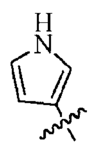, 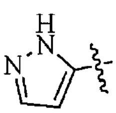, 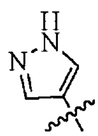, 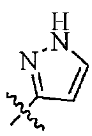, 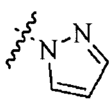, 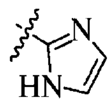,

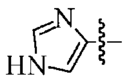 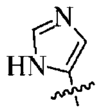 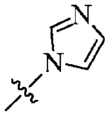 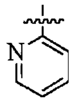 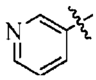 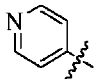 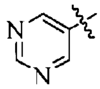 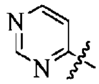 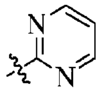 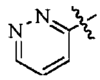

oder

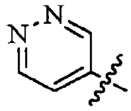

ausgewählt ist, optional substituiert mit 1-3 $Q_1$.

**5.** Verbindung oder pharmazeutisch annehmbares Salz, deuterierte Verbindung oder Stereoisomer davon gemäß Anspruch 1 oder 2, **gekennzeichnet durch** das Beinhalten einer Struktur der Formel (II-5),

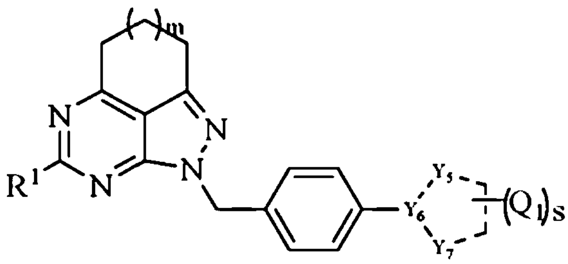

Formel (II-5),

**dadurch gekennzeichnet, dass**:

$R^1$ aus Phenyl oder 5-6-gliedrigem, Stickstoff enthaltendem Heteroaryl ausgewählt ist, optional substituiert mit 1-3 $Q_1$;

jedes $Q_1$ unabhängig aus Deuterium, Wasserstoff oder aus $C_{1-6}$-Alkyl, $C_{1-6}$-Alkoxy, halogeniertem $C_{1-6}$-Alkyl, halogeniertem $C_{1-6}$-Alkoxy und 3-6-gliedrigem Cycloalkyl ausgewählt ist, von denen jedes optional mit 1-3 Substituenten $Q_2$ substituiert ist; und

jedes $Q_2$ unabhängig aus Deuterium, $C_{1-6}$-Alkyl, $C_{1-6}$-Alkoxy, halogeniertem $C_{1-6}$-Alkyl und halogeniertem $C_{1-6}$-Alkoxy ausgewählt ist;

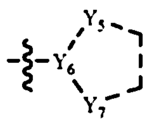

aus den folgenden Strukturen ausgewählt ist:

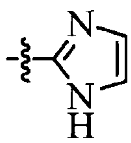 , 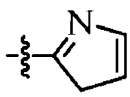 ,

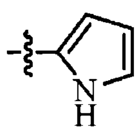 , 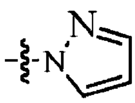 , 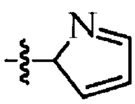 und 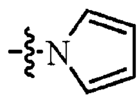 ;

m eine ganze Zahl von 1 oder 2 ist; und

jedes s unabhängig eine ganze Zahl von 0, 1 oder 2 ist.

**6.** Verbindung oder pharmazeutisch annehmbares Salz, deuterierte Verbindung oder Stereoisomer davon gemäß Anspruch 1, **gekennzeichnet durch** das Beinhalten einer Struktur der Formel (III-1'),

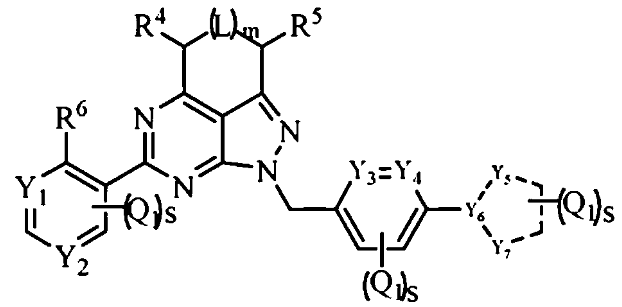

Formel (III-1'),

**dadurch gekennzeichnet, dass**:

$R^6$ aus Wasserstoff oder $C_{1-6}$-Alkyl, $C_{1-6}$-Alkoxy, halogeniertem $C_{1-6}$-Alkyl und halogeniertem $C_{1-6}$-Alkoxy ausgewählt ist, von denen jedes optional deuteriert ist;
$Y_1$, $Y_2$, $Y_3$, $Y_4$ und $Y_6$ jeweils unabhängig aus N, C oder $CR^a$ ausgewählt sind;
$Y_5$ und $Y_7$ jeweils unabhängig aus N, $NR^c$, C, $CR^aR^b$ oder $CR^a$ ausgewählt sind;
--- jeweils unabhängig aus einer Einfachbindung oder einer Doppelbindung ausgewählt ist und zwei benachbarte Bindungen nicht gleichzeitig Doppelbindungen sein können;
jedes s unabhängig aus einer ganzen Zahl von 0-2 ausgewählt ist.

**7.** Verbindung oder pharmazeutisch annehmbares Salz, deuterierte Verbindung oder Stereoisomer davon gemäß Anspruch 1, **gekennzeichnet durch** das Beinhalten einer Struktur der Formel (IV-1'),

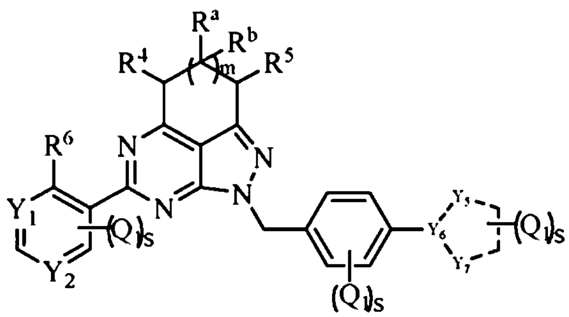

Formel (IV-1'),

wobei $R^6$ aus Wasserstoff oder optional deuteriertem $C_{1-6}$-Alkyl, $C_{1-6}$-Alkoxy, halogeniertem $C_{1-6}$-Alkyl und halogeniertem $C_{1-6}$-Alkoxy ausgewählt ist;
$Y_1$ und $Y_2$ jeweils unabhängig aus N, C oder $CR^a$ ausgewählt sind;
$Y_6$ aus N, C oder $CR^a$ ausgewählt ist;
$Y_5$ und $Y_7$ jeweils unabhängig aus N, $NR^c$, $CR^aR^b$ oder $CR^a$ ausgewählt sind;
-- und - jeweils unabhängig aus einer Einfachbindung oder einer Doppelbindung ausgewählt sind und zwei benachbarte Bindungen nicht gleichzeitig Doppelbindungen sein können; und
jedes s unabhängig aus einer ganzen Zahl von 0-2 ausgewählt ist.

**8.** Verbindung oder pharmazeutisch annehmbares Salz, deuterierte Verbindung oder Stereoisomer davon gemäß Anspruch 7, **dadurch gekennzeichnet, dass**:

$R^6$ aus deuteriertem Methoxy, deuteriertem Ethoxy, deuteriertem Propoxy oder
deuteriertem Isopropoxy ausgewählt ist und eine Deuterierungszahl 1, 2 oder 3 beträgt.

**9.** Verbindung oder pharmazeutisch annehmbares Salz, deuterierte Verbindung oder Stereoisomer davon gemäß einem der Ansprüche 1-8, **gekennzeichnet durch** das Beinhalten der folgenden Struktur:

| Nr. | Struktur | Nr. | Struktur |
|---|---|---|---|
| Verbindung 1 | 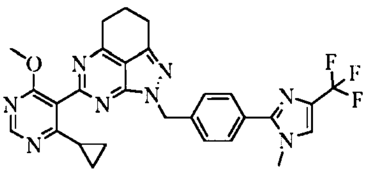 | Verbindung 2 | 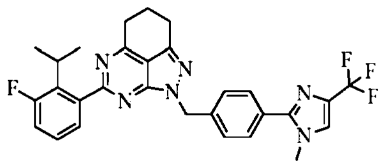 |
| Verbindung 3 | 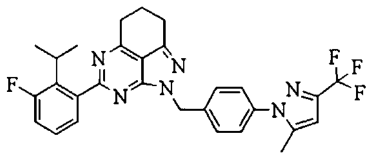 | Verbindung 4 | 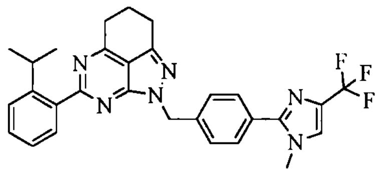 |
| Verbindung 5 | 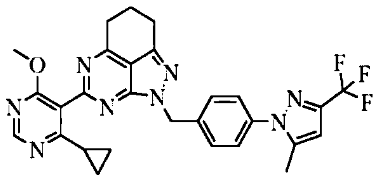 | Verbindung 6 | 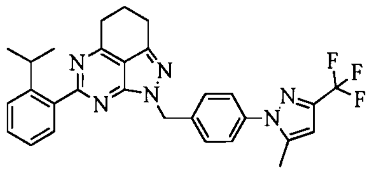 |
| Verbindung 7 | 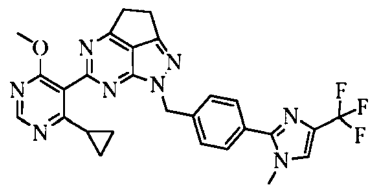 | Verbindung 8 | 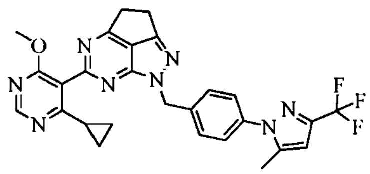 |
| Verbindung 9 | 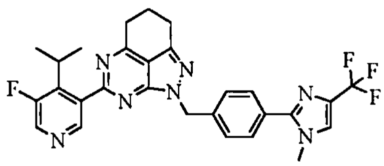 | Verbindung 10 | 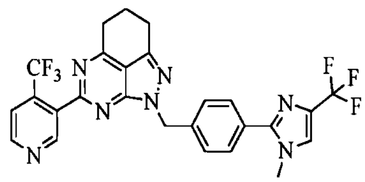 |
| Verbindung 11 | 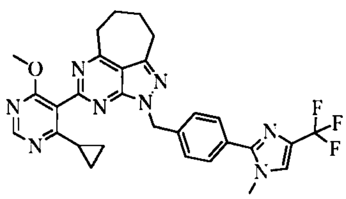 | Verbindung 12 | 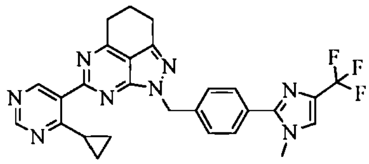 |
| Verbindung 13 | 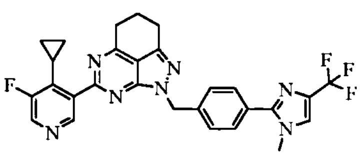 | Verbindung 14 | 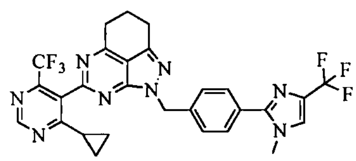 |
| Verbindung 15 | 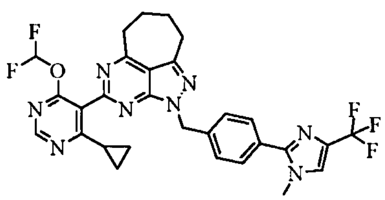 | Verbindung 16 | 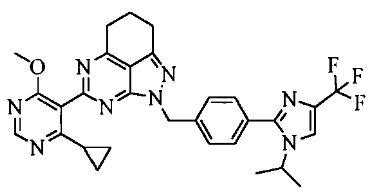 |

(continued)

| Nr. | Struktur | Nr. | Struktur |
|---|---|---|---|
| Verbindung 17 | 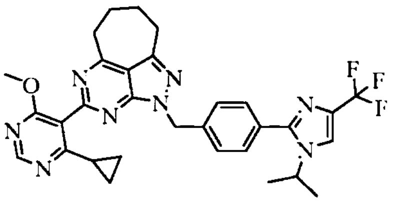 | Verbindung 18 | 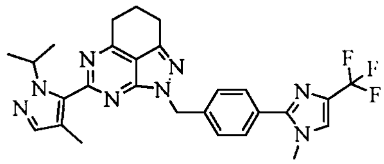 |
| Verbindung 19 | 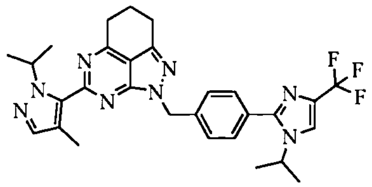 | Verbindung 20 | 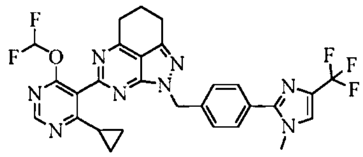 |
| Verbindung 1-1 | 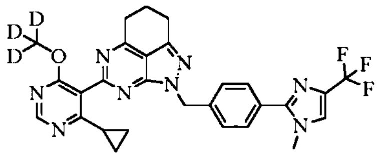 | Verbindung 5-1 | 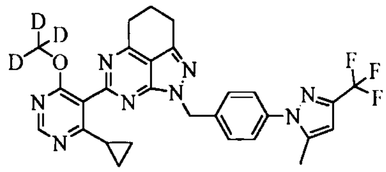 |
| Verbindung 7-1 | 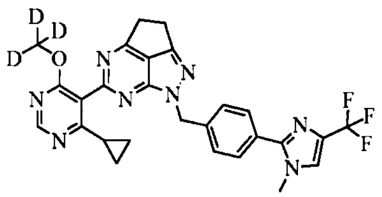 | Verbindung 8-1 | 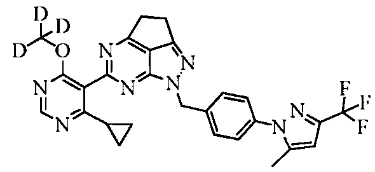 |
| Verbindung 11-1 | 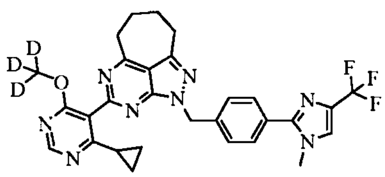 | Verbindung 16-1 | 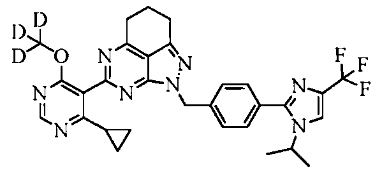 |
| Verbindung 17-1 | 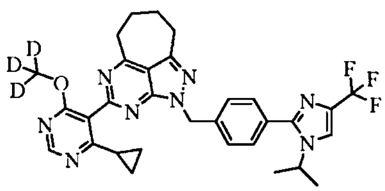 | | |

**10.** Eine pharmazeutische Formulierung, die die Verbindung oder das pharmazeutisch annehmbare Salz, die deuterierte Verbindung oder das Stereoisomer davon gemäß einem der Ansprüche 1-9 und ein(en) oder mehrere pharmazeutisch annehmbare Träger und/oder Verdünnungsmittel beinhaltet, **dadurch gekennzeichnet, dass** die pharmazeutische Formulierung eine beliebige Darreichungsform ist, die klinisch oder pharmazeutisch annehmbar ist.

**11.** Eine pharmazeutische Zusammensetzung, **gekennzeichnet durch** das Beinhalten der Verbindung oder des pharmazeutisch annehmbaren Salzes, der deuterierten Verbindung oder des Stereoisomers davon gemäß einem der Ansprüche 1-9 oder der pharmazeutischen Formulierung gemäß Anspruch 10 und eines oder mehrerer zweiter therapeutischer Wirkstoffe.

**12.** Verbindung oder pharmazeutisch annehmbares Salz, deuterierte Verbindung oder Stereoisomer davon gemäß einem der Ansprüche 1-9 oder pharmazeutische Formulierung gemäß Anspruch 10 oder pharmazeutische Zusammensetzung gemäß Anspruch 11 zur Verwendung beim Behandeln und/oder Vorbeugen einer durch USP1 vermittelten Krankheit und einer damit zusammenhängenden Krankheit; die durch USP1 vermittelte Krankheit und die

damit zusammenhängende Krankheit sind aus Krebs und gutartigem Tumor ausgewählt.

**13.** Ein Verfahren zum Herstellen der Verbindung oder des pharmazeutisch annehmbaren Salzes, der deuterierten Verbindung oder des Stereoisomers davon gemäß einem der Ansprüche 1-9, **gekennzeichnet durch** das Beinhalten der folgenden Schritte:

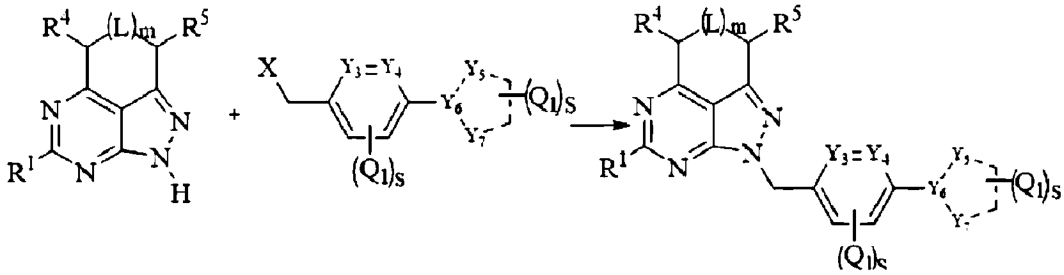

Formel (II-2)-1 Formel (II-2)-2 Formel (II-2)

Umsetzen der Verbindung der Formel (II-2)-1 mit der Verbindung der Formel (II-2)-2, um die Verbindung der Formel (II-2) zu erhalten;
X ist Halogen.

**14.** Ein Zwischenprodukt zum Herstellen der Verbindung oder des pharmazeutisch annehmbaren Salzes, der deuterierten Verbindung oder des Stereoisomers davon gemäß einem der Ansprüche 1-9, **gekennzeichnet durch** das Beinhalten der folgenden Struktur:

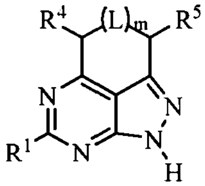
Formel (II-2)-1,

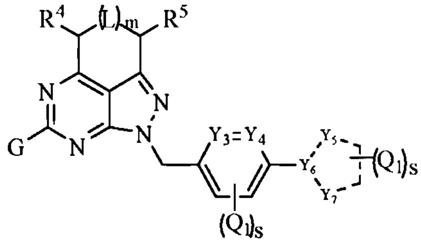
Formel (II-2)-3,

wobei G aus Wasserstoff, Hydroxyl, Amino, $C_{1-6}$-Alkylthio oder $C_{1-6}$-Alkylsulfonyl ausgewählt ist.

## Revendications

**1.** Un composé de formule générale (II-2) ou un sel pharmaceutiquement acceptable, un composé deutéré ou un stéréoisomère de celui-ci,

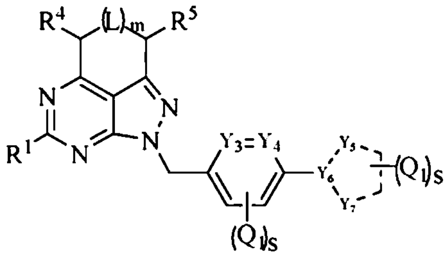

formule (II-2)

**caractérisé en ce que** :

$R^1$ est sélectionné parmi un phényle ou un hétéroaryle de 5 à 6 chaînons facultativement substitué par 1 à 4 $Q_1$ ;
$R^4$ et $R^5$ sont chacun indépendamment sélectionnés parmi un deutérium, l'hydrogène, un carboxyle, un cyano,

un nitro, un amino, un halogène, un alcényle en $C_{2-6}$, un alcynyle en $C_{2-6}$, ou parmi un alkyle en $C_{1-6}$, un alcoxy en $C_{1-6}$, un alkylamino en $C_{1-6}$, un di(alkyle en $C_{1-6}$)amino, un alkyle en $C_{1-6}$ halogéné, un hydroxyalkyle en $C_{1-6}$, un aminoalkyle en $C_{1-6}$, un carboxyalkyle en $C_{1-6}$ ou un alcoxy en $C_{1-6}$ halogéné, chacun d'entre eux étant facultativement deutéré ;
chaque $Q_1$ est indépendamment sélectionné parmi un deutérium, un halogène, un cyano, un carboxyle, un hydroxyle, un amino, un nitro, un sulfonamido, ou parmi un alkylamino en $C_{1-6}$, un di(alkyle en $C_{1-6}$)amino, un alkyle en $C_{1-6}$ halogéné, un alcoxy en $C_{1-6}$ halogéné, un hydroxyalkyle en $C_{1-6}$, un aminoalkyle en $C_{1-6}$, un carboxyalkyle en $C_{1-6}$, un alkylcarbonyle en $C_{1-6}$, un alcoxycarbonyle en $C_{1-6}$, un alkylaminoacyle en $C_{1-6}$, un alkylamido en $C_{1-6}$, un alkylsulfonyle en $C_{1-6}$, un alkylsulfonamido en $C_{1-6}$, un alkylaminosulfonyle en $C_{1-6}$, un $-(L)_m$-alkyle en $C_{1-6}$, un $-(L)_m$-alcényle en $C_{2-6}$, un $-(L)_m$-alcynyle en $C_{2-6}$, un $-(L)_m$-alcoxy en $C_{1-6}$, un $-(L)_m$-aryle de 6 à 10 chaînons, un $-(L)_m$-hétéroaryle de 5 à 12 chaînons, un $-(L)_m$-cycloalkyle de 3 à 8 chaînons ou un $-(L)_m$-hétérocyclyle de 3 à 8 chaînons, chacun d'entre eux étant facultativement substitué par 1 à 4 substituants $Q_2$ ; et
chaque $Q_2$ est indépendamment sélectionné parmi un deutérium, un halogène, un carboxyle, un hydroxyle, un cyano, un nitro, un amino, un alkyle en $C_{1-6}$, un hydroxyalkyle en $C_{1-6}$, un carboxyalkyle en $C_{1-6}$, un alkylamino en $C_{1-6}$, un di(alkyle en $C_{1-6}$)amino, un -CO-alkylène en $C_{1-6}$-$NH_2$, un -CO-alkyle en $C_{1-6}$, un alcoxy en $C_{1-6}$, un alkyle en $C_{1-6}$ halogéné et un alcoxy en $C_{1-6}$ halogéné ;
chaque L est indépendamment sélectionné parmi $-CR^aR^b-$ ;
chaque $R^a$ et chaque $R^b$ sont chacun indépendamment sélectionnés parmi un deutérium, l'hydrogène, ou parmi un alkyle en $C_{1-6}$, un alcoxy en $C_{1-6}$, chacun d'entre eux étant facultativement deutéré ;
$Y_3$, $Y_4$ et $Y_6$ sont chacun indépendamment sélectionnés parmi N, C ou $CR^a$ ;
$Y_5$ et $Y_7$ sont chacun indépendamment sélectionnés parmi N, $NR^c$, C, $CR^aR^b$ ou $CR^a$ ; chaque $R^c$ est indépendamment sélectionné parmi un deutérium, l'hydrogène, ou parmi un alkyle en $C_{1-6}$, un alkyle en $C_{1-6}$ halogéné, un alcoxy en $C_{1-6}$ halogéné, un hydroxyalkyle en $C_{1-6}$, un aminoalkyle en $C_{1-6}$ et un carboxyalkyle en $C_{1-6}$, chacun d'entre eux étant facultativement deutéré ;
chaque m est indépendamment un entier de 0 à 3 ;
-- et - sont chacune indépendamment sélectionnées parmi une liaison simple ou une liaison double, et deux liaisons adjacentes sont incapables d'être des liaisons doubles en même temps ; et
chaque s est indépendamment sélectionné parmi un entier de 0 à 2.

2. Le composé ou le sel pharmaceutiquement acceptable, le composé deutéré ou le stéréoisomère de celui-ci selon la revendication 1, **caractérisé en ce que** :

$R^1$ est sélectionné parmi un phényle ou un hétéroaryle contenant de l'azote de 5 à 6 chaînons, dont chacun est facultativement substitué par 1 à 3 $Q_1$ ;
$R^4$ et $R^5$ sont chacun indépendamment sélectionnés parmi un deutérium, l'hydrogène, un cyano, un halogène, ou parmi un alkyle en $C_{1-6}$, un alcoxy en $C_{1-6}$, un alkylamino en $C_{1-6}$, un di(alkyle en $C_{1-6}$)amino, un alkyle en $C_{1-6}$ halogéné, un hydroxyalkyle en $C_{1-6}$, un aminoalkyle en $C_{1-6}$, un carboxyalkyle en $C_{1-6}$ ou un alcoxy en $C_{1-6}$ halogéné, chacun d'entre eux étant facultativement deutéré ;
chaque $Q_1$ est indépendamment sélectionné parmi un deutérium, l'hydrogène, ou parmi un alkyle en $C_{1-6}$, un alcoxy en $C_{1-6}$, un alkyle en $C_{1-6}$ halogéné, un alcoxy en $C_{1-6}$ halogéné, et un cycloalkyle de 3 à 6 chaînons, chacun d'entre eux étant facultativement substitué par 1 à 3 substituants $Q_2$ ; et
chaque $Q_2$ est indépendamment sélectionné parmi un deutérium, un alkyle en $C_{1-6}$, un alcoxy en $C_{1-6}$, un alkyle en $C_{1-6}$ halogéné et un alcoxy en $C_{1-6}$ halogéné ;
chaque L est indépendamment sélectionné parmi $-CR^aR^b-$ ;
chaque $R^a$ et chaque $R^b$ sont chacun indépendamment sélectionnés parmi un deutérium, l'hydrogène, ou parmi un méthyle, un éthyle, un propyle, un isopropyle, un butyle, un isobutyle, un sec-butyle, un tert-butyle, un méthoxy, un éthoxy, un propoxy ou un isopropoxy, chacun d'entre eux étant facultativement deutéré ;
$Y_3$ et $Y_4$ sont chacun indépendamment sélectionnés parmi N, C ou $CR^a$ ;

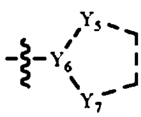

est sélectionné parmi les structures suivantes :

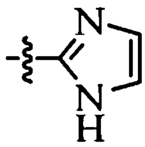, 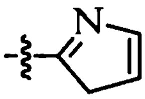, 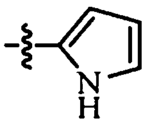, 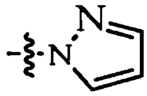, 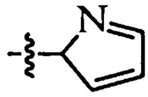,

et

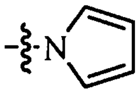
;

chaque $R^c$ est indépendamment sélectionné parmi un deutérium, l'hydrogène, ou parmi un alkyle en $C_{1-6}$, un alkyle en $C_{1-6}$ halogéné et un alcoxy en $C_{1-6}$ halogéné, chacun d'entre eux étant facultativement deutéré ;
chaque m est indépendamment un entier de 0 à 2 ;
-- et - sont chacune indépendamment sélectionnées parmi une liaison simple ou une liaison double, et deux liaisons adjacentes sont incapables d'être des liaisons doubles en même temps ; et
chaque s est indépendamment sélectionné parmi un entier de 0 à 2.

**3.** Le composé ou le sel pharmaceutiquement acceptable, le composé deutéré ou le stéréoisomère de celui-ci selon la revendication 1,
**caractérisé en ce que** :

$R^1$ est sélectionné parmi un phényle, un furyle, un thiényle, un pyrrolyle, un thiazolyle, un isothiazolyle, un thiadiazolyle, un oxazolyle, un isoxazolyle, un oxadiazole, un imidazolyle, un pyrazolyle, un 1,2,3-triazolyle, un 1,2,4-triazolyle, un pyridyle, une 2-pyridone, une 4-pyridone, un pyrimidinyle, un pyridazinyle, un pyrazinyle, un 1,2,3-triazolyle, un 1,3,5-triazinyle et un 1,2,4,5-tétrazinyle, dont chacun est facultativement substitué par 1 à 3 $Q_1$ ;
$R^4$ et $R^5$ sont chacun indépendamment sélectionnés parmi un deutérium, l'hydrogène, ou parmi un méthyle, un éthyle, un propyle, un isopropyle, un butyle, un isobutyle, un sec-butyle, un tert-butyle, un méthoxy, un éthoxy, un propoxy, un isopropoxy, un méthylamino, un diméthylamino, un monofluorométhyle, un difluorométhyle, un trifluorométhyle, un hydroxyméthyle, un hydroxyéthyle, un hydroxypropyle, un hydroxybutyle, un aminométhyle, un carboxyméthyle, un carboxyéthyle, un monofluorométhoxy, un difluorométhoxy et un trifluorométhoxy, dont chacun est facultativement deutéré ;
chaque $Q_1$ est indépendamment sélectionné parmi un deutérium, le fluor, le chlore, le brome, l'iode, ou parmi un méthoxy, un éthoxy, un propoxy, un isopropoxy, un méthylamino, un diméthylamino, un monofluorométhyle, un difluorométhyle, un trifluorométhyle, un monofluorométhoxy, un difluorométhoxy, un trifluorométhoxy, un hydroxyméthyle, un hydroxyéthyle, un hydroxypropyle, un hydroxybutyle, un aminométhyle, un carboxyméthyle, un carboxyéthyle, un $-(L)_m$-alkyle en $C_{1-4}$, ou un - $(L)_m$-cycloalkyle de 3 à 6 chaînons, chacun d'entre eux étant facultativement substitué par 1 à 3 substituants $Q_2$, et chaque $Q_2$ est indépendamment sélectionné parmi un deutérium, un halogène, un carboxyle, un hydroxyle, un cyano, un nitro, un amino, un méthyle, un éthyle, un propyle, un isopropyle, un butyle, un isobutyle, un sec-butyle, un tert-butyle, un méthoxy, un éthoxy, un propoxy, un isopropoxy, un hydroxyméthyle, un hydroxyéthyle, un hydroxypropyle, un hydroxybutyle, un carboxyméthyle, un carboxyéthyle, un méthylamino, un diméthylamino, un monofluorométhyle, un difluorométhyle, un trifluorométhyle, un monofluorométhoxy, un difluorométhoxy et un trifluorométhoxy ;
chaque L est indépendamment sélectionné parmi $-CR^aR^b-$ ;
chaque $R^a$ et chaque $R^b$ sont chacun indépendamment sélectionnés parmi un deutérium, l'hydrogène ;
$Y_3$, $Y_4$ et $Y_6$ sont chacun indépendamment sélectionnés parmi N, C ou $CR^a$ ;
$Y_5$ et $Y_7$ sont chacun indépendamment sélectionnés parmi N, $NR^c$, C, $CR^aR^b$ ou $CR^a$ ;
chaque $R^c$ est indépendamment sélectionné parmi un deutérium, l'hydrogène, ou parmi un alkyle en $C_{1-4}$, un alkyle en $C_{1-4}$ halogéné et un alcoxy en $C_{1-4}$ halogéné, chacun d'entre eux étant facultativement deutéré ;
chaque m est indépendamment 0, 1 ou 2 ;
-- et - sont chacune indépendamment sélectionnées parmi une liaison simple ou une liaison double, et deux liaisons adjacentes sont incapables d'être des liaisons doubles en même temps ; et
chaque s est indépendamment 0, 1 ou 2.

**4.** Le composé ou le sel pharmaceutiquement acceptable, le composé deutéré ou le stéréoisomère de celui-ci selon

l'une quelconque des revendications 1 à 3, **caractérisé en ce que** :

$R^1$ est sélectionné parmi

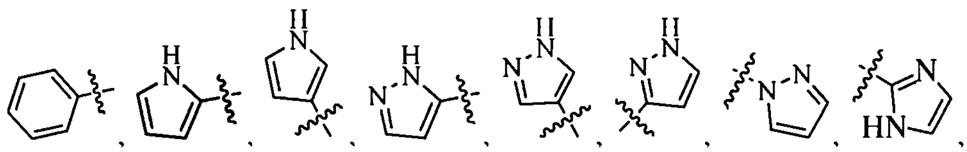
, , , , , , , ,

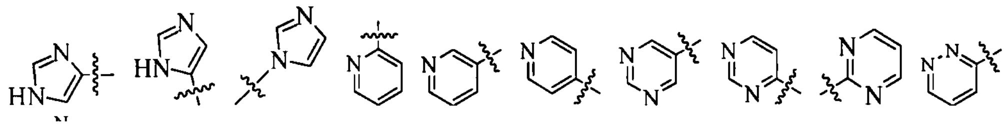

ou

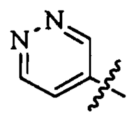

facultativement substitué par 1 à 3 $Q_1$.

**5.** Le composé ou le sel pharmaceutiquement acceptable, le composé deutéré ou le stéréoisomère de celui-ci selon la revendication 1 ou la revendication 2, **caractérisé en ce qu'**il comprend une structure de formule (II-5),

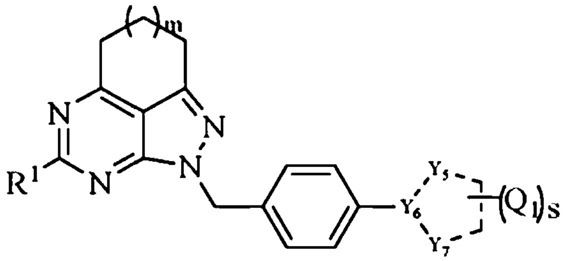
formule (II-5)

**caractérisé en ce que** :

$R^1$ est sélectionné parmi un phényle ou un hétéroaryle contenant de l'azote de 5 à 6 chaînons facultativement substitué par 1 à 3 $Q_1$ ;
chaque $Q_1$ est indépendamment sélectionné parmi un deutérium, l'hydrogène, ou parmi un alkyle en $C_{1-6}$, un alcoxy en $C_{1-6}$, un alkyle en $C1_{-6}$ halogéné, un alcoxy en $C_{1-6}$ halogéné, et un cycloalkyle de 3 à 6 chaînons, chacun d'entre eux étant facultativement substitué par 1 à 3 substituants $Q_2$ ; et chaque $Q_2$ est indépendamment sélectionné parmi un deutérium, un alkyle en $C_{1-6}$, un alcoxy en $C_{1-6}$, un alkyle en $C_{1-6}$ halogéné et un alcoxy en $C_{1-6}$ halogéné ;

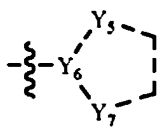

est sélectionné parmi les structures suivantes :

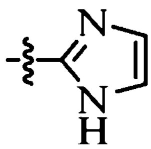, 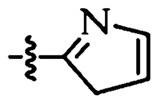, 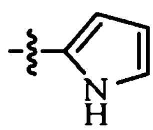, 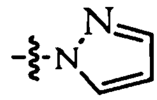, 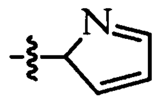,

et

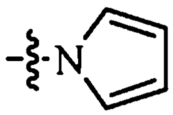;

m est un entier de 1 ou 2 ; et
chaque s est indépendamment un entier de 0, 1 ou 2.

**6.** Le composé ou le sel pharmaceutiquement acceptable, le composé deutéré ou le stéréoisomère de celui-ci selon la revendication 1, **caractérisé en ce qu'**il comprend une structure de formule (III-1'),

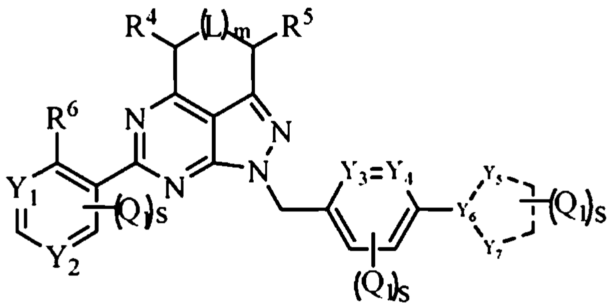

formule (III-1')

**caractérisé en ce que** :

$R^6$ est sélectionné parmi l'hydrogène, ou un alkyle en $C_{1-6}$, un alcoxy en $C_{1-6}$, un alkyle en $C_{1-6}$ halogéné et un alcoxy en $C_{1-6}$ halogéné, chacun d'entre eux étant facultativement deutéré ;
$Y_1$, $Y_2$, $Y_3$, $Y_4$ et $Y_6$ sont chacun indépendamment sélectionnés parmi N, C ou $CR^a$ ;
$Y_5$ et $Y_7$ sont chacun indépendamment sélectionnés parmi N, $NR^c$, C, $CR^aR^b$ ou $CR^a$ ;
--- sont chacune indépendamment sélectionnées parmi une liaison simple ou une liaison double, et deux liaisons adjacentes sont incapables d'être des liaisons doubles en même temps ;
chaque s est indépendamment sélectionné parmi un entier de 0 à 2.

**7.** Le composé ou le sel pharmaceutiquement acceptable, le composé deutéré ou le stéréoisomère de celui-ci selon la revendication 1, **caractérisé en ce qu'**il comprend une structure de formule (IV-1'),

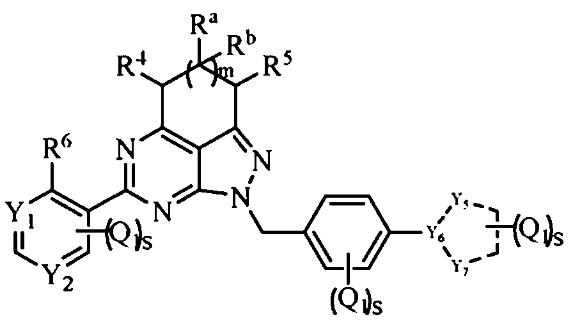

formule (IV-1')

dans laquelle, $R^6$ est sélectionné parmi l'hydrogène, ou un alkyle en $C_{1-6}$ facultativement deutéré, un alcoxy en $C_{1-6}$, un alkyle en $C_{1-6}$ halogéné et un alcoxy en $C_{1-6}$ halogéné ;

$Y_1$ et $Y_2$ sont chacun indépendamment sélectionnés parmi N, C ou $CR^a$ ;
$Y_6$ est sélectionné parmi N, C ou $CR^a$ ;
$Y_5$ et $Y_7$ sont chacun indépendamment sélectionnés parmi N, $NR^c$, $CR^aR^b$ ou $CR^a$ ; -- et - sont chacune indépendamment sélectionnées parmi une liaison simple ou une liaison double, et deux liaisons adjacentes sont incapables d'être des liaisons doubles en même temps ; et
chaque s est indépendamment sélectionné parmi un entier de 0 à 2.

**8.** Le composé ou le sel pharmaceutiquement acceptable, le composé deutéré ou le stéréoisomère de celui-ci selon la revendication 7, **caractérisé en ce que** :
$R^6$ est sélectionné parmi un méthoxy deutéré, un éthoxy deutéré, un propoxy deutéré ou un isopropoxy deutéré, et un nombre de deutération est 1, 2 ou 3.

**9.** Le composé ou le sel pharmaceutiquement acceptable, le composé deutéré ou le stéréoisomère de celui-ci selon l'une quelconque des revendications 1 à 8, **caractérisé en ce qu'**il comprend la structure suivante :

| N° | Structure | N° | Structure |
|---|---|---|---|
| Composé 1 | 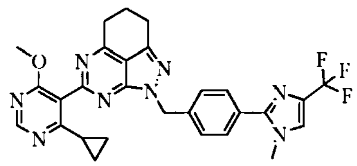 | Composé 2 | 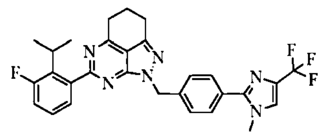 |
| Composé 3 | 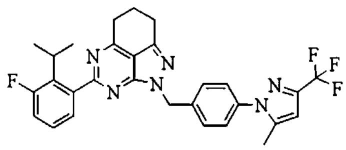 | Composé 4 | 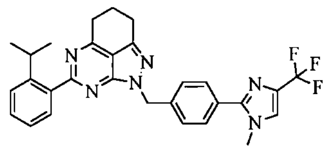 |
| Composé 5 | 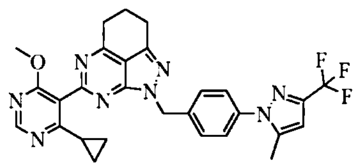 | Composé 6 | 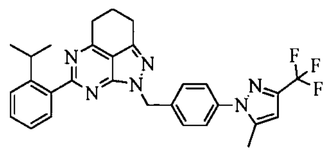 |
| Composé 7 | 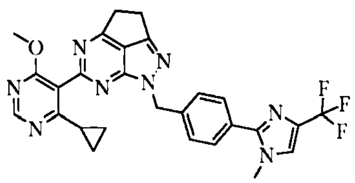 | Composé 8 | 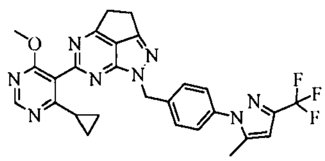 |
| Composé 9 | 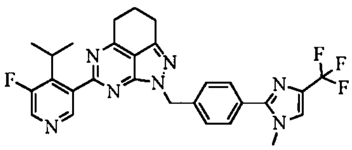 | Composé 10 | 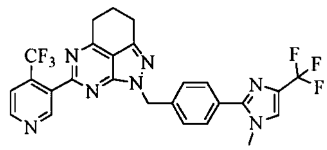 |
| Composé 11 | 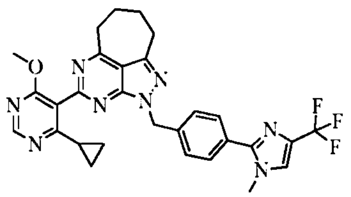 | Composé 12 | 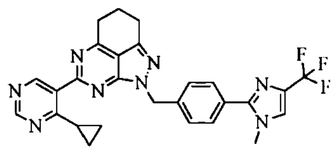 |
| Composé 13 | 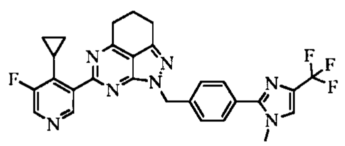 | Composé 14 | 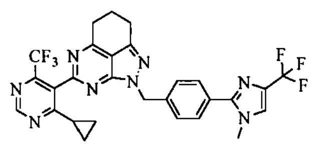 |

(continued)

| N° | Structure | N° | Structure |
|---|---|---|---|
| Composé 15 | 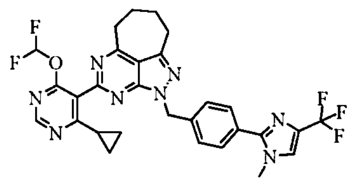 | Composé 16 | 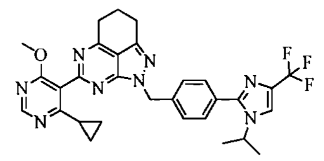 |
| Composé 17 | 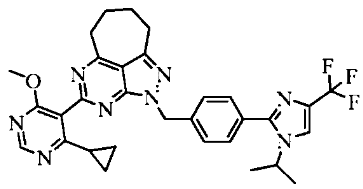 | Composé 18 | 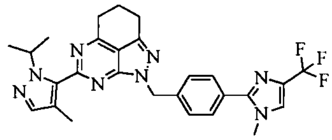 |
| Composé 19 | 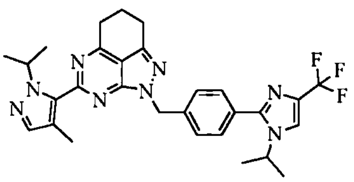 | Composé 20 | 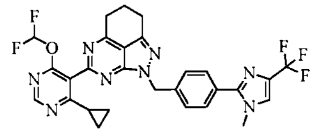 |
| Composé 1-1 | 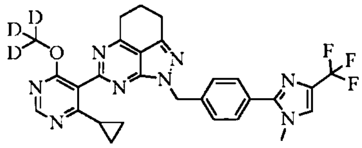 | Composé 5-1 | 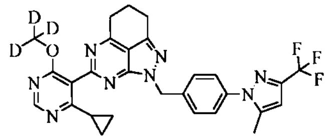 |
| Composé 7-1 | 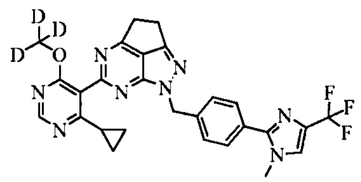 | Composé 8-1 | 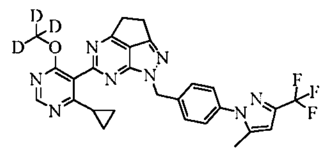 |
| Composé 11-1 | 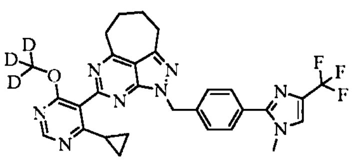 | Composé 16-1 | 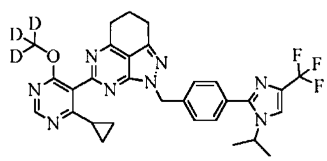 |
| Composé 17-1 | 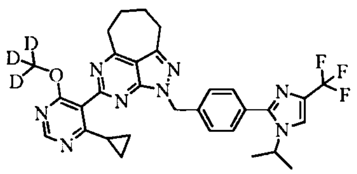 | | |

**10.** Une formulation pharmaceutique, comprenant le composé ou le sel pharmaceutiquement acceptable, le composé deutéré ou le stéréoisomère de celui-ci selon l'une quelconque des revendications 1 à 9, et un ou plusieurs excipients et/ou diluants pharmaceutiquement acceptables, **caractérisée en ce que** la formulation pharmaceutique est n'importe quelle forme posologique qui est cliniquement ou pharmaceutiquement acceptable.

**11.** Une composition pharmaceutique, **caractérisée en ce qu'**elle comprend le composé ou le sel pharmaceutiquement acceptable, le composé deutéré ou le stéréoisomère de celui-ci selon l'une quelconque des revendications 1 à 9, ou la formulation pharmaceutique selon la revendication 10, et un ou plusieurs deuxièmes agents actifs thérapeutiques.

**12.** Le composé ou le sel pharmaceutiquement acceptable, le composé deutéré ou le stéréoisomère de celui-ci selon l'une quelconque des revendications 1 à 9, ou la formulation pharmaceutique selon la revendication 10, ou la

composition pharmaceutique selon la revendication 11 pour son utilisation dans le traitement et/ou la prévention d'une maladie médiée par USP1 et d'une maladie liée à celle-ci ; la maladie médiée par USP1 et la maladie liée à celle-ci sont sélectionnées parmi un cancer ou une tumeur bénigne.

**13.** Un procédé pour la préparation du composé ou du sel pharmaceutiquement acceptable, du composé deutéré ou du stéréoisomère de celui-ci selon l'une quelconque des revendications 1 à 9, **caractérisé en ce qu'**il comprend les étapes suivantes de :

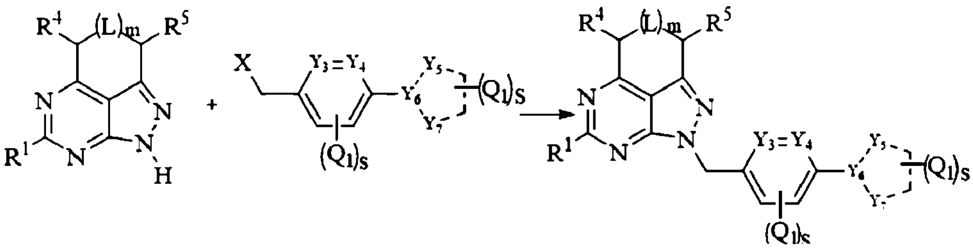

formule (II-2)-1 formule (II-2)-2 formule (II-2)

mise en réaction du composé de formule (II-2)-1 avec le composé de formule (II-2)-2 afin d'obtenir le composé de formule (II-2) ;
X est un halogène.

**14.** Un intermédiaire pour la préparation du composé ou du sel pharmaceutiquement acceptable, du composé deutéré ou du stéréoisomère de celui-ci selon l'une quelconque des revendications 1 à 9, **caractérisé en ce qu'**il comprend la structure suivante :

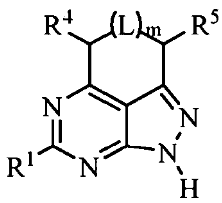
formule (II-2)-1,

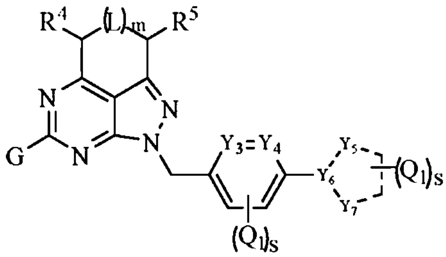
formule (II-2)-3,

dans laquelle, G est sélectionné parmi l'hydrogène, un hydroxyle, un amino, un alkylthio en $C_{1-6}$ ou un alkylsulfonyle en $C_{1-6}$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011036280 A1
- WO 2020132269 A1
- US 2011098483 A1
- JP 2017043603 A